(19) Europäisches Patentamt
European Patent Office
Office européen des brevets

(11) EP 1 250 935 A1

(12) **EUROPEAN PATENT APPLICATION**
published in accordance with Art. 158(3) EPC

(43) Date of publication:
**23.10.2002 Bulletin 2002/43**

(21) Application number: **01946792.7**

(22) Date of filing: **25.01.2001**

(51) Int Cl.$^7$: **A61K 45/00**, A61K 31/506, A61P 9/08, A61P 9/10 // (C07D401/12, 409:14)

(86) International application number:
**PCT/JP01/00496**

(87) International publication number:
**WO 01/054726 (02.08.2001 Gazette 2001/31)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **25.01.2000 JP 2000016342**

(71) Applicant: **Meiji Seika Kaisha, Ltd.**
**Tokyo 104-8002 (JP)**

(72) Inventors:
- **FUJISHIMA, Kazuyuki**
**Pharmaceutical Research Center**
**Yokohama-shi, Kanagawa 222-8567 (JP)**
- **MURAKAMI, Shoichi,**
**Pharmaceutical Research Center**
**Yokohama-shi, Kanagawa 222-8567 (JP)**
- **YAMAMOTO, Mikio,**
**Pharmaceutical Research Center**
**Yokohama-shi, Kanagawa 222-8567 (JP)**
- **ABE, Mitsuhiro,**
**Pharmaceutical Research Center**
**Yokohama-shi, Kanagawa 222-8567 (JP)**
- **ISHIKAWA, Minoru,**
**Pharmaceutical Research Center**
**Yokohama-shi, Kanagawa 222-8567 (JP)**
- **OUCHI, Shokichi,**
**Pharmaceutical Research Center**
**Yokohama-shi, Kanagawa 222-8567 (JP)**
- **AJITO, Keiichi, Pharmaceutical Research Center**
**Yokohama-shi, Kanagawa 222-8567 (JP)**

(74) Representative: **Kyle, Diana**
**Elkington and Fife**
**Prospect House**
**8 Pembroke Road**
**Sevenoaks, Kent TN13 1XR (GB)**

(54) **REMEDIES FOR REPERFUSION INJURY CONTAINING INTEGRIN ALPHAvBETA3 ANTAGONIST**

(57) An objective of the present invention is to provide an agent that is clinically effective in the treatment or prevention of reperfusion injury. The agent for use in the treatment or prevention of reperfusion injury according to the present invention comprises an integrin $\alpha_v\beta_3$ antagonist, a pharmaceutically acceptable salt thereof, or a solvate thereof as active ingredient.

EP 1 250 935 A1

## Description

BACKGROUND OF THE INVENTION

### Field of the Invention

The present invention relates to a pharmaceutical composition comprising an integrin $\alpha_v\beta_3$ antagonist as active ingredient, for use in the prevention or treatment of ischemia-reperfusion injury.

### Description of Related Art

Regarding grave diseases in developed nations, for example, a change in eating habits, life-styles, smoking habits and the like has led to a tendency toward an increase in ischemic diseases, which are deeply involved in the so-called lifestyle-related disease, as a major cause of death. Among others, for ischemic heart diseases and cerebral ischemic diseases, at the present time, in the world, there are very few drugs having potency high enough to effectively prevent or treat these diseases.

Medical systems for coping with ischemic diseases are recently being rapidly improved in Japan and abroad, partly thanks to directions and/or policy on medical administration. At the present time, however, studies on clinical effect in emergency medical treatment and a prognostic improvement, for example, for acute ischemic heart diseases reveal that the trend in current therapy is not always satisfactory in Japan and abroad.

As soon as a patient with acute myocardial infarction is carried into emergency medical facilities, the condition, clinical history of the patient and the like are quickly examined and an emergency medical system on the site, such as equipment and the staff, is instantaneously judged for performing best suited blood flow recanalization therapy, generally PTCA (including stent), administration of a thrombolytic drug, CABG (coronary-aorta bypass surgery) or the like. However, despite proper blood circulation repair operation which has been made as emergency medical treatment, the occurrence of prognostic event cannot be fully prevented. As a result, the life of a certain proportion, generally 7 to 15%, of patients suffering from ischemic heart diseases is threatened.

As a result of biochemical and pathological studies on the affected parts and the peripheral portion thereof in patients after the recanalization therapy, reperfusion injury has recently drawn attention as a great issue of the recanalization therapy. The reperfusion injury leads to the expansion of the focus of myocardial infarction which results in lowered cardiac function. It was already known that the expansion of the focus of infarct leads to death (T. D. Miller et al., Circulation, 92, 334, (1995)) and that the lowered cardiac function leads to death (R. Stevenson et al., Br. Med. J., 307, 349, (1993)). Up to now, various clinical and pre-clinical tests have been carried out in various research institutions for purposes of developing therapeutic agents for reperfusion injury. For detailed information, reference may be made to journals of information on medicines, for example, Acute Myocardial Infarction (Decision Resources, June 1998)). Scientific mechanisms in representative approaches, which have hitherto been studied, include Na/H exchange inhibitory action, nonselective antagonism to endothelin, PKC activating action, selectin inhibitory action, Na/Ca exchange inhibitory action, and radical scavenging action. While compounds having these action mechanisms are valid in animal tests, compounds that are definitely effective for the treatment or prevention of reperfusion injury have hardly been known up to now.

For integrin $\alpha_v\beta_3$ antagonists, the following documents have been published: WO 95/32710 (Merck); WO 96/37492 (DuPont-Merc); WO 97/01540 (SmithKline Beecham); WO 97/08145 (Searle); WO 97/23451 (Merck); WO 97/23480 (DuPont-Merc); WO 97/24119 (SKB); WO 97/26250 (Merck); WO 97/33887 (DuPont-Merc); WO 97/36858 (Searle); WO 97/36859 (Searle); WO 97/36860 (Searle); WO 97/36861 (Searle); WO 97/36862 (Searle); WO 97/24336 (SmithKline Beecham); WO 97/37655 (Merck); WO 98/08840 (Merck); WO 98/18460 (Merck); WO 98/25892 (Lilly); WO 98/30542 (SmithKline Beecham); WO 98/31359 (Merck); WO 98/35949 (Merck); WO 98/43962 (DuPont-Merc); WO 98/46220 (Merck); WO 99/05107 (SmithKline Beecham); WO 99/06049 (SmithKline Beecham); WO 99/11626 (SmithKline Beecham); WO 99/15170 (SmithKline Beecham); WO 99/15178 (SmithKline Beecham); WO 99/15506 (Hoechst Marion Roussel); WO 99/15507 (Hoechst Marion Roussel); WO 99/15508 (SmithKline Beecham); WO 99/30709 (Merck); WO 99/30713 (Merck); WO 99/31061 (Merck); WO 99/31099 (Merck); WO 99/32457 (Hoechst Marion Roussel); WO 99/33798 (Yamanouchi Pharmaceutical Co., Ltd.); WO 99/37621 (Hoechst Marion Roussel); WO 99/38849 (Meiji Seika Kaisha Co., Ltd.); WO 99/44994 (Searle); WO99/45927 (SmithKline Beecham); WO99/50249 (DuPont-Merc); WO99/52872 (Meiji Seika Kaisha Co., Ltd.); WO99/52879 (American Home Products); US 5843906 (Searle); US 5852210 (Searle); EP 796855 (Hoechst); EP 820988 (Hoechst); EP 820991 (Hoechst); EP 853084 (Hoechst); EP 928790 (Hoffmann-La Roche Ltd.); EP 928793 (Hoffmann-La Roche Ltd.); GB 2326609 (Merck); and GB 2327672 (Merck). These documents, however, describe neither action mechanisms of reperfusion injury through leukocytes nor the effectiveness of the compounds listed therein for reperfusion injury.

Further, the results of animal tests of integrin $\alpha_v\beta_3$ antagonists in various pathological areas have been reported: (S. S. Srivatsa et al., The 69th Annual Meeting of American Heart Association, 0231, 1996 (DuPont-Merc); J. F. Gourvest et al., The 18th Annual Meeting of The American Society for Bone and Mineral Research, p228, 1996 (Roussel-Hoechst); S. B. Rodan et al., The 18th Annual Meeting of The American Society for Bone and Mineral Research, M430, 1996 (Merck); T. L. Yue et al., The 70th Annual Meeting of American Heart Association, 3733, 1997 (SmithKline Beecham); A. L. Racanelli et al., The 70th Annual Meeting of American Heart Association, 3734, 1997 (DuPont-Merc); M. Friedlander et al., Conference of American IBC, September 11, 1997 (The Scripps Research Institute); W. S. Westlin, Conference of American IBC, February 23, 1998 (Searle); M. W. Lark et al., The 2nd Joint Conference of The American Society for Bone and Mineral Research and International Bone and Mineral Society, T064, 1998 (SmithKline Beecham); R. K. Keenan et al., Bioorg. Med. Chem. Lett., 8, 3171, 1998 (SmithKline Beecham); C. P. Carron et al., Cancer Res., 58, 1930, 1998 (Searle); W. H. Miller et al., Bioorg. Med. Chem. Lett., 9, 1807, 1999 (SmithKline Beecham); and S. A. Mousa et al., The 17th International Congress on Thrombosis and Hemostasis, 228, 1999 (DuPont Pharmaceuticals)). These documents, however, disclose neither the fact that integrin $\alpha_v\beta_3$ antagonists inhibit the expansion of the focus of myocardial infarction nor scientific data demonstrating the effectiveness of integrin $\alpha_v\beta_3$ antagonists for reperfusion injury.

Further, Y. Okada et al., Am. J. Pathol., 149, 37, (1996) and M. Gawaz et al., Circulation, 96, 1809, (1997) disclose the relationship of the integrin $\alpha_v\beta_3$ expression or the antiplatelet activity with reperfusion injury, but neither the fact of therapy of reperfusion injury nor the developing mechanism of reperfusion injury.

## SUMMARY OF THE INVENTION

An object of the present invention is to provide a medicament that is clinically effective for the treatment or prevention of reperfusion injury.

The present inventors have made extensive and intensive studies on action mechanisms of the inhibition of adhesion and invasion of leukocytes and the inhibition of minimal thrombus (M. Gawaz et al., Circulation, 96, 1809, (1997)) and edema (H. Tsukada et al., Circ. Res., 77, 651, (1995)). As a result, the present inventors have obtained important scientific finding for the first time that compounds, which antagonize integrin $\alpha_v\beta_3$ as adhesive molecules, inhibit the adhesion and invasion of leukocytes and this in turn inhibits reperfusion injury (Pharmacological Test Examples 2 and 6). It was further found that, in an assay system using rats, guinea pigs, and dogs, integrin $\alpha_v\beta_3$ antagonists actually significantly reduce the size of myocardial infarct in an ischemia-reperfusion model through this action mechanism (Pharmacological Test Examples 3, 4, and 5). The present invention is based on such finding.

An agent for use in the treatment or prevention of reperfusion injury according to the present invention comprises an integrin $\alpha_v\beta_3$ antagonist, a pharmaceutically acceptable salt thereof, or a solvate thereof as active ingredient.

## DETAILED DESCRIPTION OF THE INVENTION

### Therapeutic or prophylactic agent for reperfusion injury

It is known that the activation of tissue injury factors caused by ischemia of tissues and subsequent rapid recanalization of blood flow themselves as well as microcirculation injuries including no-reflow phenomenon cause necrosis of cardiac tissues, increase myocardial infarct size, and consequently deteriorate cardiac function (H. Ito et al., Circulation, 93, 223, (1996), K. Matsumura et al., Circulation, 97, 795, (1998)). In fact, cardiac tissue injury and microcirculation injury is considered to be primarily caused by adhesion or invasion of leukocytes into vascular walls and cardiac tissues (L. H. Manciet et al., Am. J. Physiol., 266, H1541, (1994)) and secondarily caused by minimal thrombus and edema (M. Gawaz et al., Circulation, 96, 1809, (1997), H. Tsukada et al., Circ. Res., 77, 651, (1995)).

The present inventors have found that integrin $\alpha_v\beta_3$ antagonists inhibit the adhesion of rat macrophages to vitronectin (Pharmacological Test Example 2).

The present inventors have also found that integrin $\alpha_v\beta_3$ antagonists inhibit adhesion of leukocytes to activated vascular endothelial cells (Pharmacological Test Example 6).

These results demonstrate that:

- vitronectin receptors are involved in adhesion and invasion of leukocytes and the occurrence of minimal thrombus and edema which are causative of reperfusion injury; and
- reperfusion injury can be treated and prevented by inhibiting adhesion and invasion of leukocytes and the occurrence of minimal thrombus and edema by integrin $\alpha_v\beta_3$ antagonists.

Integrin $\alpha_v\beta_3$ antagonists actually inhibited infarct size in myocardial ischemia-reperfusion models in rats, hamsters, and dogs (Pharmacological Test Examples 3, 4, and 5).

Accordingly, integrin $\alpha_v\beta_3$ antagonists are effective for the treatment or prevention of reperfusion injury.

More specifically, the reperfusion injury may be involved in recanalization therapy of ischemic heart diseases, reperfusion injury involved in recanalization therapy of cerebral ischemic diseases, reperfusion injury involved in organ transplantation or blood transplantation, or reperfusion injury involved in the use of artificial organs.

According to the present invention, there is provided use of an integrin $\alpha_v\beta_3$ antagonist, a pharmaceutically acceptable salt thereof, or a solvate thereof, for the manufacture of an agent for use in the treatment or prevention of reperfusion injury.

Further, according to the present invention, there is provided a method for treating or preventing reperfusion injury, comprising the step of administering an effective amount of an integrin $\alpha_v\beta_3$ antagonist, a pharmaceutically acceptable salt thereof, or a solvate thereof together with a pharmaceutically acceptable carrier, to a mammal.

Integrin $\alpha_v\beta_3$ antagonist

The term "integrin $\alpha_v\beta_3$ antagonist" as used herein means a compound which inhibits the binding between integrin $\alpha_v\beta_3$ and a ligand thereof, for example, vitronectin, fibrinogen, or von Willebrand factor.

Whether or not a compound has integrin $\alpha_v\beta_3$ antagonistic activity may be determined by assaying the antagonistic action by integrin $\alpha_v\beta_3$ binding assay as described, for example, in Pharmacological Test Example 1 in WO 99/38849. For example, compounds having $IC_{50}$ of not more than 10 nM, preferably not more than 1.0 nM, may be regarded as "integrin $\alpha_v\beta_3$ antagonist."

The "integrin $\alpha_v\beta_3$ antagonist" is not particularly limited so far as the compound can inhibit the binding between integrin $\alpha_v\beta_3$ and a ligand thereof. Examples of integrin $\alpha_v\beta_3$ antagonists include low-molecular compounds, for example, compounds having a molecular weight of 200 to 850, antibodies, cyclic peptides, and poison of snakes.

The "integrin $\alpha_v\beta_3$ antagonist" may be selected, for example, from those described in the following documents: WO 95/32710 (Merck); WO 96/37492 (DuPont-Merc); WO 97/01540 (SmithKline Beecham); WO 97/08145 (Searle); WO 97/23451 (Merck); WO 97/23480 (DuPont-Merc); WO 97/24119 (SKB); WO 97/26250 (Merck); WO 97/33887 (DuPont-Merc); WO 97/36858 (Searle); WO 97/36859 (Searle); WO 97/36860 (Searle); WO 97/36861 (Searle); WO 97/36862 (Searle); WO 97/24336 (SmithKline Beecham); WO 97/37655 (Merck); WO 98/08840 (Merck); WO 98/18460 (Merck); WO 98/25892 (Lilly); WO 98/30542 (SmithKline Beecham); WO 98/31359 (Merck); WO 98/35949 (Merck); WO 98/43962 (DuPont-Merc); WO 98/46220 (Merck); WO 99/05107 (SmithKline Beecham); WO 99/06049 (SmithKline Beecham); WO 99/11626 (SmithKline Beecham); WO 99/15170 (SmithKline Beecham); WO 99/15178 (SmithKline Beecham); WO 99/15506 (Hoechst Marion Roussel); WO 99/15507 (Hoechst Marion Roussel); WO 99/15508 (SmithKline Beecham); WO 99/30709 (Merck); WO 99/30713 (Merck); WO 99/31061 (Merck); WO 99/31099 (Merck); WO 99/32457 (Hoechst Marion Roussel); WO 99/33798 (Yamanouchi Pharmaceutical Co., Ltd.); WO 99/37621 (Hoechst Marion Roussel); WO 99/38849 (Meiji Seika Kaisha Co., Ltd.); WO 99/44994 (Searle), WO 99/45927 (SmithKline Beecham); WO 99/50249 (DuPont-Merc); WO 99/52872 (Meiji Seika Kaisha Co., Ltd.); WO 99/52879 (American Home Products); US 5843906 (Searle); US 5852210 (Searle); EP 796855 (Hoechst); EP 820988 (Hoechst); EP 820991 (Hoechst); EP 853084 (Hoechst); EP 928790 (Hoffmann-La Roche Ltd.); EP 928793 (Hoffmann-La Roche Ltd.); GB 2326609 (Merck); GB 2327672 (Merck); PCT/JP00/05177(Meiji Seika Kaisha Co., Ltd.); PCT/JP00/07031 (Meiji Seika Kaisha Co., Ltd.); and PCT/JP00/07033 (Meiji Seika Kaisha Co., Ltd.).

Among them, compounds described in WO 99/52872 (Meiji Seika Kaisha Co., Ltd.), PCT/JP00/07033 (Meiji Seika Kaisha Co., Ltd.), and WO 95/32710 (Merck) are preferred.

Preferred integrin $\alpha_v\beta_3$ antagonists are compounds represented by formula (I):

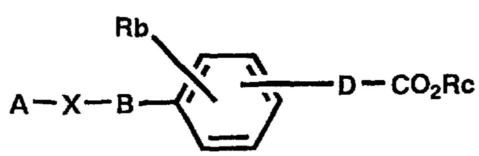

(I)

wherein

A represents

a hydrogen atom,

a saturated or unsaturated five- to seven-membered heterocyclic group containing at least one nitrogen atom, which is optionally condensed with another saturated or unsaturated five- to seven-membered carbocyclic ring or heterocyclic ring to form a bicyclic group, wherein the heterocyclic and bicyclic groups are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl and the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl,

or a group represented by formula

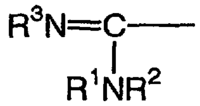

or

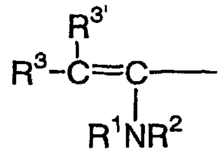

wherein $R^1$, $R^2$, $R^3$, and $R^{3'}$, which may be the same or different, represent a hydrogen atom, $C_{1-6}$ alkyl, $C_{1-6}$ alkoxy, hydroxyl, $C_{1-6}$ alkoxycarbonyl, $C_{2-6}$ alkenyl, $C_{2-6}$ alkynyl, aralkyl, or nitrile, or $R^1$ and $R^2$ may together form group $-(CH_2)i-$, wherein i represents 4 or 5, or group $-(CH_2)_2-O-(CH_2)_2-$, and the $C_{1-6}$ alkyl, $C_{1-6}$ alkoxy, hydroxyl, $C_{1-6}$ alkoxycarbonyl, $C_{2-6}$ alkenyl, $C_{2-6}$ alkynyl, and aralkyl groups are optionally substituted by a halogen atom, $C_{1-6}$ alkoxy, amino, $C_{1-6}$ alkyl, or hydroxyl;

X represents -NH-, $-CH_2-$, or a bond, preferably -NH-;

B represents

$C_{1-6}$ alkylene chain optionally containing an unsaturated bond,

$C_{1-6}$ alkylene chain optionally containing an unsaturated bond wherein one or two methylene portions in the chain may be replaced by group(s) or atom(s) selected from the group consisting of an oxygen atom, a sulfur atom, an optionally substituted imino group, or a carbonyl group at any position(s),

a piperazine ring,

a pyrrolidine ring,

a piperidine ring,

a group represented by formula $-C_6H_4CONH-$,

a group represented by formula $-C_6H_4NHCO-$,

a group represented by formula $-C_6H_4SO_2NH-$,

a group represented by formula $-C_6H_4NHSO_2-$,

a pyridine ring,

a pyrimidine ring, or

a triazine ring, wherein

the piperazine ring, the pyrrolidine ring, the piperidine ring, $-C_6H_4CONH-$, $-C_6H_4NHCO-$, $-C_6H_4SO_2NH-$, $-C_6H_4NHSO_2-$, pyridine ring, pyrimidine ring, or the triazine ring is optionally substituted by $C_{1-6}$ alkyl, $C_{1-6}$ alkoxy, a halogen atom, amino, nitro, hydroxyl, or an oxygen atom and the $C_{1-6}$ alkyl and $C_{1-6}$ alkoxy groups are optionally substituted by a halogen atom, $C_{1-6}$ alkoxy, amino, or hydroxyl;

D represents

a group represented by formula $-CONR^4CHR^5CHR^6-$ wherein $R^4$ represents a hydrogen atom, $C_{1-6}$ alkyl, $C_{2-6}$ alkenyl, $C_{2-6}$ alkynyl, or aralkyl and the $C_{1-6}$ alkyl, $C_{2-6}$ alkenyl, $C_{2-6}$ alkynyl, and aralkyl groups are optionally substituted by a halogen atom, $C_{1-6}$ alkoxy, amino, or hydroxyl; $R^5$ and $R^6$, which may be the same or different, represent a hydrogen atom, a group represented by formula $-NR^7COR^8$ wherein $R^7$ represents a hydrogen atom, $C_{1-6}$ straight chain alkyl, or $C_{3-6}$ branched chain alkyl and $R^8$ represents a hydrogen atom, $C_{1-6}$ straight chain alkyl, $C_{2-6}$ alkenyl, $C_{3-6}$ branched chain alkyl, $C_{7-10}$ aralkyl, a five- to ten-membered monocyclic or bicyclic carbocyclic group, preferably a five- to seven-membered monocyclic carbocyclic group, or a five- to ten-membered monocyclic or bicyclic heterocyclic group, preferably a five- to seven-membered monocyclic heterocyclic group and the straight chain alkyl, alkenyl, and branched chain alkyl groups optionally represented by $R^8$ are optionally substituted by $C_{1-6}$ alkyl, $C_{1-6}$ alkoxy, $C_{1-6}$ alkoxycarbonyl, aralkyl, amino, or hydroxyl and the aralkyl, carbocyclic, and heterocyclic groups optionally represented by $R^8$ may be substituted by hydroxyl, amino, azido, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl; a group represented by formula $-NR^7CO_2R^8$ wherein $R^7$ is as defined above and $R^8$ is as defined above; a group represented by formula $-NR^7SO_2R^8$ wherein $R^7$ is as defined above and $R^8$ is as defined above; a group represented by formula $-NR^7R^8$ wherein $R^7$ is as defined above and $R^8$ is as defined above; a group represented by formula $-OR^8$ wherein $R^8$ is as defined above; or a group $-R^8$ wherein $R^8$ is as defined above,

a group represented by formula $-NR^4COCHR^5CHR^6-$ wherein $R^4$, $R^5$, and $R^6$ are as defined above,

a group represented by formula $-CH_2NR^4HR^5CHR^6-$ wherein $R^4$, $R^5$, and $R^6$ are as defined above,

a group represented by formula $-CHR^5CHR^6-$ wherein $R^4$, $R^5$, and $R^6$ are as defined above,

a cyclopropyl ring optionally substituted by hydroxyl, amino, azido, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl, or

vinylene optionally substituted by $C_{1-6}$ alkyl, $C_{1-6}$ alkylcarbonyl, $C_{1-6}$ alkoxycarbonyl, carboxyl, a halogen atom,

azido, nitro, or cyano group,

$R^b$ represents

a hydrogen atom,

a halogen atom,

nitro,

amino,

alkylamino,

dialkylamino,

cyano,

hydroxyl,

thiol optionally substituted by $C_{1-2}$ alkyl or phenyl,

$C_{1-6}$ alkyl optionally substituted by $C_{1-6}$ alkyl, $C_{1-6}$ alkoxy, $C_{1-6}$ alkoxycarbonyl, aralkyl, amino, or hydroxyl, or

$C_{1-6}$ alkoxy optionally substituted by $C_{1-6}$ alkyl, $C_{1-6}$ alkoxy, $C_{1-6}$ alkoxycarbonyl, aralkyl, amino, or hydroxyl; and

$R^c$ represents a hydrogen atom, $C_{1-6}$ straight chain alkyl, $C_{3-6}$ branched chain alkyl, or $C_{7-10}$ aralkyl and this aralkyl group is optionally substituted by hydroxyl, amino, azido, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl.

The five- to ten-membered monocyclic or bicyclic carbocyclic group or heterocyclic group optionally represented by $R^8$ preferably represents a five- to seven-membered monocyclic carbocyclic or heterocyclic group. Examples of this five- to seven-membered monocyclic carbocyclic or heterocyclic group include cyclopentyl, cyclohexyl, cycloheptyl, phenyl, pyridyl, pyrimidyl, morpholinyl, piperazinyl, piperidinyl, pyrrolidinyl, thienyl, and furyl.

The term "$C_{1-6}$ alkyl" and the term "$C_{1-6}$ alkoxy" as used herein as a group or a part of a group respectively mean straight chain, branched chain, or cyclic alkyl and alkoxy having 1 to 6, preferably 1 to 4 carbon atoms, unless otherwise specified.

The term "$C_{2-6}$ alkenyl" and the term "$C_{2-6}$ alkynyl" as used herein as a group or a part of a group respectively mean straight chain, branched chain, or cyclic alkenyl and alkynyl having 2 to 6, preferably 2 to 4 carbon atoms, unless otherwise specified.

Examples of $C_{1-6}$ alkyl include methyl, ethyl, n-propyl, isopropyl, cyclopropyl, cyclopropylmethyl, n-butyl, i-butyl, s-butyl, t-butyl, n-pentyl, cyclopentyl, n-hexyl, and cyclohexyl.

Examples of $C_{1-6}$ alkoxy include methoxy, ethoxy, n-propoxy, i-propoxy, n-butoxy, i-butoxy, s-butoxy, and t-butoxy.

Examples of $C_{2-6}$ alkenyl include allyl.

Examples of $C_{2-6}$ alkynyl include 2-propynyl and ethynyl.

The term "halogen atom" as used herein means a chlorine, bromine, fluorine, or iodine atom.

The term "aralkyl" as a group or a part of a group means $C_{7-16}$ aralkyl and more specifically means $C_{1-6}$ alkyl, preferably $C_{1-4}$ alkyl, substituted by a saturated or unsaturated five- to seven-membered carbocyclic group or heterocyclic group. Examples of the aralkyl include benzyl, phenethyl, and naphthylmethyl.

The "saturated or unsaturated five- to seven-membered heterocyclic group containing at least one nitrogen atom" represented by A is preferably a saturated or unsaturated five- to seven-membered heterocyclic group containing one or two nitrogen atoms, more preferably a saturated or unsaturated five- or six-membered heterocyclic group containing one or two nitrogen atoms.

The "bicyclic group" represented by A is preferably a nine- or ten-membered heterocyclic group, more preferably a nine- or ten-membered heterocyclic group containing two or three nitrogen atoms.

In group A, $R^2$ and $R^{3'}$ preferably represent a hydrogen atom.

A preferably represents a group of formula

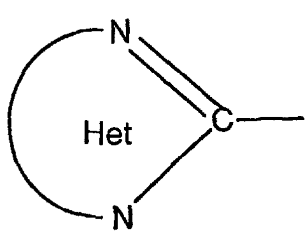

or

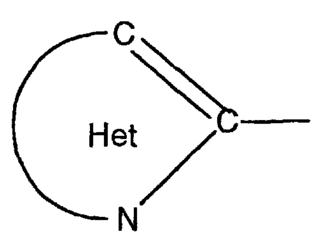

wherein

Het represents a saturated or unsaturated five- to seven-membered heterocyclic group containing at least one nitrogen atom and this heterocyclic group is optionally condensed with another saturated or unsaturated five- to seven-membered carbocyclic ring or heterocyclic ring to form a bicyclic group and the heterocyclic and bicyclic groups are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl in which the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl.

Preferably, A represents a group of formula

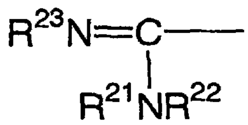

or

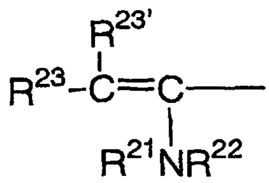

wherein

$R^{21}$, $R^{22}$, $R^{23}$, and $R^{23'}$, which may be the same or different, represent a hydrogen atom, $C_{1-6}$ alkyl, $C_{1-6}$ alkoxy, hydroxyl, $C_{1-6}$ alkoxycarbonyl, $C_{2-6}$ alkenyl, or aralkyl and the $C_{1-6}$ alkyl, $C_{1-6}$ alkoxy, hydroxyl, $C_{1-6}$ alkoxycarbonyl, $C_{2-6}$ alkenyl, and aralkyl groups are optionally substituted by a halogen atom, $C_{1-6}$ alkoxy, amino, $C_{1-6}$ alkyl, or hydroxyl, or

$R^{21}$ and $R^{23}$ may together form

group $-(CH_2)_4-$,

group $-(CH_2)_3-$,

group $-CHR^{24}CH_2CH_2-$ wherein $R^{24}$ represents $C_{1-6}$ alkyl, amino, or hydroxyl and the amino and hydroxyl groups are optionally substituted by $C_{1-6}$ alkyl, $C_{1-6}$ alkoxycarbonyl, aralkyl, or aralkyloxycarbonyl,

group $-CH_2CHR^{24}CH_2-$ wherein $R^{24}$ is as defined above,

group $-CH_2CH_2-$,

group $-CHR^{24}CH_2-$ wherein $R^{24}$ is as defined above,

group $-CR^{25}=CR^{26}-$ wherein $R^{25}$ and $R^{26}$, which may be the same or different, represent a hydrogen atom or $C_{1-6}$ alkyl, or $R^{25}$ and $R^{26}$ may together form -CH=CH-CH=CH-, $-CR^{24}=CH-CH=CH-$ wherein $R^{24}$ is as defined above, $-CH=CR^{24}-CH=CH-$ wherein $R^{24}$ is as defined above, -N=CH-CH=CH-, or -CH=N-CH=CH-, or

$R^{21}$ and $R^{23}$ may together form

=CH-CH=CH-,

=CH-CH=N-, or

=CH-N=CH-, and

$R^{22}$ may represent a single bond between $R^{21}$ and the nitrogen atom attached to $R^{21}$.

$R^{22}$ and $R^{23'}$ preferably represent a hydrogen atom.

Preferably, A represents a hydrogen atom, amidino, imidazolyl, imidazolidinyl, thiazolyl, thiazolidinyl, pyridinyl, pyrimidinyl, tetrahydropyrimidinyl, triazinyl, benzimidazolyl, or azabenzimidazolyl. The groups other than a hydrogen atom are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl, and the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl.

X preferably represents -NH-.

An example of the $C_{1-6}$ alkylene chain optionally represented by B, a part of which may be replaced, is $*-C_{1-5}$ alkylene-O-, more specifically $*-CH_2CH_2O-$, wherein the bond having * represents a bond between B and X and the other bond represents a bond between B and the phenylene portion; the same will apply hereinafter.

An example of the piperazine ring optionally represented by B is

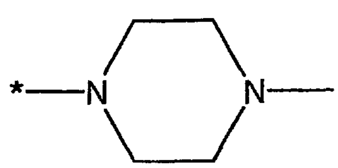.

An example of the pyrrolidine ring optionally represented by B is

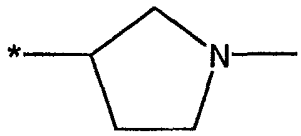

.

An example of the piperidine ring optionally represented by B is

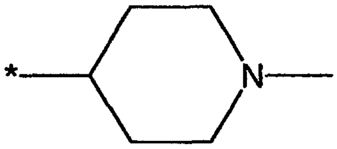

or

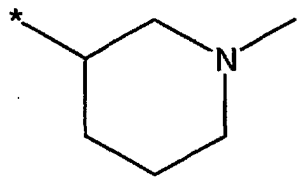

.

An example of $-C_6H_4CONH-$ optionally represented by B is

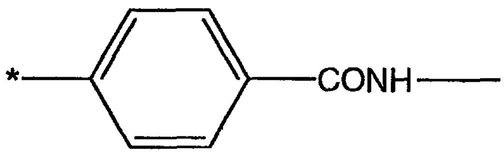

.

An example of $-C_6H_4NHCO-$ optionally represented by B is

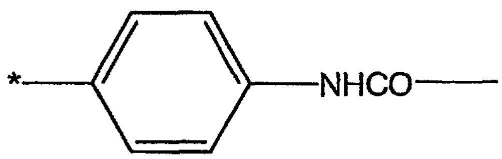

.

An example of $-C_6H_4SO_2NH-$ optionally represented by B is

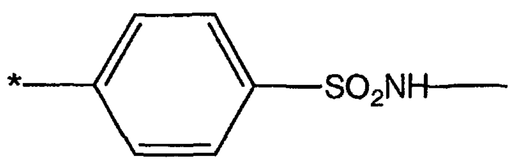

.

An example of $-C_6H_4NHSO_2-$ optionally represented by B is

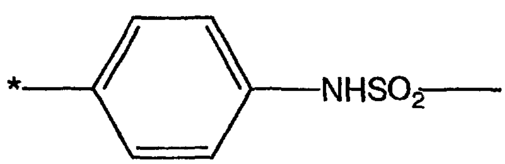

.

An example of the pyridine ring optionally represented by B is

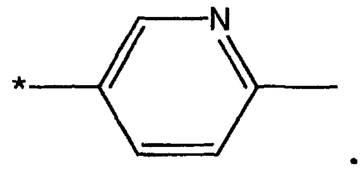

An example of the pyrimidine ring optionally represented by B is

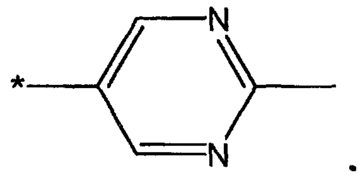

An example of the triazine ring optionally represented by B is

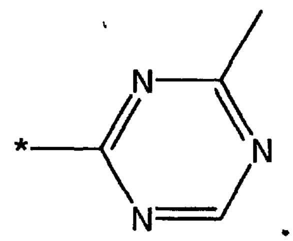

An example of the substituted piperazine ring optionally represented by B is

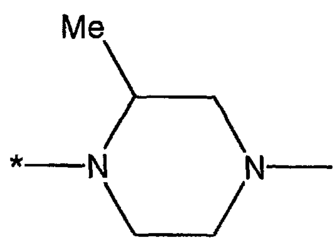

wherein Me represents methyl; the same will apply hereinafter.

Examples of the substituted piperidine ring optionally represented by B include

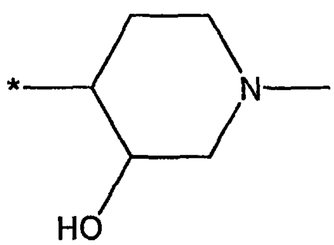

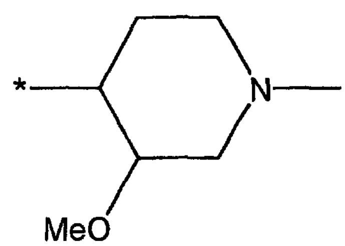

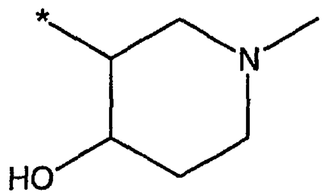
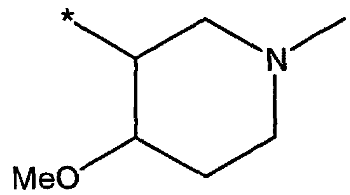

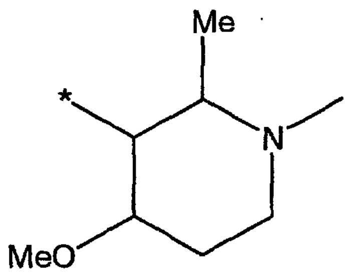
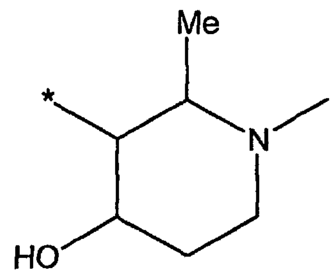

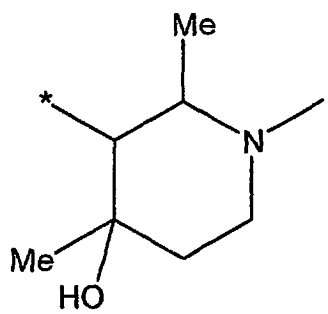
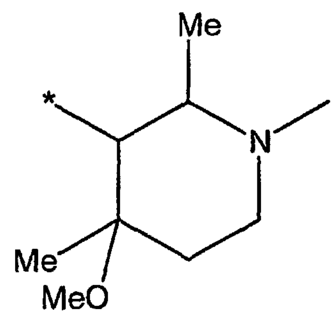

D preferably represents group $-CONR^4CHR^5CHR^6-$, more preferably group $-CONHCH_2C(-NHSO_2E)-$ wherein E is as defined in formula (II).

Integrin $\alpha_v\beta_3$ antagonists may be compounds represented by formula (II):

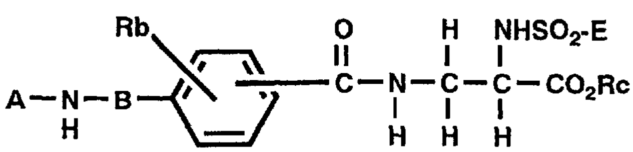 (II)

wherein

A represents a hydrogen atom, amidino, imidazolyl, imidazolidinyl, thiazolyl, thiazolidinyl, pyridinyl, pyrimidinyl, tetrahydropyrimidinyl, triazinyl, benzimidazolyl, or azabenzimidazolyl, and

the groups other than a hydrogen atom are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl and the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl;

B represents

$C_{1-6}$ alkylene chain optionally containing an unsaturated bond,

$C_{1-6}$ alkylene chain optionally containing an unsaturated bond wherein one or two methylene portions in the chain may be replaced by group(s) or atom(s) selected from the group consisting of an oxygen atom, a sulfur atom, an optionally substituted imino group, or a carbonyl group at any position(s),

a piperazine ring,

a pyrrolidine ring,
a piperidine ring,
a group represented by formula -$C_6H_4CONH$-,
a group represented by formula -$C_6H_4NHCO$-,
a group represented by formula -$C_6H_4SO_2NH$-,
a group represented by formula -$C_6H_4NHSO_2$-,
a pyridine ring,
a pyrimidine ring, or
a triazine ring, wherein

the piperazine ring, the pyrrolidine ring, the piperidine ring, -$C_6H_4CONH$-, -$C_6H_4NHCO$-, -$C_6H_4SO_2NH$-, -$C_6H_4NHSO_2$-, the pyridine ring, the pyrimidine ring, or the triazine ring is optionally substituted by $C_{1-6}$ alkyl, $C_{1-6}$ alkoxy, a halogen atom, amino, nitro, hydroxyl, or an oxygen atom and the $C_{1-6}$ alkyl and $C_{1-6}$ alkoxy groups are optionally substituted by a halogen atom, $C_{1-6}$ alkoxy, amino, or hydroxyl;

E represetns $C_{1-6}$ straight chain alkyl, $C_{2-6}$ alkenyl, $C_{3-6}$ branched chain alkyl, $C_{7-10}$ aralkyl, a five- to ten-membered monocyclic or bicyclic carbocyclic group, preferably a five- to seven-membered monocyclic carbocyclic group, or a five- to ten-membered monocyclic or bicyclic heterocyclic group, preferably a five- to seven-membered monocyclic heterocyclic group, wherein the straight chain alkyl, alkenyl, and branched chain alkyl groups optionally represented by E are optoinally substituted by $C_{1-6}$ alkyl, $C_{1-6}$ alkoxy, $C_{1-6}$ alkoxycarbonyl, aralkyl, amino, or hydroxyl, the aralkyl, carbocyclic, and heterocyclic groups optionally represented by E are optionally substituted by hydroxyl, amino, azido, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl, and examples of the five- to seven-membered monocyclic carbocyclic or heterocyclic group optionally represented by E include cyclopentyl, cyclohexyl, cycloheptyl, phenyl, pyridyl, pyrimidyl, morpholinyl, piperazinyl, piperidinyl, pyrrolidinyl, thienyl, and furyl;

$R^b$ represents
a hydrogen atom,
a halogen atom,
nitro,
amino,
alkylamino,
dialkylamino,
cyano,
hydroxyl,
thiol optionally substituted by $C_{1-2}$ alkyl or phenyl,
$C_{1-6}$ alkyl optionally substituted by $C_{1-6}$ alkyl, $C_{1-6}$ alkoxy, $C_{1-6}$ alkoxycarbonyl, aralkyl, amino, or hydroxyl, or
$C_{1-6}$ alkoxy optionally substituted by $C_{1-6}$ alkyl, $C_{1-6}$ alkoxy, $C_{1-6}$ alkoxycarbonyl, aralkyl, amino, or hydroxyl; and

$R^c$ represents a hydrogen atom, $C_{1-6}$ straight chain alkyl, $C_{3-6}$ branched chain alkyl, or $C_{7-10}$ aralkyl and this aralkyl group is optionally substituted by hydroxyl, amino, azide, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl.

The integrin $\alpha_v\beta_3$ antagonists may be compounds represented by formula (III):

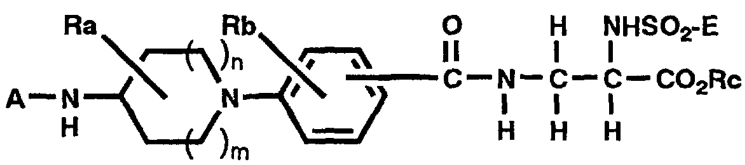

(III)

wherein

A represents a hydrogen atom, amidino, imidazolyl, imidazolidinyl, thiazolyl, thiazolidinyl, pyridinyl, pyrimidinyl, tetrahydropyrimidinyl, triazinyl, benzimidazolyl, or azabenzimidazolyl, and

the groups other than a hydrogen atom are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl and the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl;

E represents $C_{1-6}$ straight chain alkyl, $C_{2-6}$ alkenyl, $C_{3-6}$ branched chain alkyl, $C_{7-10}$ aralkyl, a five- to ten-membered monocyclic or bicyclic carbocyclic group, preferably a five- to seven-membered monocyclic carbocyclic group, or a five- to ten-membered monocyclic or bicyclic heterocyclic group, preferably a five- to seven-membered monocyclic

heterocyclic group, wherein the straight chain alkyl, alkenyl, and branched chain alkyl groups optionally represented by E are optoinally substituted by $C_{1-6}$ alkyl, $C_{1-6}$ alkoxy, $C_{1-6}$ alkoxycarbonyl, aralkyl, amino, or hydroxyl, and the aralkyl, carbocyclic, and heterocyclic groups optionally represented by E are optionally substituted by hydroxyl, amino, azido, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl;

$R^a$ represents
a hydrogen atom,
hydroxyl,
thiol optionally substituted by $C_{1-2}$ alkyl or phenyl,
$C_{1-6}$ alkyl,
$C_{1-6}$ alkoxy,
amino,
alkylamino,
dialkylamino,
a halogen atom, or
=O;

$R^b$ represents
a hydrogen atom,
a halogen atom,
nitro,
amino,
alkylamino,
dialkylamino,
cyano,
hydroxyl,
thiol optionally substituted by $C_{1-2}$ alkyl or phenyl,
$C_{1-6}$ alkyl optionally substituted by $C_{1-6}$ alkyl, $C_{1-6}$ alkoxy, $C_{1-6}$ alkoxycarbonyl, aralkyl, amino, or hydroxyl, or
$C_{1-6}$ alkoxy optionally substituted by $C_{1-6}$ alkyl, $C_{1-6}$ alkoxy, $C_{1-6}$ alkoxycarbonyl, aralkyl, amino, or hydroxyl;

$R^c$ represents a hydrogen atom, $C_{1-6}$ straight chain alkyl, $C_{3-6}$ branched chain alkyl, or $C_{7-10}$ aralkyl and this aralkyl group is optionally substituted by hydroxyl, amino, azido, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl;

m is 0, 1, or 2; and
n is 0, 1, or 2.

The integrin $\alpha_v\beta_3$ antagonist may be a compound selected from the following compounds:

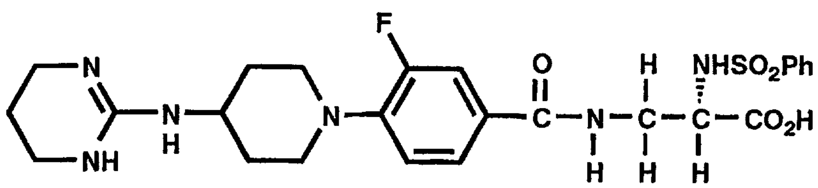

(IV)

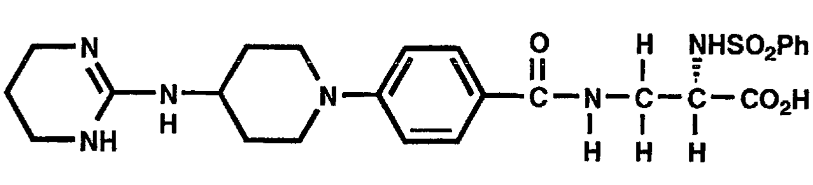

(V)

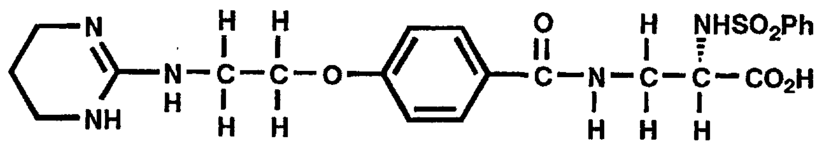

(VI)

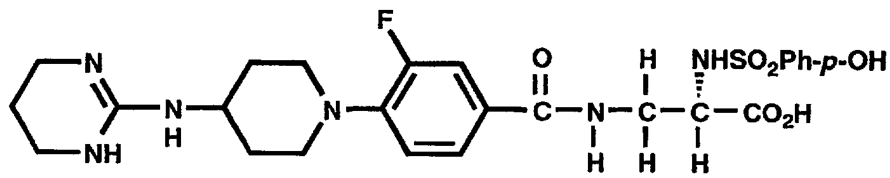

(VII)

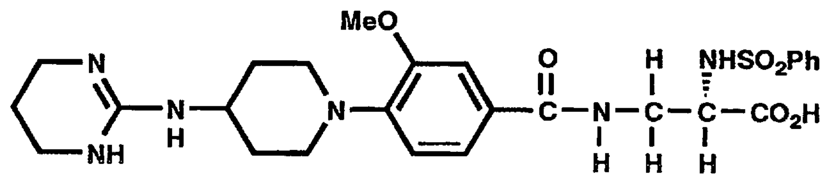

(VIII)

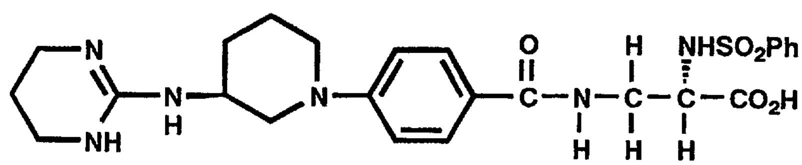

(IX)

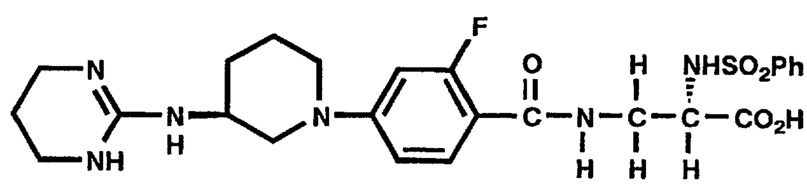

(X)

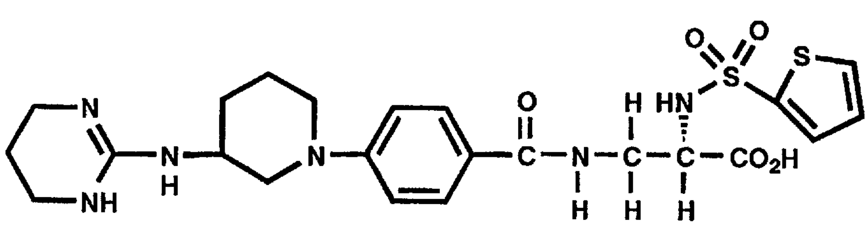

(XI)

$IC_{50}$ values in integrin $\alpha_v\beta_3$ binding assay (Pharmacological Example 1 in WO 99/38849) for the compounds represented by formula (IV), formula (V), formula (VI), formula (VII), formula (VIII), formula (IX), formula (X), and formula (XI) were 0.20 nM, 1.2 nM, 0.39 nM, 0.066 nM, 0.14 nM, 0.53 nM, 0.16 nM, and 0.14 nM, respectively.

The compounds represented by formula (I), formula (II), and formula (III), wherein B represents a piperazine ring, a pyrrolidine ring, a piperidine ring, pyridine ring, a pyrimidine ring, or a triazine ring, and the compounds represented by formula (IV), formula (V), formula (VII), formula (VIII), formula (IX), formula (X), and formula (XI) may be produced by reacting a compound corresponding to group B, for example, 3-hydroxypiperidine or 4-hydroxypiperidine,

with optionally substituted 4-fluorobenzoic acid, converting hydroxyl in the resultant compound to amino, introducing a compound corresponding to group A, for example, 2-bromopyrimidine, into the free primary amine, and constructing group -D-COOR$^c$ in the deprotected carboxyl group through an amide bond. These compounds may be produced, for example, according to the procedure described in WO 99/52872.

The compounds represented by formulae (I) and (II), wherein B represents $C_{1-6}$ alkylene chain and one or two methylene portions in the chain may be substituted by an oxygen atom or the like; a group represented by formula $-C_6H_4CONH-$; a group represented by formula $-C_6H_4NHCO-$; a group represented by formula $-C_6H_4SO_2NH-$; or a group represented by formula $-C_6H_4NHSO_2-$, and the compounds represented by formula (VI) may be produced, for example, according to the procedure described in WO 95/32710.

The compounds represented by formula (I), formula (II), and formula (III) having a 3-aminopiperidine structure or a 4-aminopiperidine structure and the compounds represented by formula (IV), formula (V), formula (VII), formula (VIII), formula (IX), formula (X), and formula (XI) may be produced, for example, by producing an intermediate represented by formula (XVI) or an intermediate represented by formula (XX) according to scheme 1 or scheme 2 below, then introducing a compound corresponding to group A, for example, 2-bromopyrimidine, into group Q, for example, deprotected amino, in intermediate (XVI) or intermediate (XX), and further constructing group -D-COOR$^c$ in group -COOR$^c$, for example, deprotected carboxyl, through an amide bond. The introduction of group A and the construction of group -D-COOR$^c$ may be carried out according to the procedure described in WO 99/52872.

Scheme 1

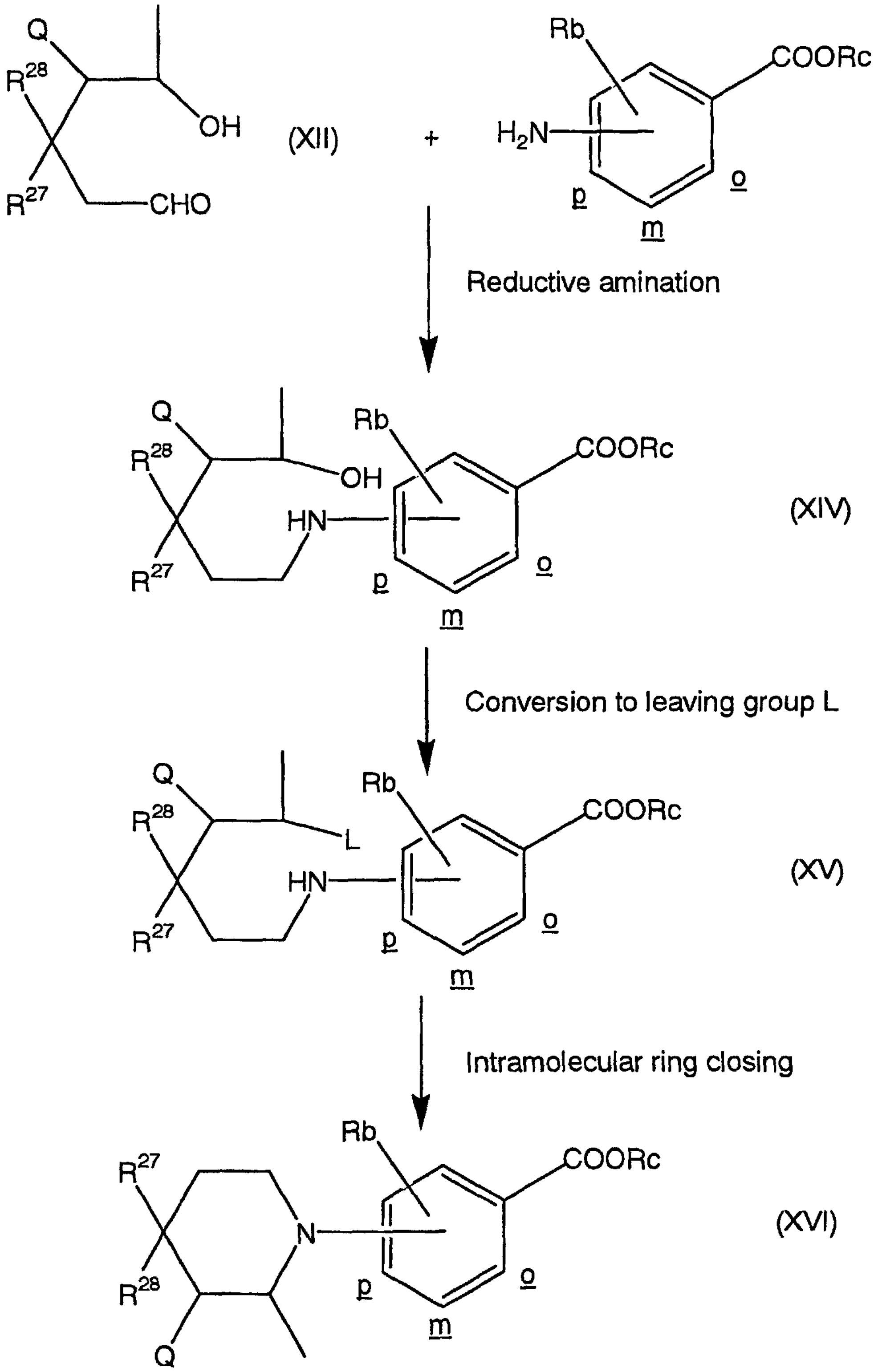


(XII) + (XIII)

(XIV)

(XV)

(XVI)

wherein $R^{27}$ and $R^{28}$, which may be the same or different, represent a hydrogen atom, hydroxyl, $C_{1-4}$ alkoxy, or $C_{1-4}$ alkyl; Q represents hydroxyl, azide, or optionally protected amino; L represents a leaving group; $R^b$ and $R^c$ are as defined in formula (I); and the nitrogen atom is attached to the ortho-position (o-position), meta-position (m-position), or para-position (p-position), preferably p-position, of phenyl.

A compound represented by formula (XIV) is first produced by reacting a compound represented by formula (XII) with a compound represented by formula (XIII).

The compound represented by formula (XII) (reducing sugar) can be produced by treating a naturally occurring organic compound, for example, midecamycin, erythromycin, or oleandomycin, with an acid in the presence of a lower alcohol to isolate the compound as a neutral sugar present in the structure, specifically a lower alkylglycoside of mycarose, cladinose, or oleandorose, and then, if necessary, converting a free hydroxyl group present at the 4-position to an azide group while performing steric inversion and, further, converting the compound to a reducing sugar by acid hydrolysis. The conversion to the azide group can be carried out according to a conventional method.

The compound represented by formula (XII) may be reacted with the compound represented by formula (XIII) by reductive amination. The reductive amination can be carried out in the presence of a hydride reagent, for example, a reagent such as sodium boron cyanohydride, sodium borohydride, sodium triacetoxy borohydride, or lithium borohydride, in a reaction solvent such as methanol, methylene chloride, dimethylformamide, or dichloroethane, or a mixed solvent composed of these solvents, at 0°C to 50°C, provided that the temperature should not exceed the boiling point of the reaction solvent, preferably at room temperature.

For some structures of naturally occurring saccharides used in the reaction, a certain reaction time is sometimes required for the formation of an imine which is one of intermediates. The timing of addition of the hydride reagent can be determined according to the substrate used. More specifically, the hydride reagent may be added immediately after the initiation of the reaction, or alternatively a method may be adopted wherein two substrates are dissolved in the reaction solvent and, after the elapse of a certain period of time after the dissolution of the substrates in the reaction solvent, for example, 24 hr after the dissolution of the substrates in the reaction solvent, the hydride reagent is added. Further, in this case, certain organic acids, for example, acetic acid, citric acid, formic acid, methanesulfonic acid, monochloroacetic acid, and monofluoroacetic acid, or inorganic acids, for example, hydrochloric acid or sulfuric acid, may be added, for example, from the viewpoint of improving the selectivity of the hydride reagent in the reaction.

Next, the compound represented by formula (XV) is produced by converting the free hydroxyl group (secondary hydroxyl group) in the compound represented by formula (XIV) to a leaving group L.

The conversion to leaving group L can be carried out by a conventional method, for example, by allowing methanesulfonyl chloride to act in methylene chloride at a temperature of 0°C to room temperature.

Leaving groups L include, for example, methanesulfonyloxy, p-toluenesulfonyloxy, benzenesulfonyloxy, trifluoromethanesulfonyloxy, and halogen atoms, for example, chlorine, bromine, and iodine atoms.

A compound represented by formula (XVI) can be produced by cyclizing the compound represented by formula (XV), in which a leaving group L has been constructed, by an intramolecular ring-closing reaction.

Bases usable in the intramolecular ring-closing reaction include tertiary organic bases, such as diisopropylethylamine, triethylamine, and tribenzylamine, and inorganic bases such as sodium carbonate and potassium carbonate. The use of organic bases is preferred from the viewpoint of the selectivity in reaction.

After the intramolecular ring-closing reaction, if necessary, the azide group may be reduced to an amino group. The reduction reaction of the azide group may be carried out according to a conventional method.

The reaction solvent used in the step of conversion to a leaving group and the step of an intramolecular ring-closing reaction is not particularly limited.

Scheme 2

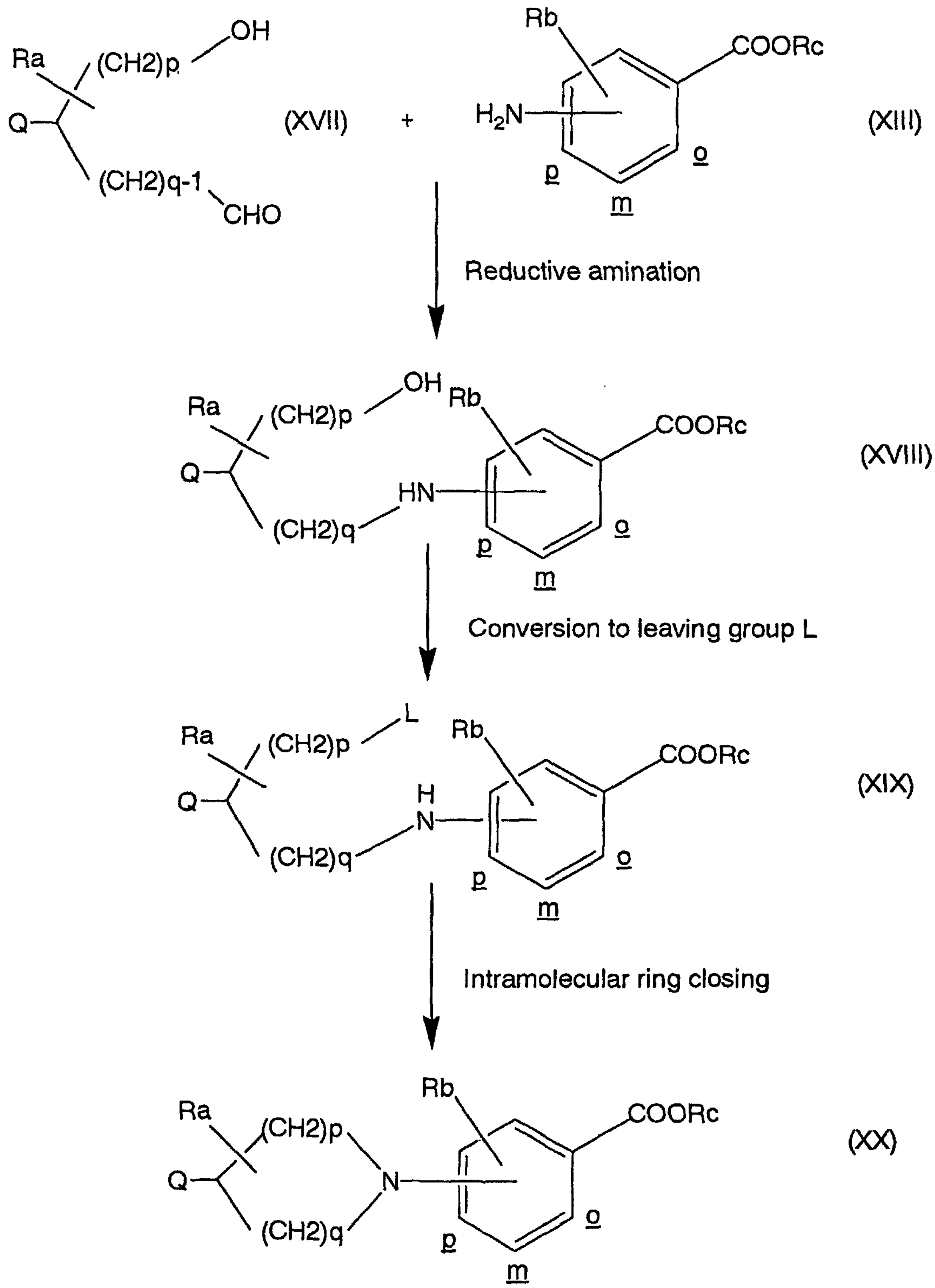


wherein Q represents hydroxyl, azide, or optionally protected amino; L represents a leaving group; $R^a$, $R^b$, and $R^c$ are as defined in the above formula; p is an integer of 0 to 3; q is an integer of 1 to 3; and the nitrogen atom is attached to the ortho-position (o-position), meta-position (m-position), or para-position (p-position), preferably m-position, of phenyl.

A compound represented by formula (XX) is first produced by reacting a compound represented by formula (XVII) with a compound represented by formula (XIII).

When a naturally occurring monosaccharide is used in the production process of the present invention, the monosaccharide is not required to be free aldehyde, that is, of ring-opened type, and may be of ring closure-type hemiacetal.

The compound represented by formula (XVII) may be reacted with the compound represented by formula (XIII) by reductive amination. The reductive amination can be carried out in the presence of a hydride reagent, for example, a reagent such as sodium boron cyanohydride, sodium borohydride, sodium triacetoxy borohydride, or lithium borohydride, in a reaction solvent such as methanol, methylene chloride, dimethylformamide, or dichloroethane, or a mixed solvent composed of these solvents, at 0°C to 50°C, provided that the temperature should not exceed the boiling point of the reaction solvent, preferably at room temperature.

For some structures of naturally occurring saccharides used in the reaction, a certain reaction time is sometimes required for the formation of an imine which is one of intermediates. The timing of addition of the hydride reagent can be determined according to the substrate used. More specifically, the hydride reagent may be added immediately after the initiation of the reaction, or alternatively a method may be adopted wherein two substrates are dissolved in the reaction solvent and, after the elapse of a certain period of time after the dissolution of the substrates in the reaction solvent, for example, 24 hr after the dissolution of the substrates in the reaction solvent, the hydride reagent is added. Further, in this case, certain organic acids, for example, acetic acid, citric acid, formic acid, methanesulfonic acid, monochloroacetic acid, and monofluoroacetic acid, or inorganic acids, for example, hydrochloric acid or sulfuric acid, may be added, for example, from the viewpoint of improving the selectivity of the hydride reagent in the reaction.

Next, the compound represented by formula (XIX) is produced by converting the primary hydroxyl group in the compound represented by formula (XVIII) to leaving group L.

The conversion to leaving group L can be carried out by a conventional method. For example, the primary hydroxyl group can be converted to a bromine atom by reacting the primary hydroxyl group with carbon tetrabromide and triphenylphosphine.

Leaving groups L include halogen atoms, for example, chlorine, bromine, and iodine atoms, and sulfonates, for example, methanesulfonyloxy, p-toluenesulfonyloxy, benzenesulfonyloxy, trifluoro-methanesulfonyloxy.

A compound represented by formula (XX) can be produced by subjecting the compound represented by formula (XIX), in which leaving group L has been constructed, to an intramolecular ring-closing reaction.

The intramolecular ring-closing reaction spontaneously proceeds upon the introduction of leaving group L.

Bases usable in the intramolecular ring-closing reaction include tertiary organic bases, such as diisopropylethylamine, triethylamine, and tribenzylamine, and inorganic bases such as sodium carbonate and potassium carbonate. The use of organic bases is preferred from the viewpoint of the selectivity in reaction.

After the intramolecular ring-closing reaction, if necessary, the functional group represented by Q may be further chemically converted. For example, the hydroxyl group may be converted to an azide group. Further, the azide group may be reduced to an amino group. The reduction reaction of the azide group may be carried out according to a conventional method.

The reaction solvent used in the step of conversion to a leaving group and the step of an intramolecular ring-closing reaction is not particularly limited.

In scheme 2, in formulae (XVII), (XVIII), (XIX), and (XX), preferably, p and q are 2, and $R^a$ and Q represent hydroxyl.

In scheme 2, the compound represented by formula (XVII) may be selected from the group consisting of pentose, hexose, and heptose, and derivatives thereof. Here derivatives include, for example, those wherein hydroxyls on monosaccharides have been converted to other functional groups, for example, $C_{1-4}$ alkyl, for example, methyl, $C_{1-4}$ alkoxy, for example, methoxy, azide, and amino.

The compound represented by formula (XVII) is more preferably 2-deoxy-D-ribose represented by formula (XVII') or derivatives thereof:

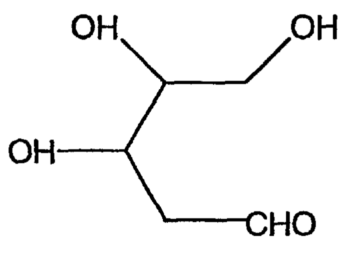

(XVII')

When the compound represented by formula (XVII) is 2-deoxy-D-ribose, the compounds represented by formulae (XVIII), (XIX), and (XX) in scheme 2 may be compounds represented by formulae (XVIII'), (XIX'), and (XX'), respectively.

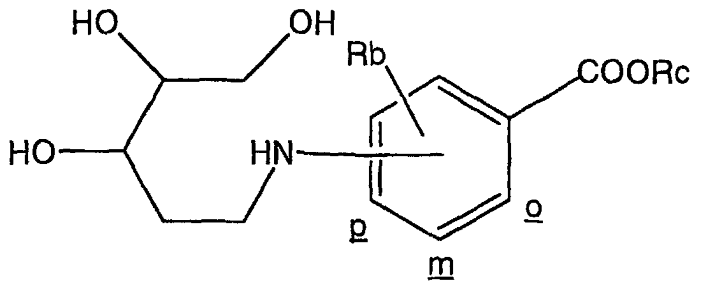

(XVIII')

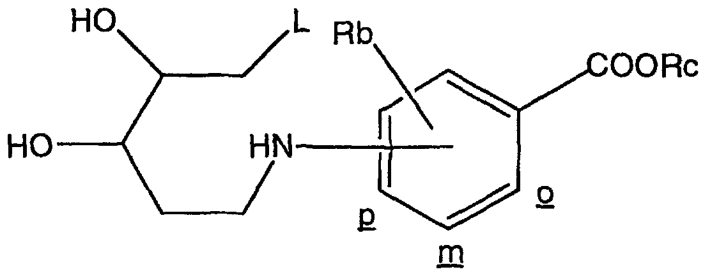

(XIX')

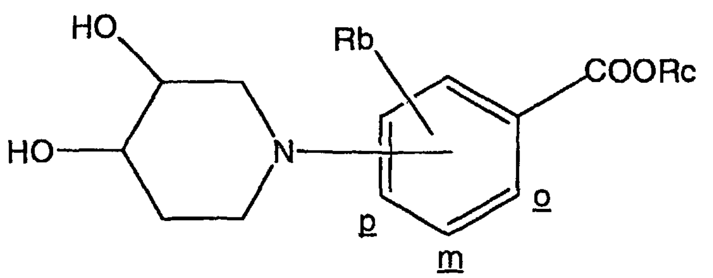

(XX')

wherein $R^b$, $R^c$, and L are as defined in formula (I); and the nitrogen atom is attached to the o-position, m-position, or p-position, preferably m-position, of phenyl.

The integrin $\alpha_v\beta_3$ antagonists may form pharmacologically acceptable salts thereof. Such salts include non-toxic salts. Preferred salts include: hydrohalogenic acid salts such as hydrochloride salts, hydrobromide salts, or hydroiodide salts; inorganic acid salts such as nitric acid salts, perchloric acid salts, sulfuric acid salts, or phosphoric acid salts; lower alkylsulfonic acid salts such as methanesulfonic acid salts, trifluoromethanesulfonic acid salts, or ethanesulfonic acid salts; arylsulfonic acid salts such as benzenesulfonic acid salts or p-toluenesulfonic acid salts; organic acid salts such as fumaric acid salts, succinic acid salts, citric acid salts, tartaric acid salts, oxalic acid salts, or maleic acid salts; amino acid salts such as glutamic acid salts or aspartic acid salts; alkali metal or alkaline earth metal salts such as sodium salts, potassium salts, and calcium salts; and organic alkali salts such as pyridine salts or triethylamine salts.

The integrin $\alpha_v\beta_3$ antagonists may form pharmacologically acceptable solvates without particular limitation, and examples of solvates include hydrates, alcoholates such as methanolate and ethanolate, and etherates such as tetrahydrofuran.

The pharmaceutical compositions comprising, as active ingredient, integrin $\alpha_v\beta_3$ antagonists, particularly the compounds represented by formulae (I) to (XI), pharmacologically acceptable salts thereof, or solvates thereof may be administered orally or parenterally by administration routes, for example, inhalation administration, rhinenchysis, instillation, subcutaneous administration, intravenous injection, intravenous drip infusion, intramuscular injection, rectal administration, or percutaneous administration, and thus may be formed into appropriate various dosage forms depending on oral or parenteral administration routes and administered to human and non-human animals.

They may be formulated into, for example, oral preparations, such as tablets, capsules, chewable preparations, granules, powders, pills, particulates, troches, syrups, emulsions, suspensions, or solutions; liquids for external use such as inhalants, nasal drops, or eye drops; patches; injections such as intravenous injections, intramuscular injections, or intravenous drip infusions; preparations for rectal administrations; oleaginous suppositories; water-soluble suppositories; and liniments such as ointments depending upon applications thereof. Further, a method may also be

adopted wherein liquid preparations, such as injections or drops, are provided, for example, as a lyophilized powdery pharmaceutical composition which, in use, is dissolved or suspended in water or other suitable media, for example, physiological saline, glucose infusion, or buffer solution.

These various preparations may be prepared by conventional methods with commonly used components, for example, excipients, extenders, binders, humidifiers, disintegrants, surface active agents, lubricants, dispersants, buffers, preservatives, dissolution aids, antiseptics, flavoring agents, analgesic agents, stabilizers and the like. Non-toxic additives usable herein include, for example, lactose, fructose, glucose, starch, gelatin, magnesium carbonate, synthetic magnesium silicate, talc, magnesium stearate, methylcellulose, carboxymethylcellulose or a salt thereof, gum arabic, olive oil, propylene glycol, polyethylene glycol, syrup, petrolatum, glycerin, ethanol, citric acid, sodium chloride, sodium sulfite, sodium phosphate, ascorbic acid, and cyclodextrins.

The content of the compound according to the present invention in the medicament may vary according to the dosage form. The content is, however, generally 1.0 to 100% (W/W), preferably 1.0 to 60% (W/W), based on the whole composition.

Regarding the pharmaceuticals according to the present invention, the dose and the number of times of administration are not particularly limited and may be appropriately determined in consideration of various conditions, for example, the purpose of treatment or prevention, the type of diseases, the age, weight, and severity of condition of patients. The dose for the the treatment and prevention of coronary diseases and the like may be appropriately determined in consideration of, for example, the dosage route and the age, sex and severity of condition of patients, and the preparation may be administered usually in an amount of about 0.1 to 4000 mg, preferably about 4 to 1000 mg per day per adult. This dose may be administered at a time daily, divided doses of several times daily, at a time every several days, or persistently.

## EXAMPLES

The present invention is further illustrated by the following Examples that are not intended as a limitation of the invention.

Synthesis Example 1: (2S)-Benzenesulfonylamino-3-[3-fluoro-4-{4-(1,4,5,6-tetrahydropyrimidin-2-ylamino)-piperidin-1-yl}benzoylamino]propionic acid (compound of formula (IV))

The title compound was synthesized as described in Example 52 of WO 99/52872.
Physicochemical properties of the title compound

(1) Color and form: Colorless solid
(2) Molecular formula: $C_{25}H_{31}N_6O_5FS$
(3) Mass spectrum (TSPMS): m/z 547 $(M+H)^+$
(4) Specific rotation: $[\alpha]_D^{26}$ +79° (c 0.56, DMSO)
(5) $^1H$ NMR spectrum (400 MHz, $CD_3OD$) δ (ppm): 1.68 (2H, br q, piperidine), 1.96 (2H, quintet, tetrahydropyrimidine), 2.02 (2H, br d, piperidine), 2.87 (2H, br t, piperidine), 3.37 (4H, t, tetrahydropyrimidine), 3.48 (1H, m, piperidine), 3.52 (2H, br d, piperidine), 3.57 (1H, dd, $CONHC\underline{H_2}CH$), 3.65 (1H, dd, $CONHC\underline{H_2}CH$), 3.73 (1H, dd, $CONHCH_2C\underline{H}$), 7.04 (1H, t, $C_6H_3CO$), 7.53 (5H, m, $C_6H_3CO$ and $C_6H_5$), 7.86 (2H, br d, $C_6H_5$)

Synthesis Example 2: (2S)-Benzenesulfonylamino-3-[4-{4-(1,4,5,6-tetrahydropyrimidin-2-ylamino)piperidin-1-yl}-benzoylamino]propionic acid (compound of formula (V))

The title compound was synthesized as described in Example 3 of WO 99/52872.
Physicochemical properties of the title compound

(1) Color and form: Colorless solid
(2) Molecular formula: $C_{25}H_{32}N_6O_5S$
(3) Mass spectrum (FABMS): m/z 529 $(M+H)^+$
(4) Specific rotation: $[\alpha]_D^{20}$ +69° (c 0.16, MeOH)
(5) $^1H$ NMR spectrum (400 MHz, $CD_3OD$) δ (ppm): 1.49 (2H, br q, piperidine), 1.85 (4H, m, piperidine and tetrahydropyrimidine), 2.84 (2H, br t, piperidine), 3.26 (4H, t, tetrahydropyrimidine), 3.45 (2H, m, $CONHC\underline{H_2}CH$ and piperidine), 3.53 (1H, dd, $CONHC\underline{H_2}CH$), 3.63 (1H, dd, $CONHCH_2C\underline{H}$), 3.73 (2H, br d, piperidine), 6.85 (2H, d, $C_6H_4$), 7.37 (2H, m, $C_6H_5$), 7.44 (1H, m, $C_6H_5$), 7.59 (2H, d, $C_6H_4$), 7.75 (2H, m, $C_6H_5$)

Synthesis Example 3: 4-[2-(3,4,5,6-Tetrahydropyrimidin-2-ylamino)ethyloxy]benzoyl-2(S)phenylsulfonylamino-β-alanine (compound of formula (VI))

The title compound was synthesized as described in WO 95/32710 (compound 19-9).
$^1$H NMR (300 MHz $CD_3OD$ + DCl) δ 7.84 (dm, J = 7 Hz, 2H), 7.74 (d, J = 9 Hz, 2H), 7.50 - 7.40 (m, 3H), 7.02 (d, J = 9 Hz, 2H), 4.22 - 4.17 (m, 3H), 3.72 (dd, J = 14. 5 Hz, 1H), 3.60 (t, J = 5 Hz, 2H), 3.49 (dd, J = 14.9 Hz, 1H), 3.38 (t, J = 5 Hz, 4H), 1.95 (qn, J = 6 Hz, 2H)

Synthesis Example 4: 3-[3-Fluoro-4-{4-(1,4,5,6-tetrahydropyrimidin-2-ylamino)piperidin-1-yl}benzoylamino]-(2S)-{(4-hydroxybenzenesulfonyl)amino}propionic acid (compound of formula (VII))

The title compound was synthesized as described in Example 75 of WO 99/52872.
Physicochemical properties of the title compound

(1) Color and form: Colorless solid
(2) Molecular formula: $C_{25}H_{31}N_6O_6FS$
(3) Mass spectrum (ESIMS): m/z 563 $(M+H)^+$
(4) Specific rotation: $[\alpha]_D^{26}$ +108° (c 0.54, MeOH-conc. $NH_4OH$ (10:1))
(5) $^1$H NMR spectrum (400 MHz, $CD_3OD$) δ (ppm): 1.69 (2H, dq, piperidine), 1.97 (2H, quintet, tetrahydropyrimidine), 2.03 (2H, br d, piperidine), 2.89 (2H, br t, piperidine), 3.37 (4H, t, tetrahydropyrimidine), 3.51 (4H, m, $CONHC\underline{H_2}CH$ and piperidine), 3.68 (1H, dd, $CONHC\underline{H_2}CH$), 3.78 (1H, dd, $CONHCH_2C\underline{H}$), 6.80 (2H, br d, $C_6\underline{H_4}OH$), 7.05 (1H, t, $C_6H_3CO$), 7.51 (1H, dd, $C_6H_3CO$), 7.56 (1H, dd, $C_6H_3CO$), 7.67 (2H, br d, $C_6\underline{H_4}OH$)

Synthesis Example 5: (2S)-Benzenesulfonylamino-3-[3-methoxy-4-{4-(1,4,5,6-tetrahydropyrimidin-2-ylamino)-piperidin-1-yl}benzoylamino]propionic acid (compound of formula (VIII))

The title compound was synthesized as described in Example 93 of WO 99/52872.
Physicochemical properties of the title compound

(1) Color and form: Colorless solid
(2) Molecular formula: $C_{26}H_{34}N_6O_6S$
(3) Mass spectrum (ESIMS): m/z 559 $(M+H)^+$
(4) Specific rotation: $[\alpha]_D^{25}$ +76° (c 0.83, MeOH-conc. $NH_4OH$ (10:1))
(5) $^1$H NMR spectrum (400 MHz, $CD_3OD$) δ (ppm): 1.68 (2H, br dq, piperidine), 1.96 (2H, quintet, tetrahydropyrimidine), 1.99 (2H, m, piperidine), 2.72 (2H, br t, piperidine), 3.37 (4H, t, tetrahydropyrimidine), 3.45 (3H, m, piperidine), 3.56 (1H, dd, $CONHC\underline{H_2}CH$), 3.68 (1H, dd, $CONHC\underline{H_2}CH$), 3.75 (1H, dd, $CONHCH_2C\underline{H}$), 3.91 (3H, s, $C_6H_3O\underline{Me}$), 6.95 (1H, d, $C_6H_3CO$), 7.39 (1H, dd, $C_6H_3CO$), 7.43 (1H, d, $C_6H_3CO$), 7.48 (2H, dt, $C_6\underline{H_5}$), 7.55 (1H, br t, $C_6\underline{H_5}$), 7.87 (2H, m, $C_6\underline{H_5}$)

Synthesis Example 6: (2S)-Benzenesulfonylamino-3-[4-{(3S)-(1,4,5,6-tetrahydropyrimidin-2-ylamino)piperidin-1-yl}benzoylamino]propionic acid (compound of formula (IX))

Intermediate 6-1: (3S)-Aminopiperidin-2-one

Methanol (120 ml) was added to and suspended in L-ornithine hydrochloride (20.0 g, 119 mmol), and the suspension was cooled to -78°C. Thionyl chloride (25.7 ml, 297 mmol) was added dropwise to this suspension at an internal temperature of -78°C to -45°C over a period of 20 min. After 15 min, the temperature of the mixture was raised to room temperature and was further vigorously stirred for 13 hr. The solution thus obtained was concentrated under the reduced pressure, and the residue was further dried by a vacuum pump for 3 hr. The amorphous material thus obtained was subjected to column chromatography using a column packed with an Amberlite IRA-400 $(OH^-)$ anion exchange resin (130 g) to give the title compound as a crude product. A mixed solution composed of 800 ml of chloroform and 80 ml of methanol was added to the crude compound. The insolubles were collected by filtration through Celite. A mixed solvent composed of 400 ml of chloroform and 40 ml of methanol was added again to 9.6 g of the insolubles, and the insolubles were then removed by filtration. The filtrates were combined followed by concentration to give the title compound (13.7 g, 77%).
Physicochemical properties of intermediate 6-1

(1) Color and form: Colorless solid

(2) Molecular formula: $C_5H_{10}N_2O$
(3) Mass spectrum (EIMS): m/z 114 $(M)^+$
(4) Specific rotation: $[\alpha]_D^{24}$ -13° (c 1.5, $CHCl_3$)
(5) $^1H$ NMR spectrum (400 MHz, $D_2O$) δ (ppm): 1.48 (1H, m, piperidine), 1.72 (2H, m, piperidine), 1.99 (1H, dddd, piperidine), 3.16 (2H, dd, piperidine), 3.24 (1H, dd, piperidine)

Intermediate 6-2: (3S)-Aminopiperidine

Tetrahydrofuran (270 ml) was added to and suspended in aluminum lithium hydride (2.52 g, 66.4 mmol) to prepare a suspension which was then ice cooled. Intermediate 6-1 (4.0 g, 26.6 mmol) was gradually added to the cooled suspension at an internal temperature of 5°C to 16°C. After 10 min, the temperature of the mixture was raised to room temperature and was further vigorously stirred for 3 hr. Aluminum lithium hydride (202 mg, 5.32 mmol) was further added thereto, and the mixture was stirred for 50 min. The mixture was ice cooled, and 2.7 ml of water, 2.7 ml of a 5.0 mol/liter aqueous sodium hydroxide solution, and 9.1 ml of water were added in that order to the cooled mixture. The temperature of the mixture was raised to room temperature, and the mixture was vigorously stirred for 1.5 hr. The precipitated inorganic material was collected by filtration through Celite and was then washed with tetrahydrofuran. The filtrate and the washings were combined followed by drying over anhydrous sodium sulfate. A 4.0 mol/liter solution of hydrochloric acid in ethyl acetate (13.3 ml, 53.2 mmol) was added to the filtrate, and the solvent was removed by distillation under the reduced pressure. The residue was subjected to azeotropic distillation with methanol to give a dihydrochloride of the title compound (3.57 g, 78%).
Physicochemical properties of intermediate 6-2

(1) Color and form: Brown solid
(2) Molecular formula: $C_5H_{12}N_2$
(3) Mass spectrum (ELMS): m/z 100 $(M)^+$
(4) $^1H$ NMR spectrum (400 MHz, $CD_3OD$) (as dihydrochloride) δ (ppm): 1.75 (1H, dddd, piperidine), 1.90 (1H, m, piperidine), 2.09 (1H, ddddd, piperidine), 2.23 (1H, br d, piperidine), 3.02 (1H, ddd, piperidine), 3.09 (1H, dd, piperidine), 3.41 (1H, br d, piperidine), 3.62 (2H, m, piperidine)

Intermediate 6-3: 4-{(3S)-Aminopiperidin-1-yl}benzonitrile

Sodium hydrogencarbonate (21.4 g, 255 mmol) was placed in a pressure test tube, and 30 ml of N-methylpyrrolidone was added thereto to prepare a suspension. 4-Fluorobenzonitrile (6.7 g, 55.5 mmol) and intermediate 6-2 (14.3 g, 82.6 mmol) were added in that order to the pressure test tube. The mixture was stirred at room temperature for 5 min in such a state that the system was opened. Thereafter, the system was hermetically sealed, and stirring was carried out at 120°C for 22 hr. The temperature of the reaction solution was returned to room temperature, and the reaction system was then purified by chromatography on silica gel (development system: methylene chloride : methanol : aqueous ammonia = 100 : 10 : 1) to give the title compound (9.89 g, 89%).
Physicochemical properties of intermediate 6-3

(1) Color and form: Light yellow syrup
(2) Molecular formula: $C_{12}H_{15}N_3$
(3) Mass spectrum (TSPMS): m/z 202 $(M+H)^+$
(4) $^1H$ NMR spectrum (400 MHz, $CDCl_3$) δ (ppm): 1.33 (1H, dddd, piperidine), 1.63 (1H, ddddd, piperidine), 1.79 - 1.87 (1H, m, piperidine), 1.95 - 2.04 (1H, m, piperidine), 2.74 (1H, dd, piperidine), 2.90 - 2.99 (2H, m, piperidine), 3.61 (1H, ddd, piperidine), 3.72 (1H, dddd, piperidine), 6.86 (2H, d, $C_6H_4$), 7.47 (2H, d, $C_6H_4$)

Intermediate 6-4: 4-{(3S)-(Pyrimidin-2-ylamino)-piperidin-1-yl}benzonitrile

Dimethyl sulfoxide (250 ml) was added to 2-bromopyrimidine (8.72 g, 54.9 mmol) and intermediate 6-3 (9.89 g, 49.2 mmol) to prepare a solution. Diisopropylethylamine (50 ml, 287 mmol) was added to the solution, and the mixture was vigorously stirred at 120°C for 12 hr. The temperature of the reaction solution was returned to room temperature. Water (500 ml) was then added to the reaction solution, and the mixture was extracted three times with 500 ml of ethyl acetate. The organic layers were combined, and the combined organic layers were washed three times with 500 ml of water and once with 500 ml of saturated brine, were dried over anhydrous magnesium sulfate, and were then concentrated under the reduced pressure. The residue was purified by chromatography on silica gel (development system: hexane : ethyl acetate = 1 : 1 → only ethyl acetate) to give the title compound (10.5 g, 77%).
Physicochemical properties of intermediate 6-4

(1) Color and form: Light yellow solid
(2) Molecular formula: $C_{16}H_{17}N_5$
(3) Mass spectrum (EIMS): m/z 279 $(M)^+$
(4) Specific rotation: $[\alpha]_D^{26}$ +46° (c 0.50, $CHCl_3$)
(5) $^1H$ NMR spectrum (400 MHz, $CDCl_3$) δ (ppm): 1.60 - 1.72 (1H, m, piperidine), 1.75 (1H, dddd, piperidine), 1.83 - 1.92 (1H, m, piperidine), 2.01 - 2.10 (1H, m, piperidine), 3.03 (1H, dd, piperidine), 3.17 (1H, ddd, piperidine), 3.56 (1H, ddd, piperidine), 3.92 (1H, dd, piperidine), 4.09 (1H, ddddd, piperidine), 6.58 (1H, t, pyrimidine), 6.92 (2H, d, $C_6H_4$), 7.47 (2H, d, $C_6H_4$), 8.30 (2H, d, pyrimidine)

Intermediate 6-5: 4-{(35)-(Pyrimidin-2-ylamino)-piperidin-1-yl}benzoic acid

Intermediate 6-4 (7.75 g, 27.7 mmol) was dissolved in 28 ml of 50% sulfuric acid to prepare a solution which was then heated under reflux for 2 hr. The temperature of the reaction solution was returned to room temperature, and the reaction solution was then slowly poured into 800 ml of a saturated aqueous sodium hydrogencarbonate solution under ice cooling. The mixture was adjusted to pH 4 by the addition of 1.0 mol/liter hydrochloric acid and a saturated aqueous sodium hydrogencarbonate solution. The precipitate was collected by filtration through a glass filter, was washed with water, and was then dried to give the title compound (8.11 g, 98%).
Physicochemical properties of intermediate 6-5

(1) Color and form: Colorless solid
(2) Molecular formula: $C_{16}H_{18}N_4O_2$
(3) Mass spectrum (EIMS): m/z 298 $(M)^+$
(4) $^1H$ NMR spectrum (400 MHz, DMSO-$d_6$) δ (ppm): 1.52 - 1.61 (2H, m, piperidine), 1.73 - 1.81 (1H, m, piperidine), 1.93 - 2.01 (1H, m, piperidine), 2.76 (1H, dd, piperidine), 2.87 (1H, ddd, piperidine), 3.79 - 3.91 (2H, m, piperidine), 3.98 (1H, br d, piperidine), 6.58 (1H, t, pyrimidine), 6.96 (2H, d, $C_6H_4$), 7.74 (2H, d, $C_6H_4$), 8.29 (2H, d, pyrimidine)

Intermediate 6-6: t-Butyl (2S)-benzenesulfonylamino-3-[4-{(3S)-(pyrimidin-2-ylamino)piperidin-1-yl}benzoylamino] propionate

Dimethylformamide (3.4 ml) was added to intermediate 6-5 (50 mg, 0.17 mmol) to prepare a solution. N,N-Diisopropylethylamine (0.087 ml, 0.50 mmol) and BOP reagent (90 mg, 0.20 mmol) were added to the solution, and the mixture was stirred at room temperature for 10 min. t-Butyl (2S)-N-benzenesulfonyl-2,3-diaminopropionate (60 mg, 0.20 mmol) was then added thereto, and the mixture was stirred at room temperature for 14 hr. Water was added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: chloroform : methanol = 9 : 1) to give the title compound (93 mg, 95%).
Physicochemical properties of intermediate 6-6

(1) Color and form: Colorless solid
(2) Molecular formula: $C_{29}H_{36}N_6O_5S$
(3) Mass spectrum (TSPMS): m/z 581 $(M+H)^+$
(4) Specific rotation: $[\alpha]_D^{25}$ +75° (c 0.26, $CHCl_3$)
(5) $^1H$ NMR spectrum (400 MHz, $CDCl_3$) δ (ppm): 1.28 (9H, s, t-Bu), 1.66 (1H, m, piperidine), 1.75 (1H, m, piperidine), 1.86 (1H, m, piperidine), 1.99 (1H, m, piperidine), 3.02 (1H, dd, piperidine), 3.16 (1H, ddd, piperidine), 3.48 (1H, m, piperidine), 3.58 (1H, ddd, CONH$CH_2$CH), 3.81 (1H, dd, piperidine), 3.89 (2H, m, CONH$CH_2CH$), 4.15 (1H, m, piperidine), 5.30 (1H, d, NH), 5.78 (1H, d, NH), 6.52 (1H, m, NH), 6.55 (1H, t, pyrimidine), 6.93 (2H, d, $C_6H_4$), 7.47 (2H, m, $C_6H_5$), 7.55 (1H, m, $C_6H_5$), 7.66 (2H, d, $C_6H_4$), 7.84 (2H, m, $C_6H_5$), 8.29 (2H, d, pyrimidine)

Intermediate 6-7: (2S)-Benzenesulfonylamino-3-[4-{(3S)-(pyrimidin-2-ylamino)piperidin-1-yl}benzoylamino]-propionic acid

Methylene chloride (1.3 ml) was added to intermediate 6-6 (72 mg, 0.13 mmol) to prepare a solution. Trifluoroacetic acid (1.3 ml) was added to the solution, and the mixture was stirred at room temperature for 5 hr. The reaction solution was concentrated under the reduced pressure to give a trifluoroacetate of the title compound.
Physicochemical properties of intermediate 6-7 (as trifluoroacetate)

(1) Color and form: Colorless solid

(2) Molecular formula: $C_{25}H_{28}N_6O_5S$

Title compound (compound of formula (IX)): (2S)-Benzenesulfonylamino-3-[4-{(3S)-(1,4,5,6-tetrahydropyrimidin-2-ylamino)piperidin-1-yl}benzoylamino]-propionic acid

Dioxane (2.0 ml) and 0.2 ml of water were added to the trifluoroacetate of intermediate 6-7 (0.072 mmol) as the crude compound to prepare a solution. To the solution was added 13 mg of 10% palladium-carbon, and the mixture was vigorously stirred under a hydrogen pressure of 1 atm at room temperature for 3 hr. The reaction solution was filtered through Celite, followed by washing with dioxane and water. The filtrate and the washings were combined, and the combined solution was concentrated under the reduced pressure. The residue was purified by preparative thin-layer chromatography on silica gel (development system: methylene chloride : ethanol : water : concentrated aqueous ammonia = 8 : 8 : 1 : 1) and was then purified by Sephadex LH-20 (development system: methanol) to give the title compound (26 mg, 68%).
Physicochemical properties of title compound (Synthesis Example 6)

(1) Color and form: Colorless solid
(2) Molecular formula: $C_{25}H_{32}N_6O_5S$
(3) Mass spectrum (TSPMS): m/z 529 $(M+H)^+$
(4) Specific rotation: $[\alpha]_D^{28}$ +54° (c 0.23, MeOH)
(5) $^1H$ NMR spectrum (400 MHz, $CD_3OD$) δ (ppm): 1.62 (1H, m, piperidine), 1.75 (1H, m, piperidine), 1.87 (1H, m, piperidine), 1.98 (3H, m, piperidine and tetrahydropyrimidine), 3.12 (1H, dd, piperidine), 3.18 (1H, m, piperidine), 3.33 (1H, m, piperidine), 3.36 (4H, t, tetrahydropyrimidine), 3.54 (2H, m, CONH$\underline{CH_2}$CH and piperidine), 3.66 (2H, m, CONHC$\underline{H_2}$CH and piperidine), 3.87 (1H, dd, $CONHCH_2\underline{CH}$), 6.97 (2H, d, $C_6H_4$), 7.46 (2H, m, $C_6H_5$), 7.53 (1H, m, $C_6H_5$), 7.69 (2H, d, $C_6H_4$), 7.84 (2H, m, $C_6H_5$)

Synthesis Example 7: (2S)-Benzenesulfonylamino-3-[2-fluoro-4-{(3S)-(1,4,5,6-tetrahydropyrimidin-2-ylamino)-piperidin-1-yl}benzoylamino]propionic acid (compound of formula (X))

Intermediate 7-1: Ethyl 4-{(3S)-aminopiperidin-1-yl}-2-fluorobenzoate

Intermediate 6-2 (630 mg, 3.7 mmol) and sodium hydrogencarbonate (1.01 g, 12 mmol) were added to ethyl 2,4-difluorobenzoate (230 mg, 1.2 mmol), and the mixture was stirred in a sealed tube at 110°C for 19 hr. Water (50 ml) was added to the reaction solution, and the mixture was extracted three times with ethyl acetate. The organic layer was extracted three times with 0.5 mol/liter hydrochloric acid, and the aqueous layer was adjusted to pH 8 by the addition of a 1.0 mol/liter aqueous sodium hydroxide solution and was then extracted three times with ethyl acetate. The organic layer was dried over anhydrous magnesium sulfate and was then concentrated under the reduced pressure. The residue was purified by column chromatography on silica gel (development system: chloroform : methanol = 9 : 1) to give the title compound (84 mg, 26%).
Physicochemical properties of intermediate 7-1

(1) Color and form: Brown liquid
(2) Molecular formula: $C_{14}H_{19}N_2O_2F$
(3) Mass spectrum (TSPMS): m/z 267 $(M+H)^+$
(4) Specific rotation: $[\alpha]_D^{26}$ +45° (c 0.28, $CHCl_3$)
(5) $^1H$ NMR spectrum (400 MHz, $CDCl_3$) δ (ppm): 1.30 (1H, m, piperidine), 1.36 (3H, t, $CH_2\underline{CH_3}$), 1.61 (1H, m, piperidine), 1.81 (1H, m, piperidine), 1.93 (1H, m, piperidine), 2.71 (1H, dd, piperidine), 2.92 (2H, m, piperidine), 3.60 (1H, dt, piperidine), 3.71 (1H, m, piperidine), 4.32 (2H, q, $\underline{CH_2}CH_3$), 6.49 (1H, dd, $C_6H_3$), 6.61 (1H, dd, $C_6H_3$), 7.78 (1H, dd, $C_6H_3$)

Intermediate 7-2: Ethyl 2-fluoro-4-{(3S)-(pyrimidin-2-ylamino)piperidin-1-yl}benzoate

N-Methylpyrrolidone (1.0 ml) was added to intermediate 7-1 (144 mg, 0.54 mmol) to prepare a solution. N,N-Diisopropylethylamine (0.47 ml, 2.7 mmol) and 2-bromopyrimidine (100 mg, 0.65 mmol) were added to the solution, and the mixture was stirred at 110°C for 20 hr. Water (50 ml) was then added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 1 : 1) to give the title compound (60 mg, 33%).

Physicochemical properties of intermediate 7-2

(1) Color and form: Colorless solid
(2) Molecular formula: $C_{18}H_{21}N_4O_2F$
(3) Mass spectrum (EIMS): m/z 344 ($M^+$)
(4) Specific rotation: $[\alpha]_D^{26}$ +35° (c 1.05, $CHCl_3$)
(5) $^1$H NMR spectrum (400 MHz, $CDCl_3$) δ (ppm): 1.36 (3H, t, $CH_2\underline{CH_3}$), 1.63 (1H, m, piperidine), 1.75 (1H, m, piperidine), 1.84 (1H, m, piperidine), 2.04 (1H, m, piperidine), 3.00 (1H, dd, piperidine), 3.15 (1H, ddd, piperidine), 3.57 (1H, dt, piperidine), 3.92 (1H, dd, piperidine), 4.08 (1H, m, piperidine), 4.32 (2H, q, $\underline{CH_2}CH_3$), 5.23 (1H, d, NH), 6.57 (1H, t, pyrimidine), 6.61 (1H, dd, $C_6H_3$), 6.67 (1H, dd, $C_6H_3$), 7.79 (1H, dd, $C_6H_3$), 8.29 (2H, d, pyrimidine)

Intermediate 7-3: 2-Fluoro-4-{(3S)-(pyrimidin-2-ylamino)piperidin-1-yl}benzoic acid

Tetrahydrofuran (2.7 ml) and 0.9 ml of methanol were added to intermediate 7-2 (60 mg, 0.17 mmol) to prepare a solution, and 0.9 ml of a 1 mol/liter aqueous sodium hydroxide solution was added to the solution. The mixture was stirred at 40°C for 16 hr and was then concentrated under the reduced pressure. Water (20 ml) was added to the residue to prepare a solution which was then washed twice with ethyl acetate. The aqueous layer was adjusted to pH 4 by the addition of 1 mol/liter hydrochloric acid. The precipitated solid was separated by centrifugation and was then dried to give the title compound (45 mg, 84%).
Physicochemical properties of intermediate 7-3

(1) Color and form: Colorless solid
(2) Molecular formula: $C_{16}H_{17}N_4O_2F$
(3) Mass spectrum (EIMS): m/z 316 ($M^+$)
(4) Specific rotation: $[\alpha]_D^{26}$+17° (c 0.26, MeOH)
(5) $^1$H NMR spectrum (400 MHz, $CDCl_3$) δ (ppm): 1.77 (2H, m, piperidine), 1.92 (1H, m, piperidine), 2.09 (1H, m, piperidine), 3.16 (2H, m, piperidine), 3.69 (1H, m, piperidine), 3.92 (1H, m, piperidine), 4.17 (1H, m, piperidine), 6.71 (1H, dd, $C_6H_3$), 6.79 (1H, dd, $C_6H_3$), 6.99 (1H, t, pyrimidine), 7.78 (1H, dd, $C_6H_3$), 8.58 (2H, m, pyrimidine)

Intermediate 7-4: t-Butyl (2S)-benzenesulfonylamino-3-[2-fluoro-4-{(3S)-(pyrimidin-2-ylamino)piperidin-1-yl}-benzoylamino]propionate

Dimethylformamide (5.0 ml) was added to intermediate 7-3 (45 mg, 0.14 mmol) to prepare a solution. N,N-Diisopropylethylamine (0.073 ml, 0.42 mmol) and BOP reagent (76 mg, 0.17 mmol) were added to the solution, and the mixture was stirred at room temperature for 10 min. t-Butyl (2S)-N-benzenesulfonyl-2,3-diaminopropionate (50 mg, 0.17 mmol) was then added thereto, and the mixture was stirred at room temperature for 14 hr. Water was added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: chloroform : methanol = 8 : 2) to give the title compound (43 mg, 51%).
Physicochemical properties of intermediate 7-4

(1) Color and form: Colorless solid
(2) Molecular formula: $C_{29}H_{35}N_6O_5FS$
(3) Mass spectrum (TSPMS): m/z 599 $(M+H)^+$
(4) Specific rotation: $[\alpha]_D^{25}$ +40° (c 0.26, $CHCl_3$)
(5) $^1$H NMR spectrum (400 MHz, $CDCl_3$) δ (ppm): 1.29 (9H, s, t-Bu), 1.65 (1H, m, piperidine), 1.73 (1H, m, piperidine), 1.84 (1H, m, piperidine), 2.05 (1H, m, piperidine), 2.98 (1H, dd, piperidine), 3.14 (1H, ddd, piperidine), 3.56 (1H, m, piperidine), 3.74 (2H, ddd, $CONH\underline{CH_2}CH$), 3.90 (1H, dd, piperidine), 4.00 (1H, m, $CONHCH_2\underline{CH}$), 4.07 (1H, m, piperidine), 5.20 (1H, d, NH), 5.82 (1H, d, NH), 6.57 (1H, t, pyrimidine), 6.58 (1H, d, $C_6H_3$), 6.73 (1H, dd, $C_6H_3$), 6.87 (1H, m, NH), 7.44 (2H, t, $C_6H_5$), 7.51 (1H, d, $C_6H_5$), 7.83 (2H, d, $C_6H_5$), 7.87 (1H, t, $C_6H_3$), 8.30 (2H, d, pyrimidine)

Intermediate 7-5: (2S)-Benzenesulfonylamino-3-[2-fluoro-4-{(3S)-(pyrimidin-2-ylainino)piperidin-1-yl}benzoylamino] propionic acid

Methylene chloride (1.3 ml) was added to intermediate 7-4 (72 mg, 0.13 mmol) to prepare a solution. Trifluoroacetic acid (1.3 ml) was added to the solution, and the mixture was stirred at room temperature for 5 hr. The reaction

solution was concentrated under the reduced pressure to give a trifluoroacetate of the title compound.
Physicochemical properties of intermediate 7-5 (as trifluoroacetate)

(1) Color and form: Colorless solid
(2) Molecular formula: $C_{25}H_{27}N_6O_5FS$
(3) Mass spectrum (TSPMS): m/z 543 $(M+H)^+$
(4) $^1H$ NMR spectrum (400 MHz, $CD_3OD$) δ (ppm): 1.77 (2H, m, piperidine), 1.92 (1H, m, piperidine), 2.10 (1H, m, piperidine), 3.15 (2H, m, piperidine), 3.44 (1H, dd, piperidine), 3.69 (1H, m, piperidine), 3.77 (1H, dd, CONH$\underline{CH_2}$CH), 3.92 (1H, m, piperidine), 4.16 (2H, m, CONH$\underline{CH_2CH}$), 6.71 (1H, dd, $C_6H_3$), 6.83 (1H, dd, $C_6H_3$), 6.93 (1H, t, pyrimidine), 7.41 (2H, d, $C_6H_5$), 7.45 (1H, m, $C_6H_5$), 7.66 (1H, dd, $C_6H_3$), 7.80 (2H, m, $C_6H_5$), 8.55 (2H, br s, pyrimidine)

Title compound (compound of formula (X)): (2S)-Benzenesulfonylamino-3-[2-fluoro-4-{(3S)-(1,4,5,6-tetrahydropyrimidin-2-ylamino)piperidin-1-yl}benzoylamino]propionic acid

Dioxane (2.0 ml) and 0.2 ml of water were added to the trifluoroacetate of intermediate 7-5 (0.056 mmol) as the crude compound to prepare a solution. To the solution was added 20 mg of 10% palladium-carbon. The mixture was vigorously stirred under a hydrogen pressure of 1 atm at room temperature for 16 hr. The reaction solution was filtered through Celite, followed by washing with dioxane and water. The filtrate and the washings were combined, and the combined solution was concentrated under the reduced pressure. The residue was purified by preparative thin-layer chromatography on silica gel (development system: methylene chloride : ethanol : water : concentrated aqueous ammonia = 8 : 8 : 1 : 1) and was then purified by Sephadex LH-20 (development system: methanol) to give the title compound (6.0 mg, 20%).
Physicochemical properties of title compound (Synthesis Example 7)

(1) Color and form: Colorless solid
(2) Molecular formula: $C_{25}H_{31}N_6O_5FS$
(3) Mass spectrum (TSPMS): m/z 547 $(M+H)^+$
(4) Specific rotation: $[\alpha]_D^{28}$ +35° (c 0.17, MeOH)
(5) $^1H$ NMR spectrum (400 MHz, $CD_3OD$) δ (ppm): 1.63 (1H, m, piperidine), 1.70 (1H, m, piperidine), 1.86 (1H, m, piperidine), 1.97 (3H, m, piperidine and tetrahydropyrimidine), 3.12 (1H, m, piperidine), 3.20 (1H, m, piperidine), 3.36 (4H, t, tetrahydropyrimidine), 3.42 (1H, m, piperidine), 3.65 (4H, m, CONH$\underline{CH_2}$CH and piperidine), 3.85 (1H, m, CONHCH$_2\underline{CH}$), 6.67 (1H, d, $C_6H_3$), 6.80 (1H, d, $C_6H_3$), 7.46 (2H, m, $C_6H_5$), 7.50 (1H, m, $C_6H_5$), 7.72 (1H, dd, $C_6H_3$), 7.83 (2H, m, $C_6H_5$)

Synthesis Example 8: 3-[4-{(3S)-(1,4,5,6-Tetrahydropyrimidin-2-ylamino)piperidin-1-yl}benzoylamino]-(2S)-{(thiophene-2-sulfonyl)amino}propionic acid (compound of formula (XI))

Intermediate 8-A: Ethyl 4-{(3S)-aminopiperidin-1-yl}benzoate

N-Methylpyrrolidone (2.0 ml) was added to intermediate 6-2 (200 mg, 1.16 mmol) to prepare a solution. Sodium hydrogencarbonate (487 mg, 5.80 mmol) and ethyl 4-fluorobenzoate (85 μl, 0.580 mmol) were added to the solution. The mixture was stirred at 120°C for 23 hr. Water (50 ml) and 50 ml of saturated brine were then added to the reaction solution, and the mixture was extracted three times with ethyl acetate. The organic layers were combined, and the combined organic layers were washed with a mixed solution composed of 50 ml of saturated brine and 50 ml of water, were dried over anhydrous sodium sulfate, and were then concentrated under the reduced pressure. The residue was purified by column chromatography on silica gel (development system: methylene chloride : methanol = 7 : 1) to give the title compound (61.2 mg, 42%).
Physicochemical properties of intermediate 8-A

(1) Color and form: Colorless solid
(2) Molecular formula: $C_{14}H_{20}N_2O_2$
(3) Mass spectrum (TSPMS): m/z 249 $(M+H)^+$
(4) Specific rotation: $[\alpha]_D^{22}$ +49° (c 0.71, MeOH)
(5) $^1H$ NMR spectrum (400 MHz, $CDCl_3$) δ (ppm): 1.33 (1H, m, piperidine), 1.36 (3H, t, CH$_2\underline{CH_3}$), 1.63 (1H, m, piperidine), 1.82 (1H, m, piperidine), 1.98 (1H, m, piperidine), 2.74 (1H, dd, piperidine), 2.95 (2H, m, piperidine), 3.58 (1H, dt, piperidine), 3.71 (1H, m, piperidine), 4.32 (2H, q, $\underline{CH_2}$CH$_3$), 6.86 (2H, d, $C_6H_4$), 7.89 (2H, d, $C_6H_4$)

Intermediate 8-B: Ethyl 4-{(3S)-(Pyrimidin-2-ylamino)piperidin-1-yl}benzoate

N-Methylpyrrolidone (0.40 ml) was added to intermediate 8-A (50 mg, 0.20 mmol) to prepare a solution. N,N-Diisopropylethylamine (0.18 ml, 1.0 mmol) and 2-bromopyrimidine (35 mg, 0.24 mmol) were added to the solution, and the mixture was stirred at 110°C for 20 hr. Water (50 ml) was then added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 7 : 3) to give the title compound (38 mg, 58%).
Physicochemical properties of intermediate 8-B

(1) Color and form: Colorless solid
(2) Molecular formula: $C_{18}H_{22}N_4O_2$
(3) Mass spectrum (TSPMS): m/z 327 $(M+H)^+$
(4) $^1H$ NMR spectrum (400 MHz, $CDCl_3$) δ (ppm): 1.36 (3H, t, $CH_2\underline{CH_3}$), 1.69 (1H, m, piperidine), 1.74 (1H, m, piperidine), 1.85 (1H, m, piperidine), 2.02 (1H, m, piperidine), 3.06 (1H, dd, piperidine), 3.40 (1H, ddd, piperidine), 3.50 (1H, m, piperidine), 3.84 (1H, dd, piperidine), 4.14 (1H, m, piperidine), 4.32 (2H, q, $\underline{CH_2}CH_3$), 5.24 (1H, d, NH), 6.55 (1H, t, pyrimidine), 6.91 (2H, d, $C_6H_4$), 7.89 (2H, d, $C_6H_4$), 8.28 (2H, d, pyrimidine)

Intermediate 8-1: t-Butyl (2S)-(benzyloxycarbonyl)amino-3-[4-{(3S)-(pyrimidin-2-ylamino)piperidin-1-yl}benzoylamino] propionate

Dimethylformamide (5.0 ml) was added to intermediate 8-B (60 mg, 0.20 mmol) to prepare a solution. N,N-Diisopropylethylamine (0.10 ml, 0.60 mmol) and BOP reagent (100 mg, 0.24 mmol) were added to the solution, and the mixture was stirred at room temperature for 30 min. t-Butyl (2S)-N-benzyloxycarbonyl-2,3-diaminopropionate hydrochloride (70 mg, 0.24 mmol) was then added thereto, and the mixture was stirred at room temperature for 4 hr. Water was added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 1 : 3) to give the title compound (140 mg, 100%).
Physicochemical properties of intermediate 8-1

(1) Color and form: Colorless solid
(2) Molecular formula: $C_{31}H_{38}N_6O_5$
(3) Mass spectrum (TSPMS): m/z 575 $(M+H)^+$
(4) Specific rotation: $[\alpha]_D^{25}$ +10° (c 0.17, $CHCl_3$)
(5) $^1H$ NMR spectrum (400 MHz, $CDCl_3$) δ (ppm): 1.46 (9H, s, t-Bu), 1.66 (1H, m, piperidine), 1.75 (1H, m, piperidine), 1.86 (1H, m, piperidine), 2.00 (1H, m, piperidine), 3.03 (1H, dd, piperidine), 3.16 (1H, ddd, piperidine), 3.45 (1H, m, piperidine), 3.78 (3H, m, piperidine and $CONH\underline{CH_2}CH$), 4.15 (1H, m, piperidine), 4.43 (1H, m, $CONHCH_2\underline{CH}$), 5.10 (2H, s, $\underline{CH_2}C_6H_5$), 5.27 (1H, d, NH), 5.87 (1H, brs, NH), 6.54 (1H, t, pyrimidine), 6.66 (1H, brs, NH), 6.91 (2H, d, $C_6H_4$), 7.32 (5H, m, $C_6H_5$), 7.63 (2H, d, $C_6H_4$), 8.287 (2H, d, pyrimidine)

Intermediate 8-2: t-Butyl (2S)-amino-3-[4-{(3S)-(pyrimidin-2-ylamino)piperidin-1-yl}benzoylamino]-propionate

Tetrahydrofuran was added to intermediate 8-1 (85 mg, 0.15 mmol) to prepare a solution. To the solution was added 34 mg of 5% palladium-carbon. The mixture was vigorously stirred under a hydrogen pressure of 1 atm at room temperature for 12 hr. The reaction solution was filtered through Celite and was then washed with tetrahydrofuran and ethanol. The filtrate and the washings were combined, and the combined solution was concentrated under the reduced pressure. The residue was purified by column chromatography on silica gel (development system: chloroform : methanol = 8 : 2) to give the title compound (48 mg, 74%).
Physicochemical properties of intermediate 8-2

(1) Color and form: Colorless solid
(2) Molecular formula: $C_{23}H_{32}N_6O_3$
(3) Mass spectrum (TSPMS): m/z 441 $(M+H)^+$
(4) Specific rotation: $[\alpha]_D^{25}$ +30° (c 0.48, $CHCl_3$)
(5) $^1H$ NMR spectrum (400 MHz, $CDCl_3$) δ (ppm): 1.26 (9H, s, t-Bu), 1.76 (2H, m, piperidine), 1.86 (1H, m, piperidine), 2.00 (1H, m, piperidine), 3.03 (1H, dd, piperidine), 3.17 (1H, ddd, piperidine), 3.45 (2H, m, piperidine), 3.60

(1H, m, CONHCH2CH), 3.80 (2H, m, CONHCH2CH), 4.15 (1H, m, piperidine), 5.29 (1H, d, NH), 6.54 (1H, t, pyrimidine), 6.67 (1H, brs, NH), 6.92 (2H, d, $C_6H_4$), 7.66 (2H, d, $C_6H_4$), 8.28 (2H, d, pyrimidine)

Intermediate 8-3: t-Butyl 3-[4-{(3S)-(pyrimidin-2-ylamino)piperidin-1-yl}benzoylamino]-(2S)-{(thiophene-2-sulfonyl)amino}propionate

Dimethylformamide (3.0 ml) was added to intermediate 8-2 (40.0 mg, 0.0900 mmol) to prepare a solution. N,N-Diisopropylethylamine (0.032 ml, 0.180 mmol) and 2-thiophenesulfonyl chloride (16.0 mg, 0.0900 mmol) were added to the solution, and the mixture was stirred at room temperature for 16 hr. Piperidine was added to the reaction solution, water and a saturated aqueous sodium hydrogencarbonate solution were then added thereto, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: chloroform : methanol = 9 : 1) to give the title compound (27.0 mg, 51%).
Physicochemical properties of intermediate 8-3

(1) Color and form: Colorless solid
(2) Molecular formula: $C_{27}H_{34}N_6O_5S_2$
(3) Mass spectrum (TSPMS): m/z 587 $(M+H)^+$
(4) $^1H$ NMR spectrum (400 MHz, $CDCl_3$) δ (ppm): 1.35 (9H, s, t-Bu), 1.65 (1H, m, piperidine), 1.75 (1H, m, piperidine), 1.86 (1H, m, piperidine), 2.00 (1H, m, piperidine), 3.00 (1H, dd, piperidine), 3.14 (1H, ddd, piperidine), 3.46 (1H, m, piperidine), 3.67 (1H, ddd, CONHCH2CH), 3.80 (1H, m, piperidine), 3.86 (1H, m, CONHCH2CH), 4.04 (1H, m, CONHCH2CH), 4.13 (1H, m, piperidine), 5.35 (1H, bd, NH), 5.98 (1H, bd, NH), 6.49 (1H, m, NH), 6.55 (1H, t, pyrimidine), 6.92 (2H, d, $C_6H_4$), 7.05 (1H, dd, thiophene), 7.57 (1H, dd, thiophene), 7.60 (1H, dd, thiophene), 7.65 (2H, d, $C_6H_4$), 8.29 (2H, d, pyrimidine)

Intermediate 8-4: 3-[4-{(3S)-(Pyrimidin-2-ylamino)-piperidin-1-yl}benzoylamino]-(2S)-{(thiophene-2-sulfonyl)amino}propionic acid

Methylene chloride (5.0 ml) was added to intermediate 8-3 (100 mg, 0.170 mmol) to prepare a solution. Trifluoroacetic acid (5.0 ml) was added to the solution, and the mixture was stirred at room temperature for 4 hr. The reaction solution was concentrated under the reduced pressure to give the title compound.
Physicochemical properties of intermediate 8-4

(1) Color and form: Colorless solid
(2) Molecular formula: $C_{23}H_{26}N_6O_5S_2$
(3) Mass spectrum (FABMS): m/z 531 $(M+H)^+$
(4) $^1H$ NMR spectrum (400 MHz, $CD_3OD$) δ (ppm): 1.77 (2H, m, piperidine), 1.93 (1H, m, piperidine), 2.09 (1H, m, piperidine), 3.14 (2H, m, piperidine), 3.49 (1H, dd, CONHCH2CH), 3.62 (1H, m, piperidine), 3.74 (1H, dd, CONHCH2CH), 3.85 (1H, m, piperidine), 4.23 (2H, m, piperidine and CONHCH2CH), 6.95 (1H, t, pyrimidine), 7.01 (3H, m, $C_6H_4$ and thiophene), 7.56 (1H, dd, thiophene), 7.64 (1H, dd, thiophene), 7.68 (2H, d, $C_6H_4$), 8.57 (2H, brs, pyrimidine)

Ttile compound: 3-[4-{(3S)-(1,4,5,6-Tetrahydropyrimidin-2-ylamino)piperidin-1-yl}benzoylamino]-(2S)-{(thiophene-2-sulfonyl)amino}propionic acid

Tetrahydrofuran (5.0 ml) and 0.5 ml of water were added to intermediate 8-4 to prepare a solution. To the solution was added 100 mg of 10% palladium-carbon. The mixture was vigorously stirred under a hydrogen pressure of 1 atm at room temperature for 15 hr. The reaction solution was filtered through Celite and was then washed with tetrahydrofuran and water. The filtrate and the washings were combined, and the combined solution was concentrated under the reduced pressure. The residue was purified by preparative thin-layer chromatography on silica gel (development system: chloroform : methanol : concentrated aqueous ammonia = 8 : 2 : 0.2) and was then purified by Sephadex LH-20 (development system: methanol) to give the title compound.
Physicochemical properties of the title compound

(1) Color and form: Colorless solid
(2) Molecular formula: $C_{23}H_{30}N_6O_5S_2$
(3) Mass spectrum (TSPMS): m/z 535 $(M+H)^+$

(4) $^1H$ NMR spectrum (400 MHz, $CDCl_3$) δ (ppm): 1.58 (1H, m, piperidine), 1.70 (1H, m, piperidine), 1.86 (1H, m, piperidine), 1.94 (3H, m, tetrahydropyrimidine and piperidine), 3.02 (1H, dd, piperidine), 3.08 (1H, m, piperidine), 3.34 (4H, t, tetrahydropyrimidine), 3.40 (1H, m, piperidine), 3.54 (2H, m, CONH$\underline{CH_2}$CH and piperidine), 3.63 (1H, m, piperidine), 3.67 (1H, dd, CONH$\underline{CH_2}$CH), 3.97 (1H, dd, CONH$CH_2\underline{CH}$), 6.94 (2H, d, $C_6H_4$), 7.02 (1H, dd, thiophene), 7.57 (1H, dd, thiophene), 7.66 (3H, m, thiophene and $C_6H_4$)

Synthesis Example 9: (2S)-Benzenesulfonylamino-3-[4-{(4S)-methoxy-(2R)-methyl-(3R)-(1,4,5,6-tetrahydropyrimidin-2-ylamino)piperidin-1-yl}benzoylamino]-propionic acid

Intermediate 9-1: Ethyl 4-{(4R)-azido-(5R)-hydroxy-(3S)-methoxyhexylamino}benzoate

Acetonitrile (200 ml) and 50 ml of ethanol were added to oleandomycin phosphate (25.4 g, 32.4 mmol) to prepare a solution. p-Toluenesulfonic acid (12.3 g, 64.9 mmol) was added to the solution, and the mixture was stirred at room temperature for 3 hr. A saturated aqueous sodium carbonate solution was added to the reaction solution, and the mixture was extracted three times with chloroform. The extract was dried over anhydrous sodium sulfate and was then concentrated under the reduced pressure. The residue was purified by column chromatography on silica gel (development system: ethyl acetate) to give ethyloleandroside (3.75 g, 61%).

Methylene chloride (180 ml) was added to ethyloleandroside (3.75 g, 18.4 mmol) to prepare a solution which was then ice cooled. Triethylamine (4.00 ml, 28.9 mmol) and methanesulfonyl chloride (1.85 ml, 23.9 mmol) were added to the solution, and the mixture was stirred at 0°C for 2 hr. Ice was added to the reaction solution, and the mixture was extracted once with chloroform. The organic layer was washed with water, was dried over anhydrous magnesium sulfate, and was then concentrated under the reduced pressure to give a methanesulfonyl compound (5.91 g, 100%).

Dimethylformamide (100 ml) was added to the crude methanesulfonyl compound (18.4 mmol) to prepare a solution. Sodium azide (1.41 g, 21.7 mmol) was added to the solution, and the mixture was stirred at 80°C for 18 hr. Water was added to the reaction solution, and the mixture was extracted once with ethyl acetate. The organic layer was dried over anhydrous magnesium sulfate and was then concentrated under the reduced pressure. The residue was purified by column chromatography on silica gel (development system: hexane : ethyl acetate = 2 : 1) to give an azide compound (5.22 g, 99%).

Dioxane (50 ml) was added to the azide compound (2.07 g, 9.62 mmol) to prepare a solution, and 50 ml of 1.0 mol/liter hydrochloric acid was added to the solution. The mixture was stirred at 60°C for 3 hr and was then ice cooled. The mixture was adjusted to pH 8 by the addition of a 5.0 mol/liter aqueous sodium hydroxide solution and was then extracted three times with chloroform. The organic layers were combined, and the combined organic layers were dried over anhydrous sodium sulfate and were then concentrated under the reduced pressure to give a hemiacetal compound (1.55 g, 86%).

Methylene chloride (35 ml) and 35 ml of methanol were added to the crude hemiacetal compound (1.35 g, 7.21 mmol) to prepare a solution. Ethyl 4-aminobenzoate (899 mg, 5.44 mmol), acetic acid (1.50 ml, 26.2 mmol), and sodium borocyanide (988 mg, 15.7 mmol) were added to the solution, and the mixture was stirred at room temperature for 24 hr. Ethyl 4-aminobenzoate (232 mg, 1.41 mmol), acetic acid (1.25 ml, 21.8 mmol), and sodium borocyanide (848 mg, 13.5 mmol) were added again, and the mixture was stirred at room temperature for 18 hr. Water was added to the reaction solution, and the mixture was extracted twice with chloroform. The extract was dried over anhydrous magnesium sulfate and was then concentrated under the reduced pressure. The residue was purified by column chromatography on silica gel (development system: hexane : ethyl acetate : concentrated aqueous ammonia = 5 : 5 : 0.3) to give the title compound (1.58 g, 65%).
Physicochemical properties of intermediate 9-1

(1) Color and form: Light yellow syrup
(2) Molecular formula: $C_{16}H_{24}N_4O_4$
(3) Mass spectrum (TSPMS): m/z 337 $(M+H)^+$
(4) Specific rotation: $[\alpha]_D^{27}$ +0.40° (c 1.3, $CHCl_3$)
(5) $^1H$ NMR spectrum (400 MHz, $CDCL_3$) δ (ppm): 1.27 (3H, d, H-6'), 1.36 (3H, t, $CH_2CH_3$), 1.93 (2H, m, H-2'), 3.35 (2H, m, H-1' and H-4'), 3.48 (3H, s, $OCH_3$), 3.55 (1H, dt, H-3'), 3.94 (1H, br, H-5'), 4.32 (2H, q, $CH_2CH_3$), 6.56 (2H, m, $C_6H_4$), 7.88 (2H, m, $C_6H_4$)

Intermediate 9-2: Ethyl 4-{(4R)-azido-(5R)-methanesulfonyloxy-(3S)-methoxyhexylamino}benzoate

Methylene chloride (50 ml) was added to intermediate 9-1 (1.63 g, 4.83 mmol) to prepare a solution which was then ice cooled. Triethylamine (2.00 ml, 14.4 mmol) and methanesulfonyl chloride (0.560 ml, 7.23 mmol) were added to the cooled solution. The temperature of the mixture was raised to room temperature, and the mixture was

stirred for 2 hr. Ice was added to the reaction solution, and the mixture was extracted once with chloroform. The organic layer was washed with water, was dried over anhydrous magnesium sulfate, and was then concentrated under the reduced pressure. The residue was purified by column chromatography on silica gel (development system: hexane : ethyl acetate : concentrated aqueous ammonia = 5 : 5 : 0.3) to give the title compound (1.68 g, 84%).
Physicochemical properties of intermediate 9-2

(1) Color and form: Colorless syrup
(2) Molecular formula: $C_{17}H_{26}N_4O_6S$
(3) Mass spectrum (FABMS): m/z 415 $(M+H)^+$
(4) Specific rotation: $[\alpha]_D^{27}$ -16° (c 1.2, $CHCl_3$)
(5) $^1H$ NMR spectrum (400 MHz, $CDCl_3$) δ (ppm): 1.36 (3H, t, $CH_2CH_3$), 1.50 (3H, d, H-6'), 1.97 (2H, m, H-2'), 3.08 (3H, s, $SO_2CH_3$), 3.36 (2H, t, H-1'), 3.42 (1H, dd, H-4'), 3.46 (3H, s, $OCH_3$), 3.55 (1H, dt, H-3'), 4.32 (2H, q, $CH_2CH_3$), 4.97 (1H, dq, H-5'), 6.61 (2H, brd, $C_6H_4$), 7.89 (2H, m, $C_6H_4$)

Intermediate 9-3: Ethyl 4-{(3R)-azido-(4S)-methoxy-(2R)-methylpiperidin-1-yl}benzoate

Toluene (45 ml) was added to intermediate 9-2 (1.87 g, 4.51 mmol) to prepare a solution. Diisopropylethylamine (1.60 ml, 9.19 mmol) was added to the solution, and the mixture was stirred under reflux for 18 hr. The reaction solution was concentrated under the reduced pressure, and the residue was purified by column chromatography on silica gel (development system: hexane : ethyl acetate : concentrated aqueous ammonia = 5 : 5 : 0.3) to give the title compound (1.16 g, 81%).
Physicochemical properties of intermediate 9-3

(1) Color and form: White solid
(2) Molecular formula: $C_{16}H_{22}N_4O_3$
(3) Mass spectrum (FABMS): m/z 319 $(M+H)^+$
(4) Specific rotation: $[\alpha]_D^{28}$ +62° (c 1.2, $CHCl_3$)
(5) $^1H$ NMR spectrum (400 MHz, $CDCl_3$) δ (ppm): 1.14 (3H, d, $CH_3$), 1.36 (3H, t, $CH_2CH_3$), 2.01 (2H, m, piperidine), 3.04 (1H, m, piperidine), 3.46 (3H, s, $OCH_3$), 3.70 (2H, m, piperidine), 3.92 (1H, m, piperidine), 4.32 (1H, m, piperidine), 4.32 (2H, q, $CH_2CH_3$), 6.85 (2H, m, $C_6H_4$), 7.91 (2H, m, $C_6H_4$)

Intermediate 9-4: Ethyl 4-{(3R)-amino-(4S)-methoxy-(2R)-methylpiperidin-1-yl}benzoate

Ethanol (24 ml) was added to intermediate 9-3 (858 mg, 2.70 mmol) to prepare a solution. To the solution was added 76.9 mg of 10% palladium-carbon. The mixture was vigorously stirred under a hydrogen pressure of 1 atm at room temperature for 18 hr. The insolubles were collected by filtration and were then washed with ethanol. The filtrate and the washings were combined, and the combined solution was concentrated under the reduced pressure. The residue was purified by column chromatography on silica gel (development system: ethyl acetate : concentrated aqueous ammonia = 10 : 0.3) to give the title compound (846 mg, 100%).
Physicochemical properties of intermediate 9-4

(1) Color and form: Light yellow syrup
(2) Molecular formula: $C_{16}H_{24}N_2O_3$
(3) Mass spectrum (TSPMS): m/z 293 $(M+H)^+$
(4) Specific rotation: $[\alpha]_D^{26}$ +47° (c 1.1, $CHCl_3$)
(5) $^1H$ NMR spectrum (400 MHz, $CDCl_3$) δ (ppm): 1.11 (3H, d, $CH_3$), 1.37 (3H, t, $CH_2CH_3$), 1.84 (2H, m, piperidine), 3.04 (1H, ddd, piperidine), 3.20 (1H, m, piperidine), 3.40 (3H, s, $OCH_3$), 3.58 (2H, m, piperidine), 4.29 (1H, m, piperidine), 4.32 (2H, q, $CH_2CH_3$), 6.89 (2H, m, $C_6H_4$), 7.91 (2H, m, $C_6H_4$)

Intermediate 9-5: Ethyl 4-{(4S)-methoxy-(2R)-methyl-(3R)-(pyrimidin-2-ylamino)piperidin-1-yl}benzoate

N-Methylpyrrolidone (32 ml) was added to intermediate 9-4 (924 mg, 3.16 mmol) to prepare a solution. Diisopropylethylamine (2.80 ml, 16.1 mmol) and bromopyrimidine (508 mg, 3.19 mmol) were added to the solution, and the mixture was stirred at 120°C for 18 hr. Water was added to the reaction solution, and the mixture was extracted twice with ethyl acetate. The organic layers were combined, and the combined organic layers were dried over anhydrous magnesium sulfate and were then concentrated under the reduced pressure. The residue was purified by column chromatography on silica gel (development system: ethyl acetate) to give the title compound (502 mg, 43%).
Physicochemical properties of intermediate 9-5

(1) Color and form: Yellow syrup
(2) Molecular formula: $C_{20}H_{26}N_4O_3$
(3) Mass spectrum (FABMS): m/z 371 $(M+H)^+$
(4) Specific rotation: $[\alpha]_D^{26}$ -28° (c 1.1, $CHCl_3$)
(5) $^1$H NMR spectrum (400 MHz, $CDCl_3$) δ (ppm): 1.19 (3H, d, $CH_3$), 1.35 (3H, t, $CH_2CH_3$), 1.93 (2H, m, piperidine), 3.13 (1H, ddd, piperidine), 3.38 (3H, s, $OCH_3$), 3.66 (1H, m, piperidine), 3.78 (1H, ddd, piperidine), 4.31 (2H, q, $CH_2CH_3$), 4.51 (2H, m, piperidine), 5.64 (1H, d, NH), 6.54 (1H, t, pyrimidine), 6.80 (2H, m, $C_6H_4$), 7.86 (2H, m, $C_6H_4$), 8.27 (2H, d, pyrimidine)

Intermediate 9-6: 4-{(4S)-Methoxy-(2R)-methyl-(3R)-(pyrimidin-2-ylamino)piperidin-1-yl}benzoic acid

Methanol (4.0 ml) and 2.8 ml of water were added to intermediate 9-5 (491 mg, 1.33 mmol) to prepare a suspension, and 1.35 ml of a 1.0 mol/liter aqueous sodium hydroxide solution was added to the suspension. The mixture was stirred at 50°C for 6 hr and was then ice cooled. The reaction solution was adjusted to pH 4 by the addition of 5.0 mol/liter hydrochloric acid. The precipitated solid was collected by filtration, was washed twice with water, and was then dried to give the title compound (275 mg, 61%).
Physicochemical properties of intermediate 9-6

(1) Color and form: Yellow solid
(2) Molecular formula: $C_{18}H_{22}N_4O_3$
(3) Mass spectrum (EIMS): m/z 342 $(M)^+$
(4) Specific rotation: $[\alpha]_D^{26}$ +191° (c 1.1, $CHCl_3$)
(5) $^1$H NMR spectrum (400 MHz, $CDCl_3$) δ (ppm): 1.25 (3H, d, $CH_3$), 2.03 (1H, m, piperidine), 2.14 (1H, dddd, piperidine), 3.18 (1H, ddd, piperidine), 3.42 (3H, s, $OCH_3$), 3.79 (1H, ddd, piperidine), 3.87 (1H, m, piperidine), 4.37 (1H, m, piperidine), 4.72 (1H, m, piperidine), 6.54 (1H, t, pyrimidine), 6.65 (2H, brd, $C_6H_4$), 7.32 (1H, brd, NH), 7.56 (2H, brd, $C_6H_4$), 8.27 (2H, br, pyrimidine)

Intermediate 9-7: t-Butyl (2S)-benzenesulfonylamino-3-[4-{(4S)-methoxy-(2R)-methyl-(3R)-(pyrimidin-2-ylamino)-piperidin-1-yl}benzoylamino]propionate

Dimethylformamide (4.0 ml) was added to intermediate 9-6 (150 mg, 438 mmol) to prepare a solution, and t-butyl (2S)-N-benzenesulfonyl-2,3-diaminopropionate (148 mg, 493 mmol) was added to the solution. Further, benzotriazol-1-yloxytri(dimethylamino)phosphonium hexafluorophosphate (BOP) (241 mg, 545 mmol) and diisopropylethylamine (0.0920 ml, 0.528 mmol) were added thereto, and the mixture was stirred at room temperature for 18 hr. Water and a saturated aqueous sodium hydrogencarbonate solution were added to the reaction solution, and the mixture was extracted twice with ethyl acetate. The organic layers were combined, and the combined organic layers were washed with a mixed solution composed of saturated brine and water, were dried over anhydrous sodium sulfate, and were then concentrated under the reduced pressure. The residue was purified by column chromatography on silica gel (development system: methylene chloride : methanol = 7 : 1) to give the title compound (250 mg, 91%).
Physicochemical properties of intermediate 9-7

(1) Color and form: Yellow syrup
(2) Molecular formula: $C_{31}H_{40}N_6O_6S$
(3) Mass spectrum (FABMS): m/z 625 $(M+H)^+$
(4) Specific rotation: $[\alpha]_D^{27}$ +46° (c 0.99, $CHCl_3$)
(5) $^1$H NMR spectrum (400 MHz, $CHCl_3$) δ (ppm): 1.18 (3H, d, $CH_3$), 1.26 (9H, s, t-Bu), 1.89 (1H, m, piperidine), 2.00 (1H, m, piperidine), 3.11 (1H, ddd, piperidine), 3.39 (3H, s, $OCH_3$), 3.58 (2H, m, piperidine), 3.78 (1H, ddd, $CONHC\underline{H_2}CH$), 3.85 (1H, ddd, $CONHC\underline{H_2}CH$), 3.92 (1H, ddd, $CONHCH_2C\underline{H}$), 4.49 (2H, m, piperidine), 5.72 (1H, d, NH), 5.98 (1H, d, NH), 6.54 (1H, t, pyrimidine), 6.58 (1H, dd, NH), 6.81 (2H, m, $C_6H_4$), 7.46 (2H, m, $C_6H_5$), 7.55 (1H, m, $C_6H_5$), 7.62 (2H, m, $C_6H_4$), 7.85 (2H, m, $C_6H_5$), 8.29 (2H, d, pyrimidine)

Intermediate 9-8: (2S)-Benzenesulfonylamino-3-[4-{(4S)-methoxy-(2R)-methyl-(3R)-(pyrimidin-2-ylamino)piperidin-1-yl}benzoylamino]propionic acid

Methylene chloride (3.0 ml) was added to intermediate 9-7 (103 mg, 0.166 mmol) to prepare a solution. Trifluoroacetic acid (3.0 ml) was added to the solution, and the mixture was stirred at room temperature for 3 hr. The reaction solution was concentrated under the reduced pressure to give a trifluoroacetate of the title compound.
Physicochemical properties of intermediate 9-8

(1) Color and form: Colorless liquid
(2) Molecular formula: $C_{27}H_{32}N_6O_6S$
(3) Mass spectrum (TSPMS): m/z 569 $(M+H)^+$
(4) $^1$H NMR spectrum (400 MHz, $CD_3OD$) (as trifluoroacetate) δ (ppm): 1.21 (3H, d, $CH_3$), 2.03 (1H, m, piperidine), 2.15 (1H, m, piperidine), 3.44 (3H, s, $OCH_3$), 3.49 (1H, m), 3.76 (2H, m), 3.90 (1H, ddd), 4.20 (2H, m), 4.40 (1H, m), 4.73 (1H, m), 6.96 (1H, t, pyrimidine), 7.20 (2H, d, $C_6H_4$), 7.44 (2H, m, $C_6H_5$), 7.51 (1H, m, $C_6H_5$), 7.76 (2H, d, $C_6H_4$), 7.83 (2H, m, $C_6H_5$), 8.57 (2H, br, pyrimidine)

Title compound (Synthesis Example 9): (2S)-Benzenesulfonylamino-3-[4-{(4S)-methoxy-(2R)-methyl-(3R)-(1,4,5,6-tetrahydropyrimidin-2-ylamino)piperidin-1-yl}benzoylamino]propionic acid

Dioxane (3.0 ml) and 0.30 ml of water were added to the trifluoroacetate of intermediate 9-8 as the crude compound (0.166 mmol) to prepare a solution. To the solution was added 18.1 mg of 10% palladium-carbon. The mixture was vigorously stirred under a hydrogen pressure of 1 atm at room temperature for 18 hr. The insolubles were collected by filtration and were then washed with ethanol. The filtrate and the washings were combined, and the combined solution was concentrated under the reduced pressure. The residue was purified by column chromatography on silica gel (development system: chloroform : methanol : concentrated aqueous ammonia = 9 : 3 : 0.3) and was then purified by. Sephadex LH-20 (development system: methanol) to give the title compound (62.6 mg, 66%).
Physicochemical properties of the title compound

(1) Color and form: White solid
(2) Molecular formula: $C_{27}H_{36}N_6O_6S$
(3) Mass spectrum (TSPMS): m/z 573 $(M+H)^+$
(4) Specific rotation: $[\alpha]_D^{25}$ +136° (c 0.15, MeOH)
(5) $^1$H NMR spectrum (400 MHz, $CD_3OD$) δ (ppm): 1.15 (3H, d, $CH_3$), 1.70 (1H, ddd, piperidine), 1.94 (2H, quintet, tetrahydropyrimidine), 2.10 (1H, m, piperidine), 3.08 (1H, m, piperidine), 3.36 (4H, t, tetrahydropyrimidine), 3.41 (3H, s, $OCH_3$), 3.47 (1H, m), 3.55 (1H, m), 3.64 (2H, m), 3.81 (1H, m), 3.87 (1H, m), 4.26 (1H, m), 6.95 (2H, d, $C_6H_4$), 7.47 (2H, m, $C_6H_5$), 7.54 (1H, dd, $C_6H_5$), 7.68 (2H, d, $C_6H_4$), 7.85 (2H, m, $C_6H_5$)

Synthesis Example 10: (2S)-Benzenesulfonylamino-3-[3-{(3R)-hydroxy-(4R)-(1,4,5,6-tetrahydropyrimidin-2-ylamino)piperidin-1-yl)benzoylamino]propionic acid

Intermediate 10-1: Ethyl 3-{(3S,4R)-3,4,5-(trihydroxy)pentan-1-ylamino}benzoate

Methanol (50 ml) was added to 1.34 g of 2-deoxy-D-ribose to prepare a solution. Separately, 50 ml of methylene chloride was added to 1.60 g of ethyl 3-aminobenzoate to prepare a solution which was then added to the above methanol solution. A reaction was allowed to proceed at room temperature for 16 hr. Acetic acid (1.0 ml) and 500 mg of sodium boron cyanohydride were then added thereto in that order, and a reaction was allowed to proceed at room temperature for 4 hr. The reaction solution was concentrated under the reduced pressure, and the residue was extracted with 300 ml of chloroform. The organic layer was washed with 200 ml of a saturated aqueous sodium hydrogencarbonate solution containing a minor amount of sodium chloride. The aqueous layer was subjected to back extraction with 100 ml of chloroform. The chloroform layers were combined and were then dried over anhydrous sodium sulfate, followed by concentration under the reduced pressure. The residue was purified by column chromatography on silica gel (100 g, chloroform : methanol : concentrated aqueous ammonia = 10 : 1 : 0.1 → 10 : 1.3 : 0.1) to give 2.23 g of the title compound.
Physicochemical properties of intermediate 10-1

(1) Color and form: Colorless solid
(2) Molecular formula: $C_{14}H_{21}NO_5$
(3) Mass spectrum (TSPMS): m/z 284 $(M+H)^+$
(4) Specific rotation: $[\alpha]_D^{25}$ -17° (c 1.0, $CHCl_3$)
(5) $^1$H NMR spectrum (400 MHz, $CDCl_3$) δ (ppm): 1.36 (3H, t, Et), 1.80 (2H, m, $NHCH_2CH_2$), 3.32 (2H, m, $NHCH_2$), 3.62 (1H, br s, CHOH), 3.77 (2H, br s, $CH_2OH$), 3.89 (1H, br s, CHOH), 4.33 (2H, q, Et), 6,78 (1H, br dd, $C_6H_4$), 7.20 (1H, t, $C_6H_4$), 7.29 (1H, br s, $C_6H_4$), 7.37 (1H, br d, $C_6H_4$)

Intermediate 10-2: Ethyl 3-{(3R,4S)-3,4-(dihydroxy)-piperidin-1-yl}benzoate

Tetrahydrofuran (15 ml) was added to 372 mg of intermediate 10-1 to prepare a solution. Carbon tetrabromide

(653 mg) was added to the solution. The mixture was cooled to 0°C, and 689 mg of triphenylphosphine was then added thereto. The temperature of the mixture was gradually raised to room temperature over a period of one hr. The reaction solution was concentrated under the reduced pressure, and the residue was purified by column chromatography on silica gel (40 g, chloroform : methanol : concentrated aqueous ammonia = 20 : 1 : 0.05) to give the title compound as a crude compound. The crude compound was purified by preparative thin-layer chromatography on silica gel (development system: chloroform : methanol : benzene : ethyl acetate = 9 : 1 : 6 : 4) to give 157 mg of the title compound.
Physicochemical properties of intermediate 10-2

(1) Color and form: Colorless syrup
(2) Molecular formula: $C_{14}H_{19}NO_4$
(3) Mass spectrum (FABMS): m/z 266 $(M+H)^+$
(4) Specific rotation: $[\alpha]_D^{25}$ +3° (c 1.0, $CHCl_3$)
(5) $^1H$ NMR spectrum (400 MHz, $CDCl_3$) δ (ppm): 1.39 (3H, t, Et), 1.94 (2H, m, $NCH_2CH_2$), 2.99 (1H, m, $NCH_2CH_2$), 3.16 (1H, dd, $NCH_2CHOH$), 3.42 (1H, m, $NCH_2CH_2$), 3.51 (1H, ddd, $NCH_2CHOH$), 3.84 (1H, br s, CHOH), 3.95 (1H, br s, CHOH), 4.37 (2H, q, Et), 7.14 (1H, br ddd, $C_6H_4$), 7.32 (1H, t, $C_6H_4$), 7.56 (1H, br ddd, $C_6H_4$), 7.63 (1H, br dd, $C_6H_4$)

Intermediate 10-3: Ethyl 3-{(3R)-acetoxy-(4S)-hydroxypiperidin-1-yl}benzoate

Intermediate 10-4: Ethyl 3-{(4S)-acetoxy-(3R) hydroxypiperidin-1-yl}benzoate

Trimethyl orthoacetate (0.50 ml) was added to intermediate 10-2 (134 mg, 0.51 mmol) to prepare a suspension. p-Toluenesulfonic acid monohydrate (15.4 mg) was added to the suspension at room temperature, and a reaction was allowed to proceed for 3 hr. The reaction solution was concentrated under the reduced pressure. Acetic acid (1.0 ml) was then added to the residue at room temperature, and a reaction was allowed to proceed for 45 min. Water (100 ml) was then added thereto. The mixture was extracted twice with 100 ml of ethyl acetate. The organic layers were combined, and the combined organic layers were washed with 100 ml of saturated brine, were dried over anhydrous magnesium sulfate, and were then concentrated under the reduced pressure. The residue was purified by column chromatography on silica gel (development system: methylene chloride : methanol : benzene : ethyl acetate = 9 : 1 : 6 : 4) to give intermediate 10-3 (47 mg, 30%) and intermediate 10-4 (86 mg, 55%).
Physicochemical properties of intermediate 10-3

(1) Color and form: Colorless syrup
(2) Molecular formula: $C_{16}H_{21}NO_5$
(3) Mass spectrum (EIMS): m/z 307 $(M)^+$
(4) Specific rotation: $[\alpha]_D^{25}$ -25° (c 1.1, $CH_2Cl_2$)
(5) $^1H$ NMR spectrum (400 MHz, $CDCl_3$) δ (ppm): 1.39 (3H, t, Et), 1.90 - 2.04 (2H, m, piperidine), 2.10 (3H, s, acetyl), 3.20 - 3.28 (1H, m, piperidine), 3.88 (1H, dd, piperidine), 3.44 (1H, ddd, piperidine), 3.52 (1H, dd, piperidine), 4.07 (1H, dddd, piperidine), 4.37 (2H, q, Et), 5.04 (1H, ddd, piperidine), 7.12 (1H, ddd, $C_6H_4$), 7.30 (1H, dd, $C_6H_4$), 7.51 (1H, ddd, $C_6H_4$), 7.60 (1H, dd, $C_6H_4$)

Physicochemical properties of intermediate 10-4

(1) Color and form: Colorless syrup
(2) Molecular formula: $C_{16}H_{21}NO_5$
(3) Mass spectrum (EIMS): m/z 307 $(M)^+$
(4) Specific rotation: $[\alpha]_D^{25}$ +4.9° (c 1.1, $CH_2Cl_2$)
(5) $^1H$ NMR spectrum (400 MHz, $CDCl_3$) δ (ppm): 1.39 (3H, t, Et), 1.94 - 1.99 (1H, m, piperidine), 2.07 - 2.16 (1H, m, piperidine), 2.15 (3H, s, acetyl), 3.07 (1H, ddd, piperidine), 3.23 (1H, dd, piperidine), 3.43 (1H, m, piperidine), 3.50 (1H, ddd, piperidine), 4.08 (1H, br s, piperidine), 4.38 (2H, q, Et), 5.00 (1H, ddd, piperidine), 7.16 (1H, dd, $C_6H_4$), 7.33 (1H, dd, $C_6H_4$), 7.58 (1H, m, $C_6H_4$), 7.64 (1H, m, $C_6H_4$)

Intermediate 10-5: Ethyl 3-{(3R)-acetoxy-(4S)-methanesulfonyloxypiperidin-1-yl}benzoate

Methylene chloride (3.0 ml) was added to intermediate 10-3 (47 mg, 0.15 mmol) to prepare a solution. Triethylamine (45 μl, 0.32 mmol) and methanesulfonyl chloride (15 μl, 0.20 mmol) were added to the solution at room temperature, and a reaction was allowed to proceed for 5 min. Water (100 ml) was added thereto, and the mixture was extracted twice with 50 ml of methylene chloride. The combined organic layers were dried over anhydrous magnesium

sulfate and were concentrated under the reduced pressure to give the title compound (40 mg, 67%).
Physicochemical properties of intermediate 10-5

(1) Color and form: Light yellow syrup
(2) Molecular formula: $C_{17}H_{23}NO_7S$
(3) Mass spectrum (EIMS): m/z 385 $(M)^+$
(4) $^1H$ NMR spectrum (400 MHz, $CDCl_3$) δ (ppm): 1.40 (3H, t, Et), 2.10 (3H, s, acetyl), 2.08 - 2.16 (1H, m, piperidine), 2.20 - 2.30 (1H, m, piperidine), 3.09 (3H, s, Ms), 3.30 - 3.46 (3H, m, piperidine), 3.50 - 3.56 (1H, m, piperidine), 4.38 (2H, q, Et), 5.08 - 5.15 (2H, m, piperidine), 7.13 (1H, dd, $C_6H_4$), 7.33 (1H, dd, $C_6H_4$), 7.56 (1H, br d, $C_6H_4$), 7.60 - 7.62 (1H, m, $C_6H_4$)

Intermediate 10-6: Ethyl 3-{(3R)-acetoxy-(4R)-azidopiperidin-1-yl}benzoate

Dimethylformamide (2.0 ml) was added to intermediate 10-5 (39 mg, 0.10 mmol) to prepare a solution. Sodium azide (15 mg, 0.23 mmol) was added to the solution, and a reaction was allowed to proceed at 90°C for 10 hr. The reaction mixture was returned to room temperature, 100 ml of water was then added thereto, and the mixture was extracted twice with 70 ml of ethyl acetate. The organic layers were then combined, and the combined organic layers were washed twice with 100 ml of water and once with 100 ml of saturated brine, were then dried over anhydrous magnesium sulfate, and were concentrated under the reduced pressure. The residue was then purified by column chromatography on silica gel (development system: hexane : ethyl acetate = 1 : 1) to give the title compound (34 mg, 100%).
Physicochemical properties of intermediate 10-6

(1) Color and form: Colorless syrup
(2) Molecular formula: $C_{16}H_{20}N_4O_4$
(3) Mass spectrum (EIMS): m/z 332 $(M)^+$
(4) Specific rotation: $[\alpha]_D^{25}$ -10° (c 1.7, $CH_2Cl_2$)
(5) $^1H$ NMR spectrum (400 MHz, $CDCl_3$) δ (ppm): 1.39 (3H, t, Et), 1.76 (1H, dddd, piperidine), 2.08 - 2.13 (1H, m, piperidine), 2.14 (3H, s, acetyl), 2.80 (1H, dd, piperidine), 2.93 (1H, ddd, piperidine), 3.60 (1H, ddd, piperidine), 3.69 (1H, dddd, piperidine), 3.89 (1H, ddd, piperidine), 4.37 (2H, q, Et), 4.90 (1H, ddd, piperidine), 7.12 (1H, dd, $C_6H_4$), 7.32 (1H, dd, $C_6H_4$), 7.55 (1H, br d, $C_6H_4$), 7.57 - 7.60 (1H, m, $C_6H_4$)

Intermediate 10-7: Ethyl 3-{(4R)-azido-(3R)-hydroxy}piperidin-1-yl}benzoate

Tetrahydrofuran (11 ml) was added to intermediate 10-6 (390 mg, 1.2 mmol) to prepare a solution. Sodium ethoxide (99 mg, 1.4 mmol) was added to the solution, and a reaction was allowed to proceed at 30°C for 3.5 hr. The reaction solution was adjusted to pH 4 by the addition of 1.0 M hydrochloric acid, and 100 ml of water was added thereto. The mixture was extracted twice with 150 ml of ethyl acetate. The organic layers were then combined, and the combined organic layers were washed with 150 ml of saturated brine, were dried over anhydrous magnesium sulfate, and were concentrated under the reduced pressure to give the title compound (346 mg, 100%).
Physicochemical properties of intermediate 10-7

(1) Color and form: Light yellow syrup
(2) Molecular formula: $C_{14}H_{18}N_4O_3$
(3) Mass spectrum (EIMS): m/z 290 $(M)^+$
(4) $^1H$ NMR spectrum (400 MHz, $CDCl_3$) δ (ppm): 1.39 (3H, t, Et), 1.79 (1H, dddd, piperidine), 2.15 (1H, dddd, piperidine), 2.84 (1H, ddd, piperidine), 2.94 (1H, ddd, piperidine), 3.45 (1H, ddd, piperidine), 3.60 (1H, dddd, piperidine), 3.72 (1H, dd, piperidine), 3.76 (1H, ddd, piperidine), 4.37 (2H, q, Et), 7.11 (1H, dd, $C_6H_4$), 7.32 (1H, dd, $C_6H_4$), 7.56 (1H, ddd, $C_6H_4$), 7.60 (1H, dd, $C_6H_4$)

Intermediate 10-8: Ethyl 3-{(4R)-amino-(3R)-hydroxypiperidin-1-yl}benzoate

1,4-Dioxane (1.0 ml) and 0.5 ml of water were successively added to intermediate 10-7 (11 mg, 0.039 mmol) to prepare a solution. To the solution was added 10% palladium-carbon (3.0 mg). The mixture was stirred in a hydrogen atmosphere at room temperature for 3 hr. The insolubles were collected by filtration and were washed with 20 ml of a solvent having the same composition as the mixed solvent used in the reaction. The filtrate and the washings were combined, followed by concentration under the reduced pressure to give the title compound (11 mg, 100%).
Physicochemical properties of intermediate 10-8

(1) Color and form: Colorless syrup
(2) Molecular formula: $C_{14}H_{20}N_2O_3$
(3) Mass spectrum (FABMS): m/z 265 $(M+H)^+$
(4) $^1H$ NMR spectrum (400 MHz, $CDCl_3$) δ (ppm): 1.39 (3H, t, Et), 1.50 - 1.62 (1H, m, piperidine), 1.93 - 2.01 (1H, m, piperidine), 2.57 - 2.70 (2H, m, piperidine), 2.83 (1H, ddd, piperidine), 3.44 (1H, ddd, piperidine), 3.65 - 3.72 (1H, m, piperidine), 3.86 - 3.93 (1H, m, piperidine), 4.37 (2H, q, Et), 7.11 (1H, dd, $C_6H_4$), 7.32 (1H, dd, $C_6H_4$), 7.51 (1H, ddd, $C_6H_4$), 7.60 (1H, dd, $C_6H_4$)

Intermediate 10-9: Ethyl 3-{(3R)-hydroxy-(4R)-(pyrimidin-2-yl)piperidin-1-ylamino}benzoate

Dimethyl sulfoxide (3.0 ml) was added to intermediate 10-8 (87 mg, 0.33 mmol) to prepare a solution. 2-Bromopyrimidine (55 mg, 0.33 mmol) and diisopropylethylamine (320 μl, 1.85 mmol) were successively added to the solution, and a reaction was allowed to proceed at 120°C for 14 hr. The temperature of the reaction mixture was returned to room temperature, and 500 ml of water was then added thereto, followed by extraction three times with 250 ml of ethyl acetate. The organic layers were then combined, and the combined organic layers were washed twice with 200 ml of water and twice with 200 ml of saturated brine, were then dried over anhydrous magnesium sulfate, and were concentrated under the reduced pressure. The residue was purified by column chromatography on silica gel (development system: benzene : ethyl acetate = 1 : 4) to give the title compound (51 mg, 45%).
Physicochemical properties of intermediate 10-9

(1) Color and form: Colorless solid
(2) Molecular formula: $C_{18}H_{22}N_4O_3$
(3) Mass spectrum (TSPMS): m/z 343 $(M+H)^+$
(4) $^1H$ NMR spectrum (400 MHz, $CDCl_3$) δ (ppm): 1.39 (3H, t, Et), 1.80 (1H, dddd, piperidine), 2.14 (1H, dddd, piperidine), 2.74 (1H, dd, piperidine), 2.89 (1H, ddd, piperidine), 3.72 - 3.86 (3H, m, piperidine), 3.97 (1H, ddd, piperidine), 4.37 (2H, q, Et), 6.64 (1H, t, pyrimidine), 7.14 (1H, dd, $C_6H_4$), 7.32 (1H, dd, $C_6H_4$), 7.53 (1H, ddd, $C_6H_4$), 7.63 (1H, dd, $C_6H_4$), 8.29 (2H, d, pyrimidine)

Intermediate 10-10: t-Butyl (2S)-benzenesulfonylamino-3-[3-{(3R)-hydroxy-(4R)-(pyrimidin-2-ylamino)piperidin-1-yl} benzoylamino] propionate

Tetrahydrofuran (1.8 ml) and 0.60 ml of methanol were successively added to intermediate 10-9 (49 mg, 0.14 mmol) to prepare a solution, and a 1.0 M aqueous sodium hydroxide solution (0.60 ml) was added to the solution. A reaction was allowed to proceed at 50°C for one hr. The temperature of the reaction mixture was then returned to room temperature, and the reaction mixture was adjusted to pH 4 by the addition of 1.0 M hydrochloric acid and was concentrated under the reduced pressure to give 3-{(3R)-hydroxy-(4R)-(pyrimidin-2-ylamino)piperidin-1-yl}benzoic acid. Dimethylformamide (7.0 ml) was added to this product to prepare a solution. Benzotriazol-1-yloxytri(dimethylamino) phosphonium hexafluorophosphate (98 mg, 0.21 mmol) and diisopropylethylamine (40 μl, 0.22 mmol) were added to the solution, and a reaction was allowed to proceed at room temperature for 30 min. Further, t-butyl (2S)-N-benzenesulfonyl-2,3-diaminopropionate (55 mg, 0.18 mmol) was added to the above active ester solution at room temperature, and a reaction was allowed to proceed at room temperature for one hr. A saturated aqueous sodium hydrogencarbonate solution (20 ml) was then added thereto, followed by extraction twice with 100 ml of ethyl acetate. The organic layers were combined, and the combined organic layers were washed with 100 ml of saturated brine, were dried over anhydrous magnesium sulfate, and were then concentrated under the reduced pressure. The residue was purified by column chromatography on silica gel (development system: methylene chloride : methanol = 20 : 1) to give the title compound (18 mg, 21%).
Physicochemical properties of intermediate 10-10

(1) Color and form: Light yellow solid
(2) Molecular formula: $C_{29}H_{36}N_6O_6S$
(3) Mass spectrum (TSPMS): m/z 597 $(M+H)^+$
(4) Specific rotation: $[\alpha]_D^{25}$ +75° (c 0.48, $CH_2Cl_2$)
(5) $^1H$ NMR spectrum (400 MHz, $CDCl_3$) δ (ppm): 1.29 (9H, s, t-Bu), 1.72 - 1.83 (1H, m, piperidine), 2.09 - 2.15 (1H, m, piperidine), 2.75 (1H, dd, piperidine), 2.90 (1H, ddd, piperidine), 3.54 - 3.63 (1H, m, piperidine), 3.70 - 3.83 (3H, m, piperidine and $CONHCH_2CH$), 3.86 - 3.95 (2H, m, piperidine and $CONHCH_2$), 3.98 (1H, ddd, piperidine), 6.62 (1H, t, pyrimidine), 7.10 (1H, dd, $C_6H_4$), 7.18 (1H, br d, $C_6H_4$), 7.31 (1H, dd, $C_6H_4$), 7.43 (1H, dd, $C_6H_4$), 7.47 - 7.60 (3H, m, $C_6H_5$), 7.84 - 7.88 (2H, m, $C_6H_5$), 8.29 (2H, d, pyrimidine)

Intermediate 10-11: (2S)-Benzenesulfonylamino-3-[3-{(3R)-hydroxy-(4R)-(pyrimidin-2-ylamino)piperidin-1-yl}-benzoylamino]propionic acid

Methylene chloride (2.0 ml) was added to intermediate 10-10 (16 mg, 0.027 mmol) to prepare a solution, and 2.0 ml of trifluoroacetic acid was added to the solution at room temperature. A reaction was allowed to proceed for 7 hr, and the reaction solution was then concentrated under the reduced pressure to give the title compound (18 mg, 75% (as tritrifluoroacetate)). Physicochemical properties of intermediate 10-11 (as tritrifluoroacetate)

(1) Color and form: Light yellow solid
(2) Molecular formula: $C_{25}H_{28}N_6O_6S$
(3) Mass spectrum (TSPMS): m/z 541 $(M+H)^+$
(4) Specific rotation: $[\alpha]_D^{25}$ +26° (c 0.50, $CH_3OH$)
(5) $^1H$ NMR spectrum (400 MHz, $CD_3OD$) δ (ppm): 1.76 (1H, dddd, piperidine), 2.15 - 2.22 (1H, m, piperidine), 2.76 (1H, dd, piperidine), 2.91 (1H, ddd, piperidine), 3.50 (1H, dd, $CONHCH_2$), 3.73 (1H, dd, $CONHCH_2$), 3.72 - 3.83 (2H, m, piperidine), 3.86 - 3.96 (2H, m, piperidine), 4.21 (1H, dd, $CONHCH_2CH$), 6.77 (1H, t, pyrimidine), 7.18 (1H, dd, $C_6H_4$), 7.21 (1H, d, $C_6H_4$), 7.32 (1H, dd, $C_6H_4$), 7.40 - 7.47 (3H, m, $C_6H_4$ and $C_6H_5$), 7.48 - 7.54 (1H, m, $C_6H_5$), 7.81 - 7.86 (2H, m, $C_6H_5$), 8.39 (2H, d, pyrimidine)

Title compound (Synthesis Example 10): (2S)-Benzenesulfonylamino-3-[3-{(3R)-hydroxy-(4R)-(1,4,5,6 tetrahydropyrimidin-2-ylamino)piperidin-1-yl}benzoylamino]propionic acid

1,4-Dioxane (2.0 ml) and 1.0 ml of water were successively added to 15 mg of intermediate 10-11 to prepare a solution. To the solution was added 10% palladium-carbon (4.4 mg). A reaction was then allowed to proceed at room temperature in a hydrogen atmosphere for 4 hr. The insolubles were collected by filtration and were washed with 90 ml of a solvent having the same composition as the mixed solvent used in the reaction. The filtrate and the washings were combined followed by concentration under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (3.1 mg, 28%). Physicochemical properties of the title compound

(1) Color and form: Colorless solid
(2) Molecular formula: $C_{25}H_{32}N_6O_6S$
(3) Mass spectrum (TSPMS): m/z 545 $(M+H)^+$
(4) Specific rotation: $[\alpha]_D^{25}$ +70° (c 0.14, $CH_3OH$)
(5) $^1H$ NMR spectrum (400 MHz, $CD_3OD$) δ (ppm): 1.68 (1H, dddd, piperidine), 1.95 (2H, dddd, tetrahydropyrimidine), 1.92 - 2.02 (1H, m, piperidine), 2.67 (1H, dd, piperidine), 2.83 (1H, ddd, piperidine), 3.26 - 3.32 (1H, m, piperidine), 3.36 (4H, br t, tetrahydropyrimidine), 3.50 - 3.57 (1H, m, piperidine), 3.56 (1H, dd, $CONHCH_2$), 3.67 (1H, dd, $CONHCH_2$), 3.74 (1H, dd, $CONHCH_2CH$), 3.77- 3.85 (1H, m, piperidine), 3.91 (1H, m, piperidine), 7.11 (1H, ddd, $C_6H_4$), 7.24 (1H, ddd, $C_6H_4$), 7.31 (1H, dd, $C_6H_4$), 7.42 (1H, dd, $C_6H_4$), 7.46 - 7.52 (2H, m, $C_6H_5$), 7.52 - 7.58 (1H, m, $C_6H_5$), 7.85 - 7.89 (2H, m, $C_6H_5$)

Pharmacological Test Example 1: Inhibitory activity against adhesion of vascular smooth muscle cells to vitronectin

At the outset, experiments were made on the mechanism of the occurrence of cardiac tissue injury and micro circulation injury which are considered as a direct cause of reperfusion injury. Adhesion inhibitory activity on a cell level was examined for the compounds represented by formulae (IV), (V), (VI), (VII), (VIII), (IX), (X), and (XI) which have been reported to have high integrin $\alpha_v\beta_3$ receptor binding inhibitory activity.

The adhesion of vascular smooth muscle cells to immobilized human vitronectin was measured in accordance with the method of Liaw et al. (L. Liaw et al., Circ. Res., 74, 214 (1994)). A Dulbecco's phosphate buffer (Dulbecco's PBS(-)) solution of human serum-derived vitronectin adjusted to a concentration of 4 μg/ml was first added to wells (50 μl/well) of a microplate (Maxisorp), and a reaction for immobilization was allowed to proceed at 4°C overnight. After washing twice with 150 μl of Dulbecco's PBS (-), Dulbecco's PBS (-) containing 10 mg/ml of bovine serum albumin was added, followed by blocking at 37°C for one hr. The microplate was then washed twice with 150 μl of Dulbecco's PBS (-) to prepare an assay plate.

Separately, human vascular smooth muscle cells cultivated at 37°C under 5% carbon dioxide in a medium for vascular smooth muscle cells were separated using a Dulbecco's PBS (-) containing trypsin-EDTA, were washed with Dulbecco's PBS (-), and were then suspended in a Dulbecco's modified Eagle's basal medium to a concentration of 2 x $10^5$/ml.

Next, 50 μl of a Dulbecco's modified Eagle's basal medium containing 20 mg/ml of bovine serum albumin with a medicament added thereto was added to the wells of the human vitronectin-coated assay microplate, followed by pre-cultivation under 5% carbon dioxide at 37°C for 10 min. Thereafter, 50 μl of the Dulbecco's modified Eagle's basal medium with human vascular smooth muscle cells suspended therein was added thereto, and the plate was thoroughly stirred. A reaction was allowed to proceed under 5% carbon dioxide at 37°C for 90 min. The reaction solution containing non-adherent cells was then removed, followed by washing three times with Dulbecco's PBS (-). For the adhered cells, 100 μl of Dulbecco's PBS (-) containing 4% paraformaldehyde was added, and immobilization was allowed to proceed at room temperature for 10 min. Next, 100 μl of a Dulbecco's PBS (-) containing 0.5% Toluidine Blue and 4% paraformaldehyde was added, and staining was allowed to proceed at room temperature for 5 min, followed by thorough washing with distilled water. The inside of the wells was then air-dried, and 1% aqueous sodium dodecylsulfate solution was then added to perform cytolysis. The absorbance of the microplate thus obtained was measured at 595 nm. The total binding was defined as the absorbance of the well not containing the test compound, and the non-specific binding (100% inhibition) was defined as the absorbance of the well which does not contain vitronectin and has been subjected to blocking with bovine serum albumin. The inhibition was calculated by the following equation. $IC_{50}$ was determined from a primary regression line of the logarithm of each concentration of the test compound and the logarithm of (100 - inhibition)/inhibition.

$$\text{Inhibition (\%)} = 100 - \frac{\text{(absorbance in the presence of test compound - non-specific binding)}}{\text{(total binding - non-specific binding)}} \times 100$$

Rat vascular smooth muscle cells were cultivated in a medium composed of a mixture of equal amounts of 15% fetal calf serum-containing 199 medium and RPMI 1640 medium. Separately, hamster and dog vascular smooth muscle cells were cultivated in a 10% fetal calf serum-containing Dulbecco's modified Eagle's basal medium. These cultivated cells were then suspended in a Dulbecco's modified Eagle's basal medium to concentrations of $3 \times 10^5$ cells/ml (rat), $3 \times 10^5$ cells/ml (hamster), and $4 \times 10^5$ cells /ml (dog), respectively. Rat vitronectin was used in the adhesion system of rats and hamsters, while bovine vitronectin was used in the adhesion system of dogs.

The concentration ($IC_{50}$) necessary for inhibition of adhesion to vitronectin and the concentration ($IC_{50}$) necessary for inhibition of platelet aggregation of human vascular smooth muscle cells were respectively $3.7 \times 10^{-8}$ M and $5.5 \times 10^{-8}$ M for the compound represented by formula (IV), $1.9 \times 10^{-7}$ M and $6.3 \times 10^{-8}$ M for the compound represented by formula (V), $7.2 \times 10^{-8}$ M and $1.8 \times 10^{-7}$ M for the compound represented by formula (VI), $6.9 \times 10^{-8}$ M and $2.3 \times 10^{-7}$ M for the compound represented by formula (VII), $1.3 \times 10^{-7}$ M and $1.3 \times 10^{-7}$ M for the compound represented by formula (VIII), $2.8 \times 10^{-8}$ M and $5.6 \times 10^{-8}$ M for the compound represented by formula (IX), $1.3 \times 10^{-8}$ M and $4.1 \times 10^{-7}$ M for the compound represented by formula (X), and $2.4 \times 10^{-8}$ M and $9.5 \times 10^{-8}$ M for the compound represented by formula (XI). On the other hand, the concentration ($IC_{50}$) necessary for inhibition of adhesion to vitronectin and the concentration ($IC_{50}$) necessary for inhibition of platelet aggregation of rat vascular smooth muscle cells were respectively $2.3 \times 10^{-7}$ M and $> 1.0 \times 10^{-4}$ M for the compound represented by formula (IV), $8.0 \times 10^{-7}$ M and $1.3 \times 10^{-5}$ M for the compound represented by formula (V), $4.5 \times 10^{-7}$ M and $> 1.0 \times 10^{-4}$ M for the compound represented by formula (VI), and $6.4 \times 10^{-7}$ M and $> 1.0 \times 10^{-4}$ M for the compound represented by formula (VII), demonstrating that the platelet aggregation inhibitory activity was very low for rats. Further, the concentration ($IC_{50}$) necessary for inhibition of adhesion to vitronectin of rat vascular smooth muscle cells was $7.6 \times 10^{-7}$ M for the compound represented by formula (VIII), $9.6 \times 10^{-8}$ M for the compound represented by formula (IX), and $9.6 \times 10^{-8}$ M for the compound represented by formula (X). Furthermore, it was found that the concentration ($IC_{50}$) necessary for inhibition of adhesion to vitronectin and the concentration ($IC_{50}$) necessary for inhibition of platelet aggregation of hamster vascular smooth muscle cells were respectively $7.3 \times 10^{-7}$ M and $1.9 \times 10^{-6}$ M for the compound represented by formula (IV) and that the concentration ($IC_{50}$) necessary for inhibition of adhesion to vitronectin and the concentration ($IC_{50}$) necessary for inhibition of platelet aggregation of dog vascular smooth muscle cells were respectively $2.6 \times 10^{-8}$ M and $4.5 \times 10^{-8}$ M for the compound represented by formula (IV), $1.8 \times 10^{-8}$ M and $1.9 \times 10^{-7}$ M for the compound represented by formula (VII), $2.8 \times 10^{-8}$ M and $5.6 \times 10^{-8}$ M for the compound represented by formula (VIII), $1.1 \times 10^{-8}$ M and $5.9 \times 10^{-8}$ M for the compound represented by formula (IX), and $9.7 \times 10^{-9}$ M and $1.1 \times 10^{-7}$ M for the compound represented by formula (X).

The above results demonstrate that, in rats, hamster, dog, and human cell assay systems, various integrin $\alpha_v\beta_3$ antagonists inhibit the adhesion of vascular smooth muscle cells to vitronectin. That is, it was substantiated that, in a receptor binding assay system, integrin $\alpha_v\beta_3$ antagonists were also active on a cell level.

Pharmacological Test Example 2: Inhibition of adhesion of rat macrophage to vitronectin

Experiments were made on adhesion and invasion of leukocytes which are considered as a direct cause of cardiac tissue injury and minimal circulation injury.

The adhesion of rat macrophage to immobilized rat vitronectin was measured in accordance with the method

of Liaw et al. (L. Liaw et al., Circ. Res., 74, 214 (1994)). A Dulbecco's phosphate buffer (PBS (-)) solution of rat serum-derived vitronectin adjusted to a concentration of 5 μg/ml was first added to wells (50 μl/well) of a microplate (Maxisorp), and a reaction for immobilization was allowed to proceed at 4°C overnight. After washing twice with 150 μl of PBS (-), PBS (-) containing 10 mg/ml of bovine serum albumin was added, followed by blocking at 37°C for one hr. The microplate was then washed twice with 150 μl of PBS (-) to prepare an assay plate.

Rat peritoneal cavity macrophage was collected after the induction of thioglycolate into the peritoneal cavity. Specifically, a 3% thioglycolate medium was intraperitoneally administered into Wistar male rats (300 to 400 g) at a dose of 100 ml/kg, and, four days after the administration, intraperitoneal fluid and cold PBS (-) (100 ml/kg) injected into the peritoneal cavity were withdrawn to collect macrophage. The macrophage was isolated by removing nonadherent cells. Specifically, the cells collected from the peritoneal cavity were washed twice with PBS (-) and were then cultivated in a Dulbecco's modified Eagle's basal medium containing 0.1% bovine serum albumin under 5% carbon dioxide for 30 min to perform adhesion of macrophage. Next, nonadherent cells were removed, followed by washing with PBS (-). Thereafter, macrophage was collected with a cell scraper. The collected rat macrophage was washed twice with PBS (-) and was then suspended in a Dulbecco's modified Eagle's basal medium to a concentration of 8 x $10^5$ /ml. Next, 50 μl of a Dulbecco's modified Eagle's basal medium containing 0.2% bovine serum albumin with a medicament added thereto was added to the wells of the vitronectin-coated assay microplate, followed by pre-cultivation under 5% carbon dioxide at 37°C for 10 min. Thereafter, 50 μl of the Dulbecco's modified Eagle's basal medium with macrophage suspended therein was added thereto, and the plate was thoroughly stirred. A reaction was allowed to proceed under 5% carbon dioxide at 37°C for 10 min. The reaction solution containing nonadherent cells was then removed, followed by washing three times with PBS (-). For the adhered cells, 100 μl of PBS (-) containing 4% paraformaldehyde was added, and immobilization was allowed to proceed at room temperature for 10 min. Next, 100 μl of PBS (-) containing 0.5% Toluidine Blue and 4% paraformaldehyde was added, and staining was allowed to proceed at room temperature for 5 min, followed by thorough washing with distilled water. The inside of the wells was then air-dried, and 1% aqueous sodium dodecylsulfate solution was then added to perform cytolysis. The absorbance of the microplate thus obtained was measured at 595 nm. The total binding was defined as the absorbance of the well not containing the test compound, and the non-specific binding (100% inhibition) was defined as the absorbance of the well which does not contain vitronectin and has been subjected to blocking with bovine serum albumin.

As compared with 0.16 ± 0.036 (fluorescence intensity; arbitrary unit) for the control group, for the test group to which the compound represented by formula (IV) had been added, the fluorescence intensity was 0.086 ± 0.069 at $10^{-7}$ M, 0.031 ± 0.037 at $10^{-6}$ M, and 0.012 ± 0.038 at $10^{-5}$ M (for each group, average on three wells ± SD), demonstrating that the compound represented by formula (IV) inhibited the adhesion of rat macrophage to vitronectin in a dose-dependent manner.

The above results demonstrate that integrin $\alpha_v\beta_3$ antagonists inhibit the adhesion of rat macrophage to vitronectin. This suggests that, in rats, integrin $\alpha_v\beta_3$ antagonists possibly inhibit cardiac tissue injury and minimal circulation injury through a leukocyte adhesion inhibition mechanism. Accordingly, in order to demonstrate that the reperfusion injury is actually inhibited, an experiment was then carried out using an acute myocardial infarct model on the effect of myocardial infarct inhibition attained by integrin $\alpha_v\beta_3$ antagonists.

<u>Pharmacological Test Example 3: Inhibition of infarct size by integrin $\alpha_V\beta_3$ antagonists in rat acute myocardial ischemia-reperfusion model</u>

Infarct size reduction effect of various integrin $\alpha_V\beta_3$ antagonists different from each other in molecular main chain in an acute myocardial infarct model was studied using rats for which the inhibition of adhesion of macrophage to vitronectin has been confirmed.

Male Wistar rats (330 to 420 g) were subjected to controlled respiration using an artificial respirator under pentobarbital anesthesia (60 mg/kg, i.p.), and the blood pressure and the heart rate were measured through a blood pressure amplifier and a heart rate unit. Further, for the ischemia of the myocardium, an increase in voltage of R-wave in the II leading in an electrocardiogram was confirmed with a monitor through a bioelectricity amplifier. The rats underwent thoracotomy, and the coronary artery, together with the myocardium, was then occluded using a suture with a needle. Ischemia for 3 min and reperfusion for 5 min were followed by ischemia for 45 min and reperfusion for 45 min. Medicaments tested and concentrations thereof were 3 mg/kg + 3 mg/kg/hr (bolus + infusion) (L; low dose group) and 6 mg/kg + 3 mg/kg/hr (bolus + infusion) (H; high dose group) for the compound represented by formula (V); 3 mg/kg + 3 mg/kg/hr (bolus + infusion) for the compound represented by formula (VI); 3 mg/kg + 3 mg/kg/hr (bolus + infusion) (L; low dose group) and 6 mg/kg + 3 mg/kg/hr (bolus + infusion) (H; high dose group) for the compound represented by formula (IV); 5 mg/kg + 5 mg/kg/hr(bolus + infusion)(L; low dose group) and 10 mg/kg + 10 mg/kg/hr (bolus + infusion) (H; high dose group) for the compound represented by formula (VII); and 1 mg/kg + 1 mg/kg/hr (bolus + infusion) (L; low dose group) and 6 mg/kg + 6 mg/kg/hr (bolus + infusion) (H; high dose group) for the compound represented by formula (VIII). For a control group (group C), a 10% (v/v) dimethyl sulfoxide/5% glucose solution was

administered, and each medicament was dissolved in the same solvent. The dose volume was 3 ml/kg + 3 ml/kg/h (bolus + infusion). The medicament was continuously administered through an infusion pump for the period starting with one min before the ischemia and ending with the completion of the experiment. After reperfusion for 45 min, the rats were slaughtered by excessive administration of pentobarbital, and the heart was removed. After perfusion with a Ringer solution, perfusion staining was carried out with 1.5% triphenyltetrazolium chloride, and the proportion of the infarct area in the left ventricle was quantitatively determined. For animals used in the experiment, each group consisted of 5 rats.

The results of the first experiment were as follows. As compared with group C, the administration of the compound represented by formula (V) significantly reduced the myocardial infarct size (the proportion of the infarct area in the left ventricle (average value ± SE, n = 5)) in a dose-dependent manner, that is, by 16.1% for group L and by 35.5% for group H (group C: 46.5 ± 0.9%, group L: 39.0 ± 1.7%, and group H: 30.0 ± 1.6%). Also for the compound represented by formula (VI) which is an $\alpha_v\beta_3$ antagonist having a molecular main chain different from the compound represented by formula (V), as compared with group C, the infraction was significantly reduced, that is, by 34.8% (group C: 46.5 ± 0.9%, and group to which the compound represented by formula (VI) has been administered: 30.3 ± 6.1%). The doses, at which the infarct inhibition action could be obtained, had no influence on the blood pressure and the heart rate.

The results of the next experiment were as follows. As compared with group C, the administration of the compound represented by formula (IV) reduced the myocardial infarct size (the proportion of the infarct area in the left ventricle (average value ± SE, n = 5)) in a dose-dependent manner, that is, by 17.8% for group L and by 35.1% for group H (group C: 39.3 ± 4.0%, group L: 32.3 ± 4.9%, and group H: 25.5 ± 1.5%). The doses, at which the infarct inhibition action could be obtained, had no influence on the blood pressure and the heart rate.

The results of the next experiment were as follows. As compared with group C, the administration of the compound represented by formula (VII) reduced the myocardial infarct size (the proportion of the infarct area in the left ventricle (average value ± SE, n = 5)) in a dose-dependent manner, that is, by 24.5% for group L and by 41.5% for group H (group C: 38.8 ± 1.7%, group L: 29.3 ± 1.7%, and group H: 22.7 ± 3.8%). The doses, at which the infarct inhibition action could be obtained, had no influence on the blood pressure and the heart rate.

The results of the next experiment were as follows. As compared with group C, the administration of the compound represented by formula (VIII) reduced the myocardial infarct size (the proportion of the infarct area in the left ventricle (average value ± SE, n = 5)) in a dose-dependent manner, that is, by 24.2% for group L and by 32.5% for group H (group C: 36.0 ± 1.9%, group L: 27.3 ± 5.2%, and group H: 24.3 ± 3.8%). The doses, at which the infarct inhibition action could be obtained, had no influence on the blood pressure and the heart rate.

The above results demonstrate that various integrin $\alpha_v\beta_3$ antagonists different from each other in molecular main chain significantly reduced the infarct size and inhibited reperfusion injury. In this connection, it should be noted that integrin $\alpha_v\beta_3$ antagonists used in this assay system had antiplatelet activity in hamsters, dogs, and humans, whereas, at doses in the animal species in this experiment, that is, at doses in rats, for the reason of animal species difference, the platelet aggregation inhibitory activity was not observed. This indicates that the infarct size reduction effect is not mainly attributable to the antiplatelet activity but is mainly based on integrin $\alpha_v\beta_3$ antagonistic activity.

Pharmacological Test Example 4: Inhibition of infarct size by integrin $\alpha_V\beta_3$ antagonists in hamster acute ischemia-reperfusion model

Hamsters were selected as another animal species for which the inhibition of the adhesion of vascular smooth muscle cells to vitronectin has been substantiated, and an experiment was carried out on infarct size reduction effect of integrin $\alpha_v\beta_3$ antagonists in an acute myocardial infarction model using hamsters.

A polyethylene tube was inserted into the carotid artery and the survical vein of Syrian male hamsters (10 to 13 weeks old) under pentobarbital anesthesia (60 mg/kg, i.p.). An electrocardiogram (I or II leading) was measured through a bioelectricity amplifier by leading from electrodes which had pierced the limbs. A polyethylene tube was inserted into the trachea, and artificial respiration was carried out with an artificial respirator (amount of ventilation: 10 ml/kg/stroke, number of times of ventilation: 70 strokes/min). The blood pressure was measured through a blood pressure amplifier, and the heart rate was measured with a heart rate unit using blood pressure as a trigger. The animals underwent thoracotomy, and the coronary, together with the myocardium, was then occluded using a suture with a needle. Ischemia for 90 min was followed by reperfusion for 2 hr to quantitatively determine myocardial infarct size.

A sodium salt of the compound represented by formula (IV) was mixed with a 2-fold equivalent of arginine, and the mixture was dissolved in a 5% glucose solution. The dose volume was 2 ml/kg + 2 ml/kg/h (bolus + infusion). For a solvent administration group, a 0.64 mg/ml arginine/5% glucose solution was used. The medicament was continuously administered through an infusion pump for the period starting with one min before ischemia and ending with the completion of the experiment. After reperfusion for 2 hr, the animals were slaughtered by intraveneous injection of 1,000 U/kg of heparin and an excessive amount of pentobarbital, and the heart was then removed. After perfusion with

a Ringer solution, blood was washed away from the heart, and the coronary artery was re-occluded. After the reocclusion, 0.4 ml of 0.1% Evans blue was injected through the aorta. The heart was rinsed with physiological saline and was then cut into about 1 mm slices. For each slice, the left ventricle (LV) was separated, and the weight of the left ventricle and the weight of the area-at-risk (AR) were measured. The area-at-risk was stained with a 50 mM Tris·HCl (pH 7.4, 37°C) buffer containing 1.5% triphenyltetrazolium chloride heated at 37°C, and the weight of the infarct area (IS; infarct size) was measured. The measurement of the weight was carried out under a blind.

The platelets aggregation was measured by the Born's method (G. V. R. Born, Nature 194, 927 (1962)). Blood was collected through an injector containing a 3.8% sodium citrate solution from the aorta abdominalis (final concentration: 0.38%). Centrifugation was carried out at 900 rpm (about 80 x g) for 10 min to collect platelet-rich plasma, and centrifugation was further carried out at 3,000 rpm (about 880 x g) for 10 min to collect platelet-poor plasma, followed by the measurement of aggregation with an aggregometer. In this case, ADP (final concentration: 5 μM) was used as an aggregation inducer.

The administration of the compound represented by formula (IV) reduced the myocardial infarct size (IS/AR) by 30.9% for group L. For group H, a 49.3% reduction, which is a significant difference, could be realized (average ± SE, n = 5), solvent administration group: 49.3 ± 7.1%, group L (0.33 mg/kg + 0.33 mg/kg/h (bolus + infusion)); 34.0 ± 5.0%, group H (1.0 mg/kg + 1.0 mg/kg/h (bolus + infusion)); 25.0 ± 2.8%). For each group, the ischemia area in the left ventricle (AR/LV, average ± SE, n = 5) was 53.6 ± 3.6% for the solvent administration group, 51.5 ± 3.0% for group L, and 51.4 ± 4.3% for group H, indicating that there was no significant difference among the groups. For hamsters, the inhibition of the platelet aggregation by the administration of the compound represented by formula (IV) at these doses (both group H and group L) was not more than 20%. For the assay of each treatment group, Dunnett's multiple comparison test was used.

The above results demonstrate that, also in this assay system, integrin $\alpha_v\beta_3$ antagonists significantly inhibited the infarct size and reduced the reperfusion injury. In this connection, it should be noted that, although the compound represented by formula (IV) used in this assay system further has antiplatelet activity, the platelet aggregation inhibition was only 20% at the medicament doses in this experiment, indicating that the infract size reduction effect is not mainly attributable to the antiplatelet activity but is mainly based on integrin $\alpha_v\beta_3$ antagonistic activity.

Pharmacological Test Example 5: Inhibition of infarct by integrin $\alpha_V\beta_3$ antagonists in dog myocardial ischemia-reperfusion model

Dogs were selected as further animal species for which the inhibition of the adhesion of vascular smooth muscle cells to vitronectin has been substantiated, and an experiment was carried out on infarct size reduction effect of integrin $\alpha_v\beta_3$ antagonists in a dog myocardial ischemia-reperfusion model.

Female and male beagles were induced anesthetisia with thiopental. A tube was inserted into the trachea, and controlled respiration was carried out. During the operation, the anesthesia was maintained with 1.5% sevoflurane. Thoracotomy was carried out at the left fourth intercostal. The base of the left circumflex coronary artery was occluded for 90 min, and reperfusion was then carried out, followed by ceasing thoracotomy and awakening. After reperfusion for 4 to 48 hr, the animals were slaughtered by an overdose of pentobarbital, and the heart was removed, followed by double staining with Evans blue and triphenyltetrazolium chloride to identify nonischemic area, ischemic area, and infarct area. Further, at the time of the completion of the reperfusion, the platelet aggregation and the bleeding time were measured. The compounds represented by formulae (IV), (VII), and (VIII) and the compounds represented by formulae (IX), (X), and (XI), which are different from the compounds represented by formulae (IV), (VII), and (VIII) in molecular main chain, were persistently intraveneously administered for the period starting with one minute before the reperfusion and ending with the completion of the reperfusion. The highest dose was 40 μg/kg + 80 μg/kg/h (bolus + infusion), and the lowest dose was 5 μg/kg + 3 μg/kg/h (bolus + infusion). The high dose was the dose at which the platelet aggregation was fully inhibited, specifically the dose at which the platelet aggregation was inhibited by about 70% to about 100%. The low dose was the dose at which the platelet aggregation was hardly inhibited, specifically the dose at which the platelet aggregation was inhibited by about 30% to about 40%. For a control group (C), a solvent used for medicament administration was administered. Each group consisted of 6 to 9 dogs (n = 6 to 9).

The administration of some compounds among the compounds represented by formulae (IV), (VII), (VIII), (IX), (X), and (XI) significantly reduced the myocardial infarct size (infarct area/ischemia area).

The above results reveal that, in this assay system, integrin $\alpha_v\beta_3$ antagonists, which had been administered one min before the reperfusion for reflecting the clinical effect, significantly reduced the infarct size and inhibited the reperfusion injury. In this assay system, in order to distinguish medicaments effective only in an early stage of reperfusion, for example, CY-1503 (Circulation, 1996; 94: 542-546), from more persistently effective medicaments, a long reperfusion time was ensured. It should be noted that the compounds represented by formulae (IV), (VII), (VIII), (IX), (X), and (XI) used in the assay system have antiplatelet activity as well, indicating that, in order to effectively reduce the infarct size, the compounds may have a combination of the inhibitory activity against the adhesion and invasion of

leukocytes based on the integrin $\alpha_v\beta_3$ antagonistic activity with the antiplatelet activity.

When integrin $\alpha_v\beta_3$ antagonists are used as prophylactic or therapeutic agents for reperfusion injury, the antiplatelet activity is not necessarily required. When integrin $\alpha_v\beta_3$ antagonists are used as therapeutic agents for angiopathy, for example, for ischemic heart diseases or cerebral ischemic diseases, in some cases, superior clinical effect can be attained if the aimed integrin $\alpha_v\beta_3$ antagonist has antiplatelet activity. Accordingly, the integrin $\alpha_v\beta_3$ antagonists referred to in the present invention are not limited by whether or not the integrin $\alpha_v\beta_3$ antagonists have antiplatelet activity and include both compounds, which do not have antiplatelet activity and have integrin $\alpha_v\beta_3$ antagonistic activity, and compounds which have a combination of antiplatelet activity with integrin $\alpha_v\beta_3$ antagonistic activity.

Pharmacological Test Example 6: Inhibition of adhesion of human monocytes to human activated vascular endothelial cells

Further, in order to extrapolate clinical usefulness, the inhibitory activity of integrin $\alpha_v\beta_3$ antagonists against the adhesion of leukocytes was examined using human hemocytes.

The adhesion of human monocytes to vascular endothelial cells was measured with reference to the method of Murphy et al. (J. F. Murphy et al., Biochem. J. 304, 537 (1994)). Human funicular venoendothelial cells (4 x $10^4$ /well), which had been cultivated in a type-I collagen-coated flask containing a 199 medium containing an endothelial cell growth factor (15 μg/ml), heparin (5 U/ml), and 20% fetal calf serum, were cultivated using a type-I collagen-coated microplate in the above medium under 5% carbon dioxide at 37°C for 2 days to prepare a layer of vascular endothelial cells. The layer of vascular endothelial cells was washed with PBS (-) and was then cultivated in PBS (-) containing 1 U/ml bovine thrombin, 5 mM $CaCl_2$, and 5 mM $MgCl_2$ for 5 min, followed by washing with PBS(-) containing $10^{-6}$ M D-phenylalanyl-L-prolyl-L-argininechloromethane and with PBS(-).

Next, U937 cells, which had been cultivated in an RPMI 1640 medium containing 10% fetal calf serum, were cultivated in the above medium containing 6 μM 2',7'-bis(carboxyethyl)carboxyfluorescein tetraacetoxymethyl ester under 5% carbon dioxide at 37°C for one hr, were washed with PBS (-), and were subjected to fluorescent labeling. The labeled U937 cells (2 x $10^5$ /well), the medicament, and 100 μl of an RPMI 1640 medium containing 1% fetal calf serum were added onto the layer of vascular endothelial cells, and an adhesion reaction was allowed to proceed under 5% carbon dioxide at 37°C for 10 min. Next, nonadherent cells were removed, and 50 mM Tris·HCl (pH 8.4) containing 0.1% sodium dodecylsulfate was added to elute the fluorescent dye, followed by fluorometry (excitation wavelength 485 nm, measurement wavelength 530 nm).

As compared with 4522 ± 1027 (fluorescence intensity; arbitrary unit) for the control group, for the medicament administration group to which the compound represented by formula (IV) had been added, the fluorescence intensity was 4389 ± 640 at $10^{-7}$ M, 3710 ± 969 at $10^{-6}$ M, 1968 ± 187 at $10^{-5}$ M, and -34 ± 74 at $10^{-4}$ M (for each group, average on three wells ± SD), demonstrating that the compound represented by formula (IV) inhibited the adhesion of monocytes in a dose-dependent manner.

The above results demonstrate that, also in the assay system using human cells, integrin $\alpha_v\beta_3$ antagonists inhibit the adhesion of leukocytes to vascular endothelial cells. That is, also in the assay system using human cells, antileukocyte activity of integrin $\alpha_v\beta_3$ antagonists very deeply involved in reperfusion injury have been proved. This suggests that integrin $\alpha_v\beta_3$ antagonists can prevent or treat reperfusion injury clinically.

## Claims

1. A pharmaceutical composition for use in the treatment or prevention of reperfusion injury, which comprises an integrin $\alpha_v\beta_3$ antagonist, a pharmaceutically acceptable salt thereof, or a solvate thereof as active ingredient.

2. The pharmaceutical composition according to claim 1, wherein the integrin $\alpha_v\beta_3$ antagonist is a compound represented by formula (I):

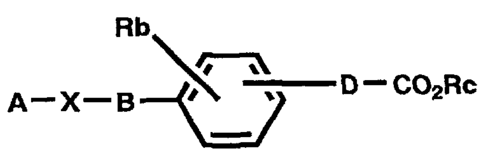

(I)

wherein

A represents

a hydrogen atom,

a saturated or unsaturated five- to seven-membered heterocyclic group containing at least one nitrogen atom, which is optionally condensed with another saturated or unsaturated five- to seven-membered carbocyclic ring or heterocyclic ring to form a bicyclic group, wherein the heterocyclic and bicyclic groups are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl and the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl,

or a group represented by formula

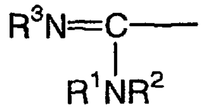

or

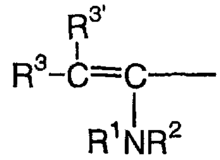

wherein $R^1$, $R^2$, $R^3$, and $R^{3'}$, which may be the same or different, represent a hydrogen atom, $C_{1-6}$ alkyl, $C_{1-6}$ alkoxy, hydroxyl, $C_{1-6}$ alkoxycarbonyl, $C_{2-6}$ alkenyl, $C_{2-6}$ alkynyl, aralkyl, or nitrile, or $R^1$ and $R^2$ may together form group $-(CH_2)i-$, wherein i represents 4 or 5, or group $-(CH_2)_2-O-(CH_2)_2-$, and the $C_{1-6}$ alkyl, $C_{1-6}$ alkoxy, hydroxyl, $C_{1-6}$ alkoxycarbonyl, $C_{2-6}$ alkenyl, $C_{2-6}$ alkynyl, and aralkyl groups are optionally substituted by a halogen atom, $C_{1-6}$ alkoxy, amino, $C_{1-6}$ alkyl, or hydroxyl;

X represents -NH-, $-CH_2-$, or a bond, preferably -NH-;

B represents

$C_{1-6}$ alkylene chain optionally containing an unsaturated bond,

$C_{1-6}$ alkylene chain optionally containing an unsaturated bond wherein one or two methylene portions in the chain may be replaced by group(s) or atom(s) selected from the group consisting of an oxygen atom, a sulfur atom, an optionally substituted imino group, or a carbonyl group at any position(s),

a piperazine ring,

a pyrrolidine ring,

a piperidine ring,

a group represented by formula $-C_6H_4CONH-$,

a group represented by formula $-C_6H_4NHCO-$,

a group represented by formula $-C_6H_4SO_2NH-$,

a group represented by formula $-C_6H_4NHSO_2-$,

a pyridine ring,

a pyrimidine ring, or

a triazine ring, wherein

the piperazine ring, the pyrrolidine ring, the piperidine ring, $-C_6H_4CONH-$, $-C_6H_4NHCO-$, $-C_6H_4SO_2NH-$, $-C_6H_4NHSO_2-$, pyridine ring, pyrimidine ring, or the triazine ring is optionally substituted by $C_{1-6}$ alkyl, $C_{1-6}$ alkoxy, a halogen atom, amino, nitro, hydroxyl, or an oxygen atom and the $C_{1-6}$ alkyl and $C_{1-6}$ alkoxy groups are optionally substituted by a halogen atom, $C_{1-6}$ alkoxy, amino, or hydroxyl;

D represents

a group represented by formula $-CONR^4CHR^5CHR^6-$ wherein $R_4$ represents a hydrogen atom, $C_{1-6}$ alkyl, $C_{2-6}$ alkenyl, $C_{2-6}$ alkynyl, or aralkyl and the $C_{1-6}$ alkyl, $C_{2-6}$ alkenyl, $C_{2-6}$ alkynyl, and aralkyl groups are optionally substituted by a halogen atom, $C_{1-6}$ alkoxy, amino, or hydroxyl; $R^5$ and $R^6$, which may be the same or different, represent a hydrogen atom, a group represented by formula $-NR^7COR^8$ wherein $R^7$ represents a hydrogen atom, $C_{1-6}$ straight chain alkyl, or $C_{3-6}$ branched chain alkyl and $R^8$ represents a hydrogen atom, $C_{1-6}$ straight chain alkyl, $C_{2-6}$ alkenyl, $C_{3-6}$ branched chain alkyl, $C_{7-10}$ aralkyl, a five- to ten-membered monocyclic or bicyclic carbocyclic group, preferably a five- to seven-membered monocyclic carbocyclic group, or a five- to ten-membered monocyclic or bicyclic heterocyclic group, preferably a five- to seven-membered monocyclic heterocyclic group and the straight chain alkyl, alkenyl, and branched chain alkyl groups optionally represented by $R^8$ are optionally substituted by $C_{1-6}$ alkyl, $C_{1-6}$ alkoxy, $C_{1-6}$ alkoxycarbonyl, aralkyl, amino, or hydroxyl and the aralkyl, carbocyclic, and heterocyclic groups optionally represented by $R^8$ may be substituted by hydroxyl, amino, azido, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl; a group represented by formula $-NR^7CO_2R^8$ wherein $R^7$ is as defined above and $R^8$ is as defined above; a group represented by formula $-NR^7SO_2R^8$ wherein $R^7$ is as defined above and $R^8$ is as defined above; a group represented by formula $-NR^7R^8$ wherein $R^7$ is as defined above and $R^8$ is as defined above; a group represented by

formula -$OR^8$ wherein $R^8$ is as defined above; or a group -$R^8$ wherein $R^8$ is as defined above,

a group represented by formula -$NR^4COCHR^5CHR^6$- wherein $R^4$, $R^5$, and $R^6$ are as defined above,

a group represented by formula -$CH_2NR^4CHR^5CHR^6$- wherein $R^4$, $R^5$, and $R^6$ are as defined above,

a group represented by formula -$CHR^5CHR^6$- wherein $R^4$, $R^5$, and $R^6$ are as defined above,

a cyclopropyl ring optionally substituted by hydroxyl, amino, azido, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl, or

vinylene optionally substituted by $C_{1-6}$ alkyl, $C_{1-6}$ alkylcarbonyl, $C_{1-6}$ alkoxycarbonyl, carboxyl, a halogen atom, azido, nitro, or cyano group,

$R^b$ represents
a hydrogen atom,
a halogen atom,
nitro,
amino,
alkylamino,
dialkylamino,
cyano,
hydroxyl,
thiol optionally substituted by $C_{1-2}$ alkyl or phenyl,
$C_{1-6}$ alkyl optionally substituted by $C_{1-6}$ alkyl, $C_{1-6}$ alkoxy, $C_{1-6}$ alkoxycarbonyl, aralkyl, amino, or hydroxyl, or
$C_{1-6}$ alkoxy optionally substituted by $C_{1-6}$ alkyl, $C_{1-6}$ alkoxy, $C_{1-6}$ alkoxycarbonyl, aralkyl, amino, or hydroxyl; and

$R^c$ represents a hydrogen atom, $C_{1-6}$ straight chain alkyl, $C_{3-6}$ branched chain alkyl, or $C_{7-10}$ aralkyl and this aralkyl group is optionally substituted by hydroxyl, amino, azido, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl.

**3.** The pharmaceutical composition according to claim 2, wherein A represents the following group:

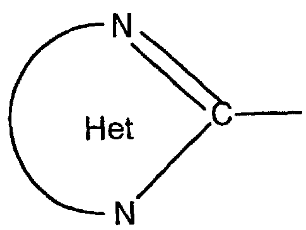

or

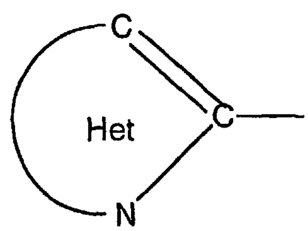

wherein

Het represents a saturated or unsaturated five- to seven-membered heterocyclic group containing at least one nitrogen atom and this heterocyclic group is optionally condensed with another saturated or unsaturated five- to seven-membered carbocyclic ring or heterocyclic ring to form a bicyclic group and the heterocyclic and bicyclic groups are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl in which the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl.

**4.** The pharmaceutical composition according to claim 2, wherein A represents the following group:

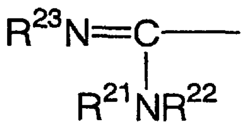

or

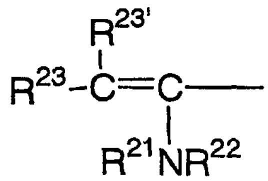

wherein

$R^{21}$, $R^{22}$, $R^{23}$, and $R^{23'}$, which may be the same or different, represent a hydrogen atom, $C_{1-6}$ alkyl, $C_{1-6}$

alkoxy, hydroxyl, $C_{1-6}$ alkoxycarbonyl, $C_{2-6}$ alkenyl, or aralkyl and the $C_{1-6}$ alkyl, $C_{1-6}$ alkoxy, hydroxyl, $C_{1-6}$ alkoxycarbonyl, $C_{2-6}$ alkenyl, and aralkyl groups are optionally substituted by a halogen atom, $C_{1-6}$ alkoxy, amino, $C_{1-6}$ alkyl, or hydroxyl, or

$R^{21}$ and $R^{23}$ may together form

group $-(CH_2)_4-$,

group $-(CH_2)_3-$,

group $-CHR^{24}CH_2CH_2-$ wherein $R^{24}$ represents $C_{1-6}$ alkyl, amino, or hydroxyl and the amino and hydroxyl groups are optionally substituted by $C_{1-6}$ alkyl, $C_{1-6}$ alkoxycarbonyl, aralkyl, or aralkyloxycarbonyl,

group $-CH_2CHR^{24}CH_2-$ wherein $R^{24}$ is as defined above,

group $-CH_2CH_2-$,

group $-CHR^{24}CH_2-$ wherein $R^{24}$ is as defined above,

group $-CR^{25}=CR^{26}-$ wherein $R^{25}$ and $R^{26}$, which may be the same or different, represent a hydrogen atom or $C_{1-6}$ alkyl, or $R^{25}$ and $R^{26}$ may together form -CH=CH-CH=CH-, $-CR^{24}=CH-CH=CH-$ wherein $R^{24}$ is as defined above, $-CH=CR^{24}-CH=CH-$ wherein $R^{24}$ is as defined above, -N=CH-CH=CH-, or -CH=N-CH=CH-, or

$R^{21}$ and $R^{23}$ may together form

=CH-CH=CH-,

=CH-CH=N-, or

=CH-N=CH-, and

$R^{22}$ may represent a single bond between $R^{21}$ and the nitrogen atom attached to $R^{21}$.

**5.** The pharmaceutical composition according to claim 2, wherein A represents a hydrogen atom, amidino, imidazolyl, imidazolidinyl, thiazolyl, thiazolidinyl, pyridinyl, pyrimidinyl, tetrahydropyrimidinyl, triazinyl, benzimidazolyl, or azabenzimidazolyl and the groups other than a hydrogen atom are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl, and the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl.

**6.** The pharmaceutical composition according to claim 2, wherein B represents a group selected from the following:

* $-CH_2CH_2O-$

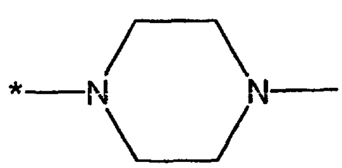
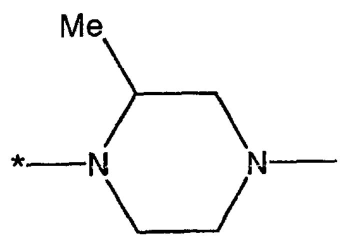

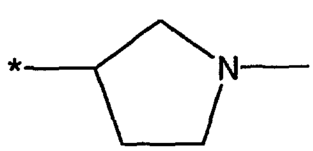
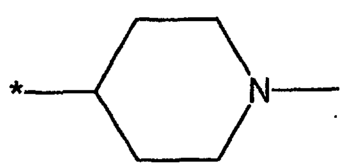

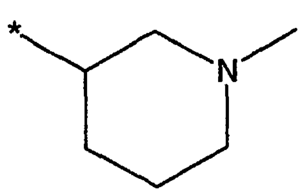
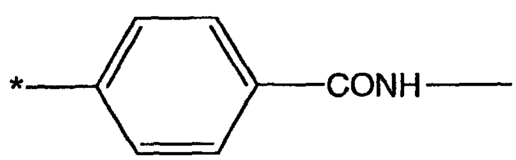

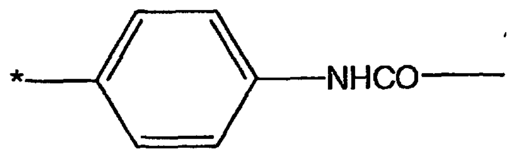

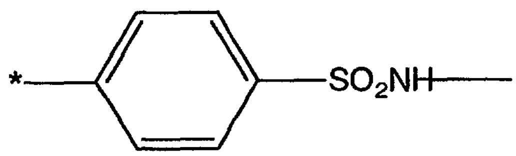

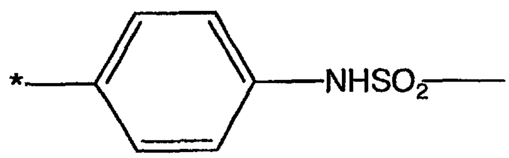

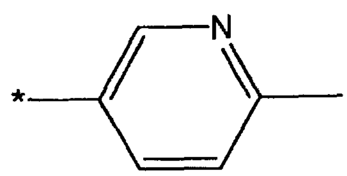

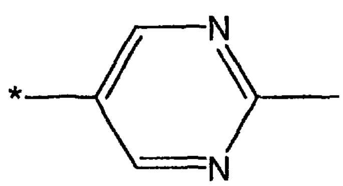

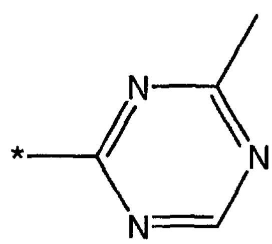

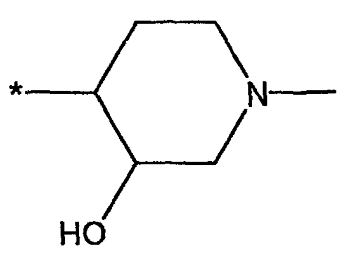

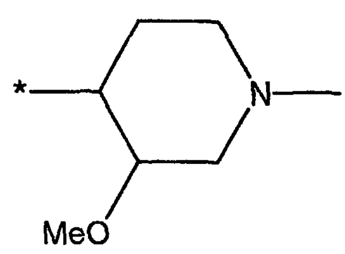

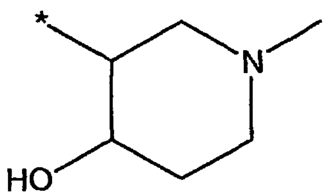

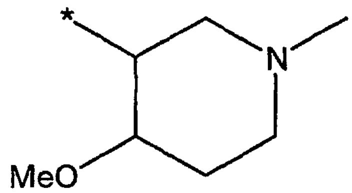

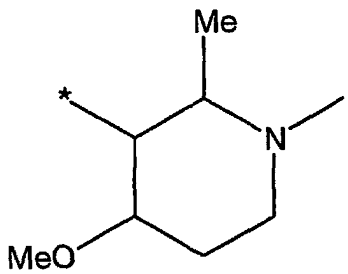

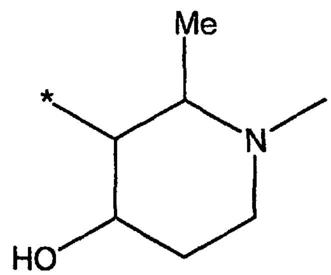

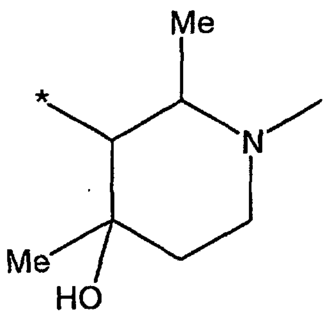

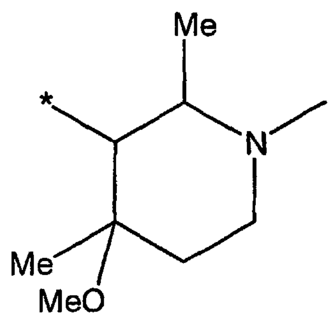

wherein Me represents methyl, the bond having * represents a bond between B and X and the other bond represents a bond between B and the phenylene portion.

7. The pharmaceutical composition according to claim 2, wherein D represents group $-CONR^4CHR^5CHR^6-$.

8. The pharmaceutical composition according to claim 1, wherein the integrin $\alpha_v\beta_3$ antagonist is a compound represented by formula (II):

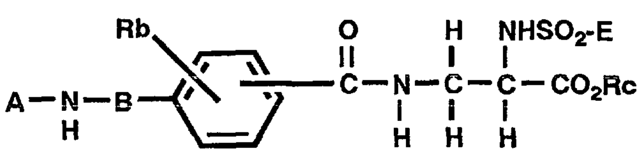
(II)

wherein

A represents a hydrogen atom, amidino, imidazolyl, imidazolidinyl, thiazolyl, thiazolidinyl, pyridinyl, pyrimidinyl, tetrahydropyrimidinyl, triazinyl, benzimidazolyl, or azabenzimidazolyl, and

the groups other than a hydrogen atom are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl and the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl;

B represents

$C_{1-6}$ alkylene chain optionally containing an unsaturated bond,

$C_{1-6}$ alkylene chain optionally containing an unsaturated bond wherein one or two methylene portions in the chain may be replaced by group(s) or atom(s) selected from the group consisting of an oxygen atom, a sulfur atom, an optionally substituted imino group, or a carbonyl group at any position(s),

a piperazine ring,

a pyrrolidine ring,

a piperidine ring,

a group represented by formula $-C_6H_4CONH-$,

a group represented by formula $-C_6H_4NHCO-$,

a group represented by formula $-C_6H_4SO_2NH-$,

a group represented by formula $-C_6H_4NHSO_2-$,

a pyridine ring,

a pyrimidine ring, or

a triazine ring, wherein

the piperazine ring, the pyrrolidine ring, the piperidine ring, $-C_6H_4CONH-$, $-C_6H_4NHCO-$, $-C_6H_4SO_2NH-$, $-C_6H_4NHSO_2-$, the pyridine ring, the pyrimidine ring, or the triazine ring is optionally substituted by $C_{1-6}$ alkyl, $C_{1-6}$ alkoxy, a halogen atom, amino, nitro, hydroxyl, or an oxygen atom and the $C_{1-6}$ alkyl and $C_{1-6}$ alkoxy groups are optionally substituted by a halogen atom, $C_{1-6}$ alkoxy, amino, or hydroxyl;

E represetns $C_{1-6}$ straight chain alkyl, $C_{2-6}$ alkenyl, $C_{3-6}$ branched chain alkyl, $C_{7-10}$ aralkyl, a five- to ten-membered monocyclic or bicyclic carbocyclic group, preferably a five- to seven-membered monocyclic carbocyclic group, or a five- to ten-membered monocyclic or bicyclic heterocyclic group, preferably a five- to seven-membered monocyclic heterocyclic group, wherein the straight chain alkyl, alkenyl, and branched chain alkyl groups optionally represented by E are optoinally substituted by $C_{1-6}$ alkyl, $C_{1-6}$ alkoxy, $C_{1-6}$ alkoxycarbonyl, aralkyl, amino, or hydroxyl, the aralkyl, carbocyclic, and heterocyclic groups optionally represented by E are optionally substituted by hydroxyl, amino, azido, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl, and examples of the five- to seven-membered monocyclic carbocyclic or heterocyclic group optionally represented by E include cyclopentyl, cyclohexyl, cycloheptyl, phenyl, pyridyl, pyrimidyl, morpholinyl, piperazinyl, piperidinyl, pyrrolidinyl, thienyl, and furyl;

$R^b$ represents

a hydrogen atom,

a halogen atom,

nitro,

amino,

alkylamino,

dialkylamino,

cyano,

hydroxyl,

thiol optionally substituted by $C_{1-2}$ alkyl or phenyl,

$C_{1-6}$ alkyl optionally substituted by $C_{1-6}$ alkyl, $C_{1-6}$ alkoxy, $C_{1-6}$ alkoxycarbonyl, aralkyl, amino, or hydroxyl, or

$C_{1-6}$ alkoxy optionally substituted by $C_{1-6}$ alkyl, $C_{1-6}$ alkoxy, $C_{1-6}$ alkoxycarbonyl, aralkyl, amino, or hydroxyl;

and

$R^c$ represents a hydrogen atom, $C_{1-6}$ straight chain alkyl, $C_{3-6}$ branched chain alkyl, or $C_{7-10}$ aralkyl and this aralkyl group is optionally substituted by hydroxyl, amino, azide, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl.

**9.** The pharmaceutical composition according to claim 1, wherein the integrin $\alpha_v\beta_3$ antagonist is a compound represented by formula (III):

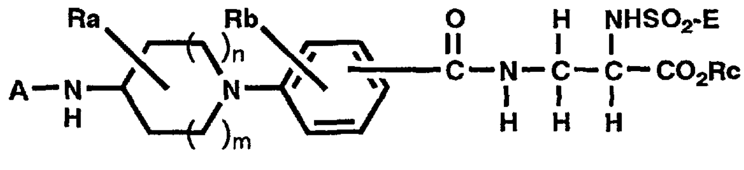

(III)

wherein

A represents a hydrogen atom, amidino, imidazolyl, imidazolidinyl, thiazolyl, thiazolidinyl, pyridinyl, pyrimidinyl, tetrahydropyrimidinyl, triazinyl, benzimidazolyl, or azabenzimidazolyl, and

the groups other than a hydrogen atom are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl and the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl;

E represents $C_{1-6}$ straight chain alkyl, $C_{2-6}$ alkenyl, $C_{3-6}$ branched chain alkyl, $C_{7-10}$ aralkyl, a five- to ten-membered monocyclic or bicyclic carbocyclic group, preferably a five- to seven-membered monocyclic carbocyclic group, or a five- to ten-membered monocyclic or bicyclic heterocyclic group, preferably a five- to seven-membered monocyclic heterocyclic group, wherein the straight chain alkyl, alkenyl, and branched chain alkyl groups optionally represented by E are optoinally substituted by $C_{1-6}$ alkyl, $C_{1-6}$ alkoxy, $C_{1-6}$ alkoxycarbonyl, aralkyl, amino, or hydroxyl, and the aralkyl, carbocyclic, and heterocyclic groups optionally represented by E are optionally substituted by hydroxyl, amino, azido, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl;

$R^a$ represents
a hydrogen atom,
hydroxyl,
thiol optionally substituted by $C_{1-2}$ alkyl or phenyl,
$C_{1-6}$ alkyl,
$C_{1-6}$ alkoxy,
amino,
alkylamino,
dialkylamino,
a halogen atom, or
=O;

$R^b$ represents
a hydrogen atom,
a halogen atom,
nitro,
amino,
alkylamino,
dialkylamino,
cyano,
hydroxyl,
thiol optionally substituted by $C_{1-2}$ alkyl or phenyl,
$C_{1-6}$ alkyl optionally substituted by $C_{1-6}$ alkyl, $C_{1-6}$ alkoxy, $C_{1-6}$ alkoxycarbonyl, aralkyl, amino, or hydroxyl, or
$C_{1-6}$ alkoxy optionally substituted by $C_{1-6}$ alkyl, $C_{1-6}$ alkoxy, $C_{1-6}$ alkoxycarbonyl, aralkyl, amino, or hydroxyl;

$R^c$ represents a hydrogen atom, $C_{1-6}$ straight chain alkyl, $C_{3-6}$ branched chain alkyl, or $C_{7-10}$ aralkyl and this aralkyl group is optionally substituted by hydroxyl, amino, azido, $C_{1-6}$ alkoxy, $C_{1-6}$ alkylamino, di-$C_{1-6}$ alkylamino, nitro, cyano, $C_{1-6}$ alkyl, $C_{1-6}$ alkoxycarbonyl, carboxyl, a halogen atom, or $C_{1-6}$ alkylcarbonyl;

m is 0, 1, or 2; and
n is 0, 1, or 2.

**10.** The pharmaceutical composition according to claim 1, wherein the integrin $\alpha_v\beta_3$ antagonist is a compound selected from the group consisting of formulae (IV), (V), (VI), (VII), (VIII), (IX), (X), and (XI):

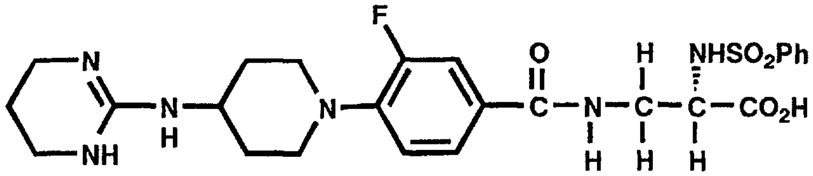

(IV)

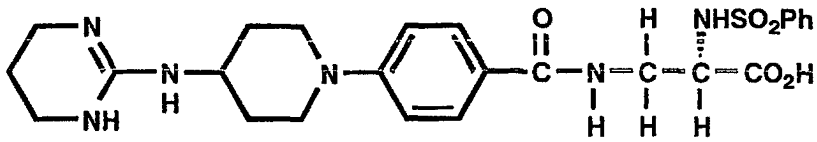

(V)

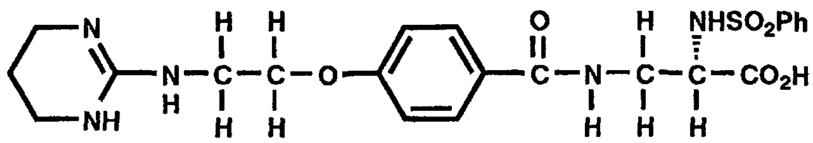

(VI)

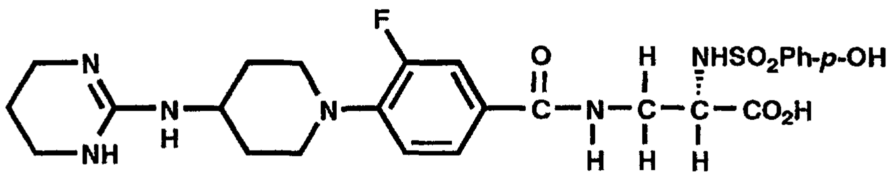

(VII)

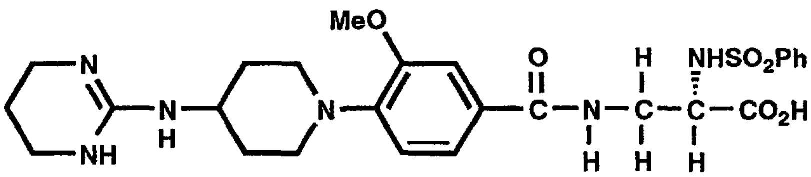

(VIII)

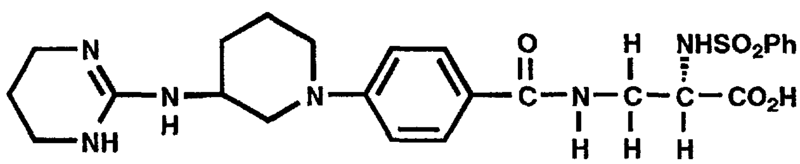

(IX)

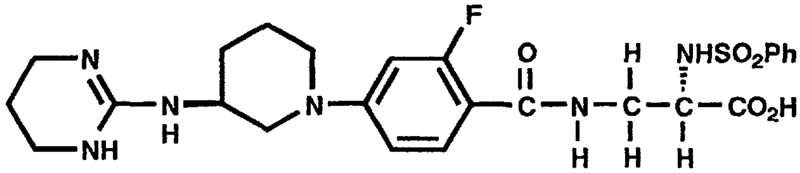

(X)

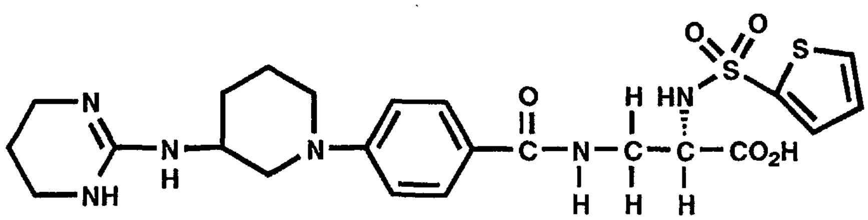

(XI)

**11.** The pharmaceutical composition according to any one of claims 1 to 10, for use in the treatment or prevention of reperfusion injury involved in recanalization therapy of ischemic heart diseases.

**12.** The pharmaceutical composition according to any one of claims 1 to 10, for use in the treatment or prevention of reperfusion injury involved in recanalization therapy of cerebral ischemic diseases.

**13.** The pharmaceutical composition according to any one of claims 1 to 10, for use in the treatment or prevention of reperfusion injury involved in organ transplantation or blood transplantation.

**14.** The pharmaceutical composition according to any one of claims 1 to 10, for use in the treatment or prevention of reperfusion injury involved in the use of artificial organs.

**15.** Use of an integrin $\alpha_v\beta_3$ antagonist, a pharmaceutically acceptable salt thereof, or a solvate thereof, for the manufacture of an agent for use in the treatment or prevention of reperfusion injury.

**16.** The use according to claim 15, wherein the integrin $\alpha_v\beta_3$ antagonist is the compound according to any one of claims 2 to 10.

**17.** The use according to claim 15 or 16, wherein the reperfusion injury is selected from reperfusion injury involved in recanalization therapy of ischemic heart diseases, reperfusion injury involved in recanalization therapy of cerebral ischemic diseases, reperfusion injury involved in organ transplantation or blood transplantation, and reperfusion injury involved in the use of artificial organs.

**18.** A method for treating or preventing reperfusion injury, which comprises the step of administering an effective amount of an integrin $\alpha_v\beta_3$ antagonist, a pharmaceutically acceptable salt thereof, or a solvate thereof together with a pharmaceutically acceptable carrier, to a mammal.

**19.** The method according to claim 17, wherein the integrin $\alpha_v\beta_3$ antagonist is the compound according to any one of claims 2 to 10.

**20.** The method according to claim 18 or 19, wherein the reperfusion injury is selected from reperfusion injury involved in recanalization therapy of ischemic heart diseases, reperfusion injury involved in recanalization therapy of cerebral ischemic diseases, reperfusion injury involved in organ transplantation or blood transplantation, and reperfusion injury involved in the use of artificial organs.

## INTERNATIONAL SEARCH REPORT

International application No.
PCT/JP01/00496

A. CLASSIFICATION OF SUBJECT MATTER
Int.Cl$^7$ A61K45/00, A61K31/506, A61P9/08, A61P9/10 // C07D401/12, C07D409/14

According to International Patent Classification (IPC) or to both national classification and IPC

B. FIELDS SEARCHED

Minimum documentation searched (classification system followed by classification symbols)
Int.Cl$^7$ A61K45/00, A61K31/506, A61P9/08, A91P9/10, C07D401/12, C07D409/14

Documentation searched other than minimum documentation to the extent that such documents are included in the fields searched

Electronic data base consulted during the international search (name of data base and, where practicable, search terms used)
REGISTRY (STN), CA (STN), CAPLUS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | GAWAZ, Meinrad et al., "Vitronectin receptor (αvβ3) mediates platelet adhesion to the luminal aspect of endothelial cells: implications for reperfusion in acute myocardial infarction", Circulation (1997), 96(6), 1809-1818 | 1,11-15,17<br>2-10,16 |
| Y | WO, 99/52872, A1 (Meiji Seika Kaisha, Ltd.), 21 October, 1999 (21.10.99), & EP, 1074543, A1 & AU, 9931678, A | 2-10,16 |
| Y | WO, 95/32710, A1 (Merck & Co., Inc.), 07 December, 1995 (07.12.95), & JP, 10-501222, A & EP, 760658, A1 & US, 5741796, A & AU, 9525868, A | 2,5-10,16 |
| Y | WO, 99/38849, A1 (Meiji Seika Kaisha, Ltd.), 05 August, 1999 (05.08.99), & EP, 1057818, A1 & AU, 9920762, A | 2-10,16 |
| Y | WO, 99/50249, A2 (Du Pont Pharmaceuticals Company), 07 October, 1999 (07.10.99), & EP, 1054871, A2 & AU, 9932137, A | 2-10,16 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 April, 2001 (17.04.01) | 01 May, 2001 (01.05.01) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/00496

C (Continuation). DOCUMENTS CONSIDERED TO BE RELEVANT

| Category* | Citation of document, with indication, where appropriate, of the relevant passages | Relevant to claim No. |
|---|---|---|
| Y | WO, 99/52879, A1 (American Home Products Corporation), 21 October, 1999 (21.10.99), & AU, 9932610, A | 2-10,16 |
| Y | WO, 97/36858, A1 (G.D. Searle & Co.), 09 October, 1997 (09.10.97), & JP, 2000-507575, A& EP, 889875, A1 & US, 5773644, A & AU, 9723238, A | 2-6,16 |
| Y | WO, 97/36859, A1 (G.D. Searle & Co.), 09 October, 1997 (09.10.97), & JP, 2000-515493, A& EP, 891325, A1 & US, 5952381, A & AU, 9725360, A | 2-6,16 |
| Y | WO, 97/36860, A1 (G.D. Searle & Co.), 09 October, 1997 (09.10.97), & JP, 2000-510098, A& EP, 894084, A1 & US, 5852210, A & AU, 9723371, A | 2-6,16 |

Form PCT/ISA/210 (continuation of second sheet) (July 1992)

**INTERNATIONAL SEARCH REPORT**

International application No.
PCT/JP01/00496

**Box I Observations where certain claims were found unsearchable (Continuation of item 1 of first sheet)**

This international search report has not been established in respect of certain claims under Article 17(2)(a) for the following reasons:

1. ☒ Claims Nos.: 18~20
because they relate to subject matter not required to be searched by this Authority, namely:

Claims 18 to 20 pertain to methods for treatment of the human body by surgery or therapy, as well as diagnostic methods, and thus relate to a subject matter which this International Searching Authority is not required to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)